# EUROPEAN PATENT SPECIFICATION

(11) **EP 4 229 060 B1**
(45) Date of publication and mention of the grant of the patent: **21.10.2026**
(21) Application number: 21802537.7
(22) Date of filing: 13.10.2021
(51) Int. Cl.: C07D 487/04, A61K 31/53, A61P 35/00

(54) **COMPOSITIONS AND METHODS FOR TREATING KIT-AND PDGFRA-MEDIATED DISEASES**
ZUSAMMENSETZUNGEN UND VERFAHREN ZUR BEHANDLUNG VON KIT- UND PDGFRA-VERMITTELTEN ERKRANKUNGEN
COMPOSITIONS ET MÉTHODES DE TRAITEMENT DE MALADIES À MÉDIATION PAR KIT ET PDGFRA

(30) Priority: 14.10.2020 US 202063091486 P
(43) Date of publication of application: 23.08.2023
(73) Proprietor: Blueprint Medicines Corporation, Cambridge, MA 02139 (US)
(72) Inventor: DINEEN, Thomas, A., Cambridge, MA 02139 (US); LYDON, Nicholas, Cambridge, MA 02139 (US)
(74) Representative: Mathys & Squire
(86) International application number: PCT/US2021/054663
(87) International publication number: WO 2022/081627

(56) References cited:
- EP-A1- 3 782 998
- WO-A1-2015/057873
- WO-A1-2018/183712
- WO-A1-2019/034128
- WO-A1-2019/201194
- WO-A1-2020/210293

## Description

This disclosure relates to novel compounds and their use as selective inhibitors of activated KIT and PDGFRα mutant protein kinases. The compounds disclosed herein are useful in pharmaceutical compositions, such as, *e.g.,* for the treatment of chronic disorders. The KIT receptor belongs to the class III receptor tyrosine kinase family that also includes the structurally related protein PDGFRα. Normally, stem cell factor binds to and activates KIT by inducing dimerization and autophosphorylation, which induces initiation of downstream signaling. In several tumor types, however, somatic activating mutations in KIT drive ligand-independent constitutive oncogenic activity, including tumor types such as acute myeloid leukemia, melanoma, intracranial germ cell tumors, mediastinal B-cell lymphoma, seminoma, and gastrointestinal stromal tumors. Mutant KIT is also known to play a role in mast cell activation, which is common and possibly necessary for maintenance. Disordered mast cell activation occurs when mast cells are pathologically overproduced or if their activation is out of proportion to the perceived threat to homeostasis. Mast cell activation syndrome refers to a group of disorders with diverse causes presenting with episodic multisystem symptoms as the result of mast cell mediator release. Mastocytosis is one type of mast cell activation syndrome. The World Health Organization (WHO) classifies mastocytosis into 7 different categories: cutaneous mastocytosis, indolent systemic mastocytosis (ISM), smoldering systemic mastocytosis (SSM), mastocytosis with an associated hematologic neoplasm (SM-AHN), aggressive systemic mastocytosis (ASM), mast cell leukemia (MCL) and mast cell sarcoma.

Systemic mastocytosis is a clonal disorder of mast cells characterized by increased mast cell burden, with focal and/or diffuse infiltrates of neoplastic mast cells in the skin, bone marrow, spleen, liver, gastrointestinal tract, and other organs, and increased release of mast cell mediators. SM includes 5 sub-types mastocytosis: indolent SM (ISM), smoldering SM (SSM), SM with an associated hematologic neoplasm of non-MC lineage (SM-AHN), aggressive SM (ASM), and MC leukemia (MCL). The latter three sub-classifications are associated with reduced overall survival and are grouped together as advanced SM (AdvSM). ISM is a chronic disorder associated with a normal or near-normal life-expectancy and the prognosis of SSM is intermediate. ISM and SSM are grouped together as non-advanced SM (non-Adv SM).

In all subtypes of SM and in a majority of patients with the disease, neoplastic mast cells display a mutation at the D816 position in exon 17 of KIT, which results in ligand-independent activation of KIT kinase activity. Wild-type mast cells require KIT activity for their differentiation and survival and, therefore, constitutive activation of KIT through D816V mutation is thought to be a pathogenic driver for SM. Specifically, KIT D816V mutations are found in 90% to 98% of patients with SM, with rare KIT D816Y, D816F, and D816H variants identified. Based on these findings, KIT D816V is considered a major therapeutic target in SM.

The chronic disorders indolent SM and SSM are characterized by severe symptoms, including pruritus, flushing, GI cramping, diarrhea, anaphylaxis, bone pain, and osteoporosis. These symptoms can be severely debilitating, having a negative impact on quality of life. There remain no approved therapies for ISM or SSM. Thus, the discovery of new treatments targeting ISM or SSM would be useful.

Compounds having mutant KIT and PDGFRα inhibitory activity have been described in WO2015/057873, CN108191874, WO2018/183712, WO2019/034128 WO2019/201194 and WO2020/210293. The chemical structures of compounds known in the art are different from the chemical structures of the compounds of this disclosure.

Furthermore, although compounds having mutant KIT and PDGFRα inhibitory activity are disclosed in the art, the properties of these known compounds are quite different from those of the compounds of the present disclosure.

An object of this disclosure is to provide novel compounds with highly selective, potent activity against mutant KIT and PDGFRα kinases for the safe and effective treatment of chronic disorders, such as ISM and SSM, as well as other diseases mediated by mutant KIT or PDGFRA. In treating these disorders, especially chronic disorders such as ISM and SSM, any new therapy should be well-tolerated. In particular, there is a need for new compounds targeting mutant KIT and PDGFRα kinases that have reduced levels of undesirable CNS side-effects which are associated with other known inhibitors of KIT and PDGFRα.

The present inventor has discovered novel compounds having high selectivity and potency against mutant KIT and PDGFRα kinases which, at the same time, possess additional desirable properties, such as, *e.g.,* little or no penetration into the CNS, low unbound concentrations in the brain and high levels or active transport out of the brain, *i.e.,* high efflux ratios from the CNS. In view of this desirable balance of properties, the compounds of the present disclosure are particularly suitable for treatment in the periphery, especially chronic treatment in the periphery, while side-effects in the CNS are reduced or minimized.

Thus, the compounds of the present disclosure aim to provide treatments having desirable efficacy, safety, and pharmaceutical properties for the treatment of KIT- and PDGFRA-mediated diseases. More specifically, the compounds of the disclosure exhibit a constellation of beneficial properties including a reduced level of brain penetration, while maintaining efficacy and other desirable pharmaceutical properties relative to the compounds known in the art having mutant KIT and PDGFRα inhibitory activity.

In one aspect, the present invention provides a compound of Formula (I) or a pharmaceutically acceptable salt thereof, and/or a solvate of any of the foregoing, as defined in the appended claims. Another aspect of the invention provides a pharmaceutical composition comprising the compounds of the invention with a pharmaceutically acceptable excipient. In a further aspect of the invention, the compounds or pharmaceutical compositions of the invention can be used in the treatment of a disease or condition in a patient in need thereof, wherein the disease or condition is as defined in the appended claims.

In more detail, the present invention relates to a compound of Formula (I): 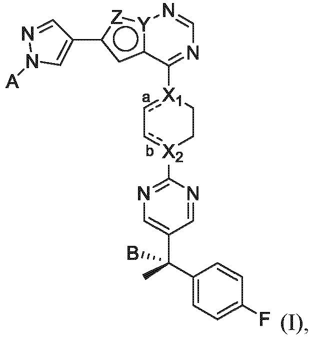 a pharmaceutically acceptable salt thereof, and/or a solvate of any of the foregoing, wherein:
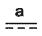 is chosen from a single bond and double bond;
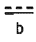 is chosen from a single bond and double bond;
Z is chosen from CH and NH;
Y is chosen from C and N;
X₁ is chosen from CH, C, and N;
X₂ is chosen from CH, C, and N;
   provided that when X₁ and X₂ are both N, then Y is not N and Z is not CH;
A is 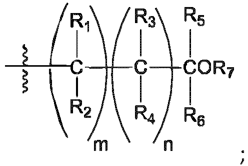
R₁ is chosen from hydrogen and methyl;
R₂ is chosen from hydrogen and methyl, or
R₁ and R₂ taken together form a cyclopropyl;
R₃ is chosen from hydrogen and methyl;
R₄ is chosen from hydrogen and methyl, or
R₃ and R₄ taken together form a cyclopropyl;
R₅ is chosen from hydrogen and methyl;
R₆ is chosen from hydrogen and methyl, or
R₅ and R₆ taken together form a cyclopropyl, or
one of R₂ or R₄ taken together with R₆ forms a cyclobutyl;
R₇ is hydrogen, or one of R₂, R₄, or R₆ taken together with R₇ forms a ring chosen from tetrahydrofuran, and tetrahydropyran, wherein said tetrahydrofuran or tetrahydropyran is optionally substituted with hydroxyl;
m is 0 or 1;
n is 0 or 1; and
B is chosen from OH and NH₂.

The present invention also provides a pharmaceutical composition comprising a compound of Formula (I), a pharmaceutically acceptable salt thereof, and/or a solvate of any of the foregoing; and a pharmaceutically acceptable excipient.

The present invention further provides a compound of Formula (I), a pharmaceutically acceptable salt thereof, and/or a solvate of any of the foregoing for use as a medicament in the treatment of a disease or condition in a patient in need thereof, wherein the disease or condition is chosen from systemic mastocytosis, gastrointestinal stromal tumors, acute myeloid leukemia, melanoma, seminoma, intracranial germ cell tumors, mediastinal B-cell lymphoma, Ewing's sarcoma, diffuse large B cell lymphoma, dysgerminoma, myelodysplastic syndrome, nasal NK/T-cell lymphoma, chronic myelomonocytic leukemia, and brain cancer; preferably wherein the disease or condition is systemic mastocytosis; preferably wherein the systemic mastocytosis is chosen from indolent systemic mastocytosis and smoldering systemic mastocytosis.

### Abbreviations and Definitions

The following abbreviations and terms have the indicated means throughout:

The term "KIT" refers to a human tyrosine kinase that may be referred to as mast/stem cell growth factor receptor (SCFR), proto-oncogene c-KIT, tyrosine-protein kinase Kit, or CD117. As used herein, the term "KIT nucleotide" encompasses the KIT gene, KIT mRNA, KIT cDNA, and amplification products, mutations, variations, and fragments thereof. "KIT gene" is used to refer to the gene that encodes a polypeptide with KIT kinase activity, *e.g.,* the sequence of which is located between nucleotides 55,524,085 and 55,606,881 of chromosome 4 of reference human genome hg19. "KIT transcript" refers to the transcription product of the KIT gene, one example of which has the sequence of NCBI reference sequence NM_000222.2. The term "KIT protein" refers to the polypeptide sequence that is produced by the translation of the KIT nucleotide or a portion thereof.

The term "PDGFRA" refers to a human tyrosine kinase that may be referred to as platelet derived growth factor alpha. As used herein, the term "PDGFRA nucleotide" encompasses the PDGFRA gene, PDGFRA mRNA, KIT cDNA, and amplification products, mutations, variations, and fragments thereof. "PDGFRA gene" is used to refer to the gene that encodes a polypeptide with PDGFRA kinase activity, *e.g*., the sequence of which is located between nucleotides 54,229,089 and 54,298,247 of chromosome 4 of reference Homo sapiens Annotation Release 109, GRCh38.p12. "PDGFRA transcript" refers to the transcription product of the PDGFRA gene, one example of which has the sequence of NCBI reference sequence NM_006206.6. The term "PDGFRA protein" or "PDGFRα" refers to the polypeptide sequence that is produced by the translation of the PDGFRA nucleotide or a portion thereof.

As used herein, a "malignant disease" refers to a disease in which abnormal cells divide without control and can invade nearby tissues. Malignant cells can also spread to other parts of the body through the blood or lymph system. Non-limiting examples of malignant diseases are carcinoma, sarcoma, leukemia, and lymphoma. Cancer is a non-limiting example of a malignant disease. In some embodiments, systemic mastocytosis is a non-limiting example of a malignant disease.

Non-limiting examples of cancer include gastrointestinal stomal tumor (GIST), AML (acute myeloid leukemia), melanoma, seminoma, intracranial germ cell tumors, and mediastinal B-cell lymphoma.

As used herein, an "eosinophilic disorder" refers to a disorder where eosinophils are found in an above-normal amount in various parts of the body and/or when there is a higher than normal ratio of hypodense versus normodense eosinophils (*e.g.,* greater than 30%). The eosinophilic disorder described herein are characterized by an overabundance of eosinophils (eosinophilia). The increased number of eosinophils inflame tissues and cause organ damage. The heart, lungs, skin, and nervous system are most often affected, but any organ can be damaged.

Eosinophilic disorders are diagnosed according to the location where the levels of eosinophils are elevated:
Eosinophilic pneumonia (lungs)
Eosinophilic cardiomyopathy (heart)
Eosinophilic esophagitis (esophagus - EoE)
Eosinophilic gastritis (stomach - EG)
Eosinophilic gastroenteritis (stomach and small intestine - EGE)
Eosinophilic enteritis (small intestine)
Eosinophilic colitis (large intestine - EC)
Hypereosinophilic syndrome (blood and any organ - HES)

As used herein, the term "subject" or "patient" refers to organisms to be treated by the methods of the present disclosure. Such organisms include, but are not limited to, mammals (e.g., murines, simians, equines, bovines, porcines, canines, felines, and the like), and in some embodiments, humans. References to methods of treatment throughout this description are to be interpreted as references to compounds and pharmaceutical compositions of the present disclosure for use in those methods of treatment.

As used herein, the phrase "therapeutically effective amount" refers to the amount of an active agent that is sufficient to effect beneficial or desired results. A therapeutically effective amount can be administered in one or more administrations, applications, or dosages and is not intended to be limited to a specific formulation or administration route.

As used herein, the phrase "weight equivalent of a pharmaceutically acceptable salt thereof" in reference to a specific compound includes the weight of both the compound and the associated salt.

As used herein, the phrase "pharmaceutically acceptable salt thereof," if used in relation to an active agent distributed as a salt form, refers to any pharmaceutically acceptable salt form of the active agent.

As used herein, the term "treating" includes any effect, *e.g*., lessening, reducing, modulating, ameliorating, or eliminating, that results in the improvement of the condition, disease, disorder, and the like, or ameliorating a symptom thereof.

While it is possible for an active agent to be administered alone, in some embodiments, the active agent can be administered as a pharmaceutical formulation, wherein the active agent is combined with one or more pharmaceutically acceptable excipients or carriers. For example, the active agent may be formulated for administration in any convenient way for use in human or veterinary medicine. In certain embodiments, the compound included in the pharmaceutical preparation may be active itself, or may be a prodrug, *e.g*., capable of being converted to an active compound in a physiological setting.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the definition of each expression, *e.g*., m, n, etc., when it occurs more than once in any structure, is intended to be independent of its definition elsewhere in the same structure.

Certain compounds of the disclosure may exist in particular geometric or stereoisomeric forms. The present disclosure contemplates all such compounds, including *cis-* and *trans*-isomers, *R-* and *S*-enantiomers, diastereomers, (*D*)-isomers, (*L*)-isomers, racemic mixtures thereof, and other mixtures thereof, as falling within the scope of the disclosure. Additional asymmetric carbon atoms may be present in a substituent. All such isomers, as well as mixtures thereof, are intended to be included in this disclosure.

If, for instance, a particular enantiomer of compound of the disclosure is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as, *e.g.,* amino, or an acidic functional group, such as, *e.g.,* carboxyl, diastereomeric salts are formed with an appropriate optically-active acid or base, followed by resolution of the diastereomers thus formed by fractional crystallization or chromatographic means well known in the art, and subsequent recovery of the pure enantiomers.

Unless otherwise indicated, when a disclosed compound is named or depicted by a structure without specifying the stereochemistry and has one or more chiral centers, it is understood to represent all possible stereoisomers of the compound, as well as enantiomeric mixtures thereof.

The "enantiomeric excess" or "% enantiomeric excess" of a composition can be calculated using the equation shown below. In the example shown below, a composition contains 90% of one enantiomer, *e.g.,* the S enantiomer, and 10% of the other enantiomer, *i.e*., the *R* enantiomer. $ee = \left(90-10\right) / 100 = 80 % .$

Thus, a composition containing 90% of one enantiomer and 10% of the other enantiomer is said to have an enantiomeric excess of 80%.

The compounds or compositions described herein may contain an enantiomeric excess of at least 50%, 75%, 90%, 95%, or 99% of one form of the compound, *e.g.*, the *S*-enantiomer. In other words, such compounds or compositions contain an enantiomeric excess of the *S* enantiomer over the *R* enantiomer.

In one embodiment, the compounds described herein may also contain unnatural proportions of deuterium at one or more of the atoms that constitute such compounds. In addition, all tautomeric forms of the compounds described herein are intended to be within the scope of the disclosure.

The compounds disclosed herein can be useful in the form of a free base or as a salt. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, naphthylate, mesylate, glucoheptonate, lactobionate, and laurylsulphonate salts and the like. (*See, e.g.,* Berge et al. (1977) "Pharmaceutical Salts", J. Pharm. Sci. 66:1-19.)

Certain compounds disclosed herein can exist in unsolvated forms as well as solvated forms, including hydrated forms. As used herein, the term "hydrate" or "hydrated" refers to a compound formed by the union of water with the parent compound.

In general, the solvated forms are equivalent to unsolvated forms and are encompassed within the scope of the present disclosure. Certain compounds disclosed herein may exist in multiple crystalline or amorphous forms. In general, all physical forms are equivalent for the uses contemplated by the disclosure and are intended to be within the scope of the present disclosure.

The present disclosure provides compounds of Formula (I-0): 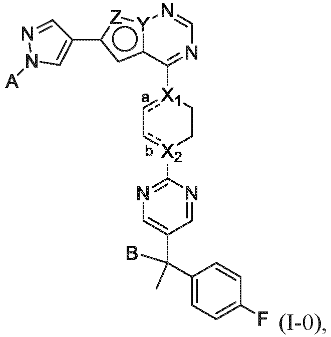 a pharmaceutically acceptable salt or a stereoisomer thereof, and/or a solvate of any of the foregoing, wherein:
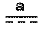 is chosen from a single bond and double bond;
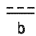 is chosen from a single bond and double bond;
Z is chosen from CH and NH;
Y is chosen from C and N;
X₁ is chosen from CH, C, and N;
X₂ is chosen from CH, C, and N;
   provided that when X₁ and X₂ are both N, then Y is not N and Z is not CH;
A is 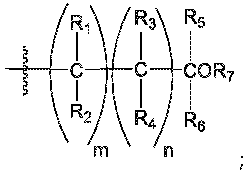
R₁ is chosen from hydrogen and methyl;
R₂ is chosen from hydrogen and methyl, or
R₁ and R₂ taken together form a cyclopropyl;
R₃ is chosen from hydrogen and methyl;
R₄ is chosen from hydrogen and methyl, or
R₃ and R₄ taken together form a cyclopropyl;
R₅ is chosen from hydrogen and methyl;
R₆ is chosen from hydrogen and methyl, or
R₅ and R₆ taken together form a cyclopropyl, or
one or R₂ or R₄ taken together with R₆ forms a cyclobutyl;
R₇ is hydrogen, or one of R₂, R₄, or R₆ taken together with R₇ forms a ring chosen from oxetane, tetrahydrofuran, and tetrahydropyran, wherein said tetrahydrofuran or tetrahydropyran is optionally substituted with hydroxyl;
m is 0 or 1;
n is 0 or 1; and
B is chosen from OH and NH₂, provided that the compound is not

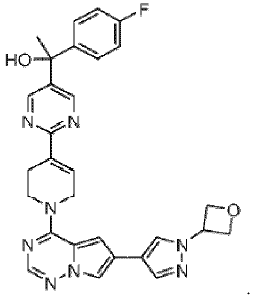

Compounds of Formula (I-0) in which R₆ taken with R₇ forms oxetane are not expressly recited in the appended claims and are not part of the present invention.

Therefore, alternatively, R₇ is hydrogen, or one of R₂, R₄, or R₆ taken together with R₇ forms a ring chosen from tetrahydrofuran and tetrahydropyran, wherein said tetrahydrofuran or tetrahydropyran is optionally substituted with hydroxyl.

Nonlimiting embodiments of the present disclosure include:
Embodiment 1. A compound of Formula (I): 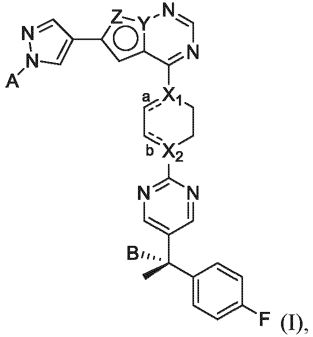 a pharmaceutically acceptable salt thereof, and/or a solvate of any of the foregoing, wherein:
   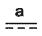 is chosen from a single bond and double bond;
   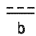 is chosen from a single bond and double bond;
   Z is chosen from CH and NH;
   Y is chosen from C and N;
   X₁ is chosen from CH, C, and N;
   X₂ is chosen from CH, C, and N;
      provided that when X₁ and X₂ are both N, then Y is not N and Z is not CH;
   A is 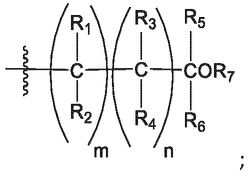
   R₁ is chosen from hydrogen and methyl;
   R₂ is chosen from hydrogen and methyl, or
   R₁ and R₂ taken together form a cyclopropyl;
   R₃ is chosen from hydrogen and methyl;
   R₄ is chosen from hydrogen and methyl, or
   R₃ and R₄ taken together form a cyclopropyl;
   R₅ is chosen from hydrogen and methyl;
   R₆ is chosen from hydrogen and methyl, or
   R₅ and R₆ taken together form a cyclopropyl, or
   one or R₂ or R₄ taken together with R₆ forms a cyclobutyl;
   R₇ is hydrogen, or one of R₂, R₄, or R₆ taken together with R₇ forms a ring chosen from oxetane, tetrahydrofuran, and tetrahydropyran, wherein said tetrahydrofuran or tetrahydropyran is optionally substituted with hydroxyl;
   m is 0 or 1;
   n is 0 or 1; and
   B is chosen from OH and NH₂.

Compounds of Formula I in which R₆ taken with R₇ forms oxetane are not expressly recited in the appended claims and are not part of the present invention.

Therefore, alternatively, R₇ is hydrogen, or one of R₂, R₄, or R₆ taken together with R₇ forms a ring chosen from tetrahydrofuran and tetrahydropyran, wherein said tetrahydrofuran or tetrahydropyran is optionally substituted with hydroxyl.

In one embodiment, the compound of Formula (I) is not a *S*-isomer of

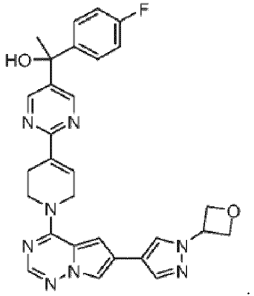

In some embodiments of embodiment 1, when m is 0, R₁ and R₂ are absent. In some embodiments of embodiment 1, when n is 0, R₃ and R₄ are absent. In some embodiments of embodiment 1, m + n = 1 or m and n cannot both be 0.

It is noted that in the present disclosure, when any two R groups (*e.g.,* R₁ and R₂) taken together form a ring structure (*e.g.,* a cyclopropyl), it is intended to include the intervening carbon atoms and/or the oxygen atom in the same ring structure.

Embodiment 2. The compound of embodiment 1 of Formula (II): 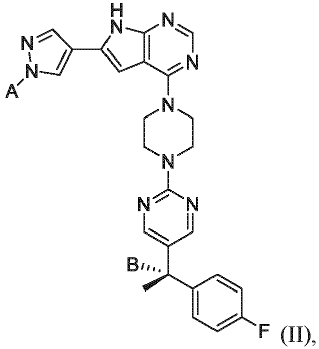 a pharmaceutically acceptable salt thereof, and/or a solvate of any of the foregoing. The definitions of variables A and B are provided in Formula (I-0) or Formula (I).

Embodiment 3. The compound of embodiment 1 of Formula (III): 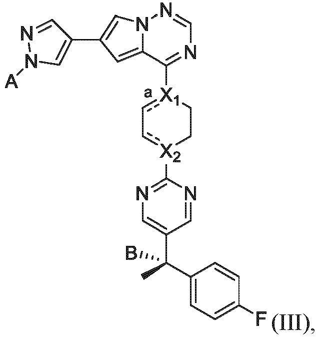 a pharmaceutically acceptable salt thereof, and/or a solvate of any of the foregoing, wherein
X₁ is chosen from CH, C, and N;
X₂ is chosen from CH, C, and N; and
provided that only one of X₁ and X₂ is N.

The definitions of variables A and B are provided in Formula (I-0) or Formula (I).

In one embodiment, the compound of Formula (III) is not a S-isomer of

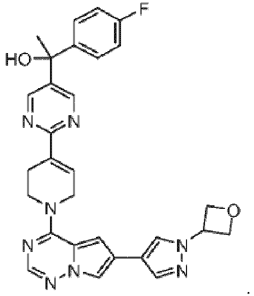

Embodiment 4. The compound of embodiment 3, a pharmaceutically acceptable salt thereof, and/or a solvate of any of the foregoing, wherein: X₁ is N and 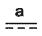 is a single bond and X₂ is C and 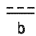 is a double-bond.

Embodiment 5. The compound of embodiment 3, a pharmaceutically acceptable salt thereof, and/or a solvate of any of the foregoing, wherein: X₁ is C and 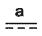 is a double bond and X₂ is N and 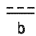 is a single bond.

Embodiment 6. The compound of any one of embodiments 1-5, a pharmaceutically acceptable salt thereof, and/or a solvate of any of the foregoing, wherein:
A is: 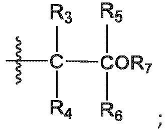
R₃ is chosen from hydrogen and methyl;
R₄ is chosen from hydrogen and methyl, or R₃ and R₄ taken together form a cyclopropyl;
R₅ is chosen from hydrogen and methyl;
R₆ is chosen from hydrogen and methyl; or
R₅ and R₆ taken together form a cyclopropyl,
and R₇ is hydrogen.

Embodiment 7. The compound of any one of embodiments 1-5, a pharmaceutically acceptable salt thereof, and/or a solvate of any of the foregoing, wherein:
A is 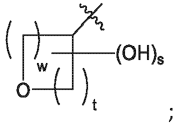
w is 1 or 2;
t is 1 or 2; and
s is 0 or 1 is not expressly recited in the appended claims and is not part of the present invention.

Embodiment 8. The compound of any one of embodiments 1-7, a pharmaceutically acceptable salt thereof, and/or a solvate of any of the foregoing, wherein B is NH₂.

Embodiment 9. The compound of any one of embodiments 1-7, a pharmaceutically acceptable salt thereof, and/or a solvate of any of the foregoing, wherein B is OH.

Embodiment 10. The compound of any one of embodiments 1-9, a pharmaceutically acceptable salt thereof, and/or a solvate of any of the foregoing, wherein the compound has a Kₚ < 0.4.

In some embodiments of embodiment 10 the compound has a Kₚ < 0.4 as measured according to the procedure described in Biological Example 3. In some embodiments of embodiment 10, the compound, a pharmaceutically acceptable salt thereof, and/or a solvate of any of the foregoing is chosen from compounds 1 and 2.

Embodiment 11. The compound of any one of embodiments 1-9, a pharmaceutically acceptable salt thereof, and/or a solvate of any of the foregoing, wherein the compound has a Kₚ ≤ 0.30.

In some embodiments of embodiment 11, the compound has a Kₚ ≤ 0.30 as measured according to the procedure described in Biological Example 3. In some embodiments of embodiment 11, the compound, a pharmaceutically acceptable salt thereof, and/or a solvate of any of the foregoing is chosen from compounds 1 and 2.

Embodiment 12. The compound of any one of embodiments 1-9, a pharmaceutically acceptable salt thereof, and/or a solvate of any of the foregoing, wherein the compound has a Kₚ ≤ 0.20.

In some embodiments of embodiment 12, the compound has a Kₚ ≤ 0.20 as measured according to the procedure described in Biological Example 3. In some embodiments of embodiment 12, the compound, a pharmaceutically acceptable salt thereof, and/or a solvate of any of the foregoing is chosen from compounds 1 and 2.

Embodiment 13. The compound of any one of embodiments 1-9, a pharmaceutically acceptable salt thereof, and/or a solvate of any of the foregoing, wherein the compound has a Kₚ ≤ 0.10.

In some embodiments of embodiment 13, the compound has a Kₚ ≤ 0.10 as measured according to the procedure described in Biological Example 3. In some embodiments of embodiment 13, the compound, a pharmaceutically acceptable salt thereof, and/or a solvate of any of the foregoing is chosen from compound 2.

Embodiment 14. The compound of any one of embodiments 1-13, a pharmaceutically acceptable salt thereof, and/or a solvate of any of the foregoing, wherein the compound has a K_{p, uu} ≤ 0.2 in homogenate rat brain.

In some embodiments of embodiment 14, the compound has a K_{p, uu} ≤ 0.2 in homogenate rat brain as measured according to the procedure described in Biological Example 3. In some embodiments of embodiment 14, the compound, a pharmaceutically acceptable salt thereof, and/or a solvate of any of the foregoing is chosen from compounds 1 and 2.

Embodiment 14-1.The compound of any one of embodiments 1-13, a pharmaceutically acceptable salt thereof, and/or a solvate of any of the foregoing, wherein the compound has a K_{p, uu} < 0.1 in homogenate rat brain.

In some embodiments of embodiment 14-1, the compound has a K_{p, uu} < 0.1 in homogenate rat brain as measured according to the procedure described in Biological Example 3. In some embodiments of embodiment 14-1, the compound, a pharmaceutically acceptable salt thereof, and/or a solvate of any of the foregoing is chosen from compounds 1 and 2.

Embodiment 14-2.The compound of any one of embodiments 1-13, a pharmaceutically acceptable salt thereof, and/or a solvate of any of the foregoing, wherein the compound has a K_{p, uu} ≤ 0.05 in homogenate rat brain.

In some embodiments of embodiment 14-2, the compound has a K_{p, uu} ≤ 0.05 in homogenate rat brain as measured according to the procedure described in Biological Example 3. In some embodiments of embodiment 14-2, the compound, a pharmaceutically acceptable salt thereof, and/or a solvate of any of the foregoing is chosen from compounds 1 and 2.

Embodiment 14-3.The compound of any one of embodiments 1-13, a pharmaceutically acceptable salt thereof, and/or a solvate of any of the foregoing, wherein the compound has a K_{p, uu} ≤ 0.02 in rat brain slice.

In some embodiments of embodiment 14-3, the compound has a K_{p, uu} ≤ 0.02 in rat brain slice as measured in according to the procedure described in Biological Example 3. In some embodiments of embodiment 14-3 the compound, a pharmaceutically acceptable salt thereof, and/or a solvate of any of the foregoing is chosen from compounds 1 and 2.

Embodiment 15. The compound of any one of embodiments 1-14, a pharmaceutically acceptable salt thereof, and/or a solvate of any of the foregoing, wherein the compound has an unbound clearance (Clᵤ) in rat of < 900 mL/min/kg.

Embodiment 16. The compound of any one of embodiments 1-14, a pharmaceutically acceptable salt thereof, and/or a solvate of any of the foregoing, wherein the compound has an unbound clearance (Clᵤ) in rat of < 750 mL/min/kg.

Embodiment 17. The compound of any one of embodiments 1-14, a pharmaceutically acceptable salt thereof, and/or a solvate of any of the foregoing, wherein the compound has an IC₅₀ for CYP3A4 of < 10 µM.

Embodiment 18. The compound of any one of embodiments 1-5, 8, and 9, a pharmaceutically acceptable salt thereof, and/or a solvate of any of the foregoing, wherein A is chosen from

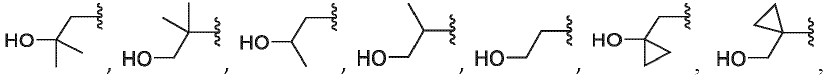

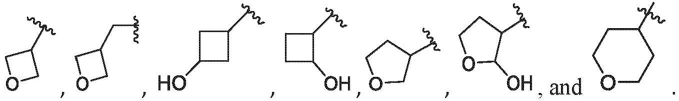

Embodiment 19. The compound of any one of embodiments 1-5, 8, and 9, a pharmaceutically acceptable salt thereof, and/or a solvate of any of the foregoing, wherein A is chosen from 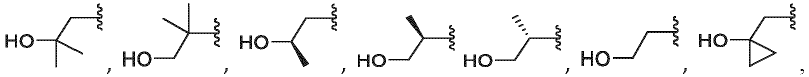 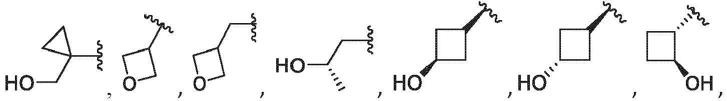 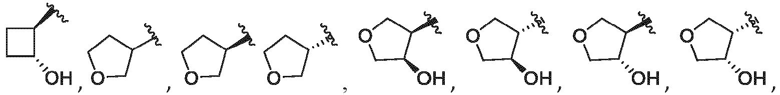 and

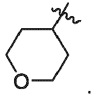

Embodiment 20. The compound of any one of embodiments 1-5, 8, and 9, a pharmaceutically acceptable salt thereof, and/or a solvate of any of the foregoing, wherein A is chosen from

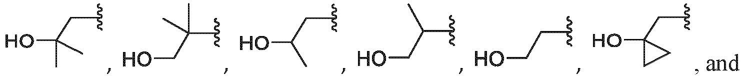

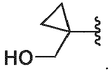

Embodiment 21. The compound of any one of embodiments 1-5, 8, and 9, a pharmaceutically acceptable salt thereof, and/or a solvate of any of the foregoing, wherein A is chosen from

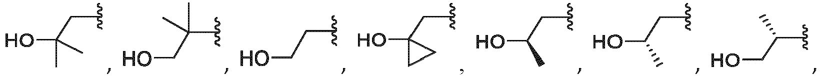

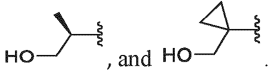

Embodiment 22. The compound of any one of embodiments 1-5, 8, and 9, a pharmaceutically acceptable salt thereof, and/or a solvate of any of the foregoing, wherein A is chosen from

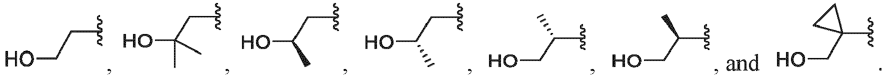

Embodiment 23. The compound of any one of embodiments 1-5, 8, and 9, a pharmaceutically acceptable salt thereof, and/or a solvate of any of the foregoing, wherein A is chosen from

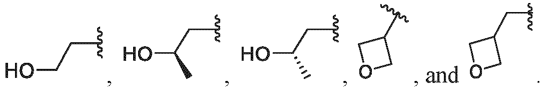

Embodiment 24. The compound of any one of embodiments 1-5, 8, and 9, a pharmaceutically acceptable salt thereof, and/or a solvate of any of the foregoing, wherein A is chosen from

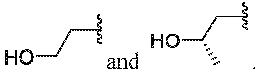

Embodiment 25. The compound of any one of embodiments 1-5, 8, and 9, a pharmaceutically acceptable salt thereof, and/or a solvate of any of the foregoing, wherein the compound is any one of the compounds listed in Table 1 below.

In some embodiments of embodiment 25, the compound is any one of compounds 1-7, 13, 14, 14-A, 28, 33, and 38. In some embodiments of embodiment 25, the compound is any one of compounds 1-7, 13, 14, 28, 33, and 38. In some embodiments of embodiment 25, the compound is any one of compounds 1-6. In some embodiments of embodiment 25, the compound is any one of compounds 1 and 2.

Embodiment 26. A pharmaceutical composition comprising:
a compound of any one of embodiments 1-25, a pharmaceutically acceptable salt thereof, and/or a solvate of any of the foregoing; and
a pharmaceutically acceptable excipient.

Embodiment 27. A method of treating a disease or condition in a patient in need thereof, wherein the method comprises administering to the patient a compound according to any one of embodiments 1-25, a pharmaceutically acceptable salt thereof, and/or a solvate of any of the foregoing, wherein the disease or condition is chosen from systemic mastocytosis, gastrointestinal stromal tumors, acute myeloid leukemia, melanoma, seminoma, intracranial germ cell tumors, mediastinal B-cell lymphoma, Ewing's sarcoma, diffuse large B cell lymphoma, dysgerminoma, myelodysplastic syndrome, nasal NK/T-cell lymphoma, chronic myelomonocytic leukemia, and brain cancer.

Embodiment 28. A method of treating a disease or condition mediated by mutant KIT or PDGFRα in a patient in need thereof, wherein the method comprises administering to the patient a compound according to any one of embodiments 1-25, a pharmaceutically acceptable salt thereof, and/or a solvate of any of the foregoing.

Embodiment 29. The method of embodiment 28, wherein the disease or condition is chosen from systemic mastocytosis, gastrointestinal stromal tumors, acute myeloid leukemia, melanoma, seminoma, intracranial germ cell tumors, mediastinal B-cell lymphoma, Ewing's sarcoma, diffuse large B cell lymphoma, dysgerminoma, myelodysplastic syndrome, nasal NK/T-cell lymphoma, chronic myelomonocytic leukemia, and brain cancer.

Embodiment 30. A compound according to any one of embodiments 1-25, a pharmaceutically acceptable salt thereof, and/or a solvate of any of the foregoing for use as a medicament for treating a disease or condition in a patient in need thereof, wherein the disease or condition is chosen from systemic mastocytosis, gastrointestinal stromal tumors, acute myeloid leukemia, melanoma, seminoma, intracranial germ cell tumors, mediastinal B-cell lymphoma, Ewing's sarcoma, diffuse large B cell lymphoma, dysgerminoma, myelodysplastic syndrome, nasal NK/T-cell lymphoma, chronic myelomonocytic leukemia, and brain cancer.

Embodiment 31. A compound according to any one of embodiments 1-25, a pharmaceutically acceptable salt thereof, and/or a solvate of any of the foregoing for use as a medicament for treating a disease or condition mediated by mutant KIT or PDGFRA in a patient in need thereof.

Embodiment 32. The compound of embodiment 31, wherein the disease or condition is chosen from systemic mastocytosis, gastrointestinal stromal tumors, acute myeloid leukemia, melanoma, seminoma, intracranial germ cell tumors, mediastinal B-cell lymphoma, Ewing's sarcoma, diffuse large B cell lymphoma, dysgerminoma, myelodysplastic syndrome, nasal NK/T-cell lymphoma, chronic myelomonocytic leukemia, and brain cancer.

Embodiment 33. A method of treating an eosinophilic disorder, comprising administering to a subject in need thereof a therapeutically effective amount of a compound according to any one of embodiments 1-25, a pharmaceutically acceptable salt thereof, and/or a solvate of any of the foregoing.

Embodiment 34. The method of embodiment 33, wherein the eosinophilic disorder is selected from hypereosinophilic syndrome, eosinophilia, eosinophilic enterogastritis, eosinophilic leukemia, eosinophilic granuloma and Kimura's disease.

Embodiment 35. The method of embodiment 33, wherein the eosinophilic disorder is hypereosinophilic syndrome.

Embodiment 36. The method of embodiment 33, wherein the eosinophilic disorder is eosinophilic leukemia.

Embodiment 37. The method of embodiment 36, wherein the eosinophilic leukemia is chronic eosinophilic leukemia.

Embodiment 38. The method of any one of embodiments 33-37, wherein the eosinophilic disorder is refractory to treatment with imatinib, sunitinib, and/or regorafenib.

Embodiment 39. A compound according to any one of embodiments 1-25, a pharmaceutically acceptable salt thereof, and/or a solvate of any of the foregoing for use as a medicament for treating an eosinophilic disorder.

Embodiment 40. The compound of embodiment 39, wherein the eosinophilic disorder is selected from hypereosinophilic syndrome, eosinophilia, eosinophilic enterogastritis, eosinophilic leukemia, eosinophilic granuloma and Kimura's disease.

Embodiment 41. The compound of embodiment 39, wherein the eosinophilic disorder is hypereosinophilic syndrome.

Embodiment 42. The compound of embodiment 39, wherein the eosinophilic disorder is eosinophilic leukemia.

Embodiment 43. The compound of embodiment 42, wherein the eosinophilic leukemia is chronic eosinophilic leukemia.

Embodiment 44. The method of any one of embodiments 39-43, wherein the eosinophilic disorder is refractory to treatment with imatinib, sunitinib, and/or regorafenib.

Embodiment 45. A method of treating a mast cell disorder, comprising administering to a subject in need thereof a therapeutically effective amount of a compound according to any one of embodiments 1-25, a pharmaceutically acceptable salt thereof, and/or solvate of any of the foregoing.

Embodiment 46. The method of embodiment 45, wherein the mast cell disorder is mediated by mutant KIT or PDGFRα.

Embodiment 46-1. The method of embodiment 45, wherein the mast cell disorder is mediated by wild type KIT or PDGFRα.

Embodiment 47. The method of any one of embodiments 46, wherein the mast cell disorder is selected from mast cell activation syndrome (MCAS) and hereditary alpha tryptasemia (HAT).

Embodiment 48. The method of embodiment 47, wherein the MCAS is selected from monoclonal mast cell activation syndrome (MMAS), secondary MCAS, and idiopathic MCAS.

Embodiment 48-1. The method of embodiment 27, wherein the disease or condition is systemic mastocytosis.

Embodiment 49. The method of any one of embodiments 48, wherein the systemic mastocytosis is chosen from indolent systemic mastocytosis and smoldering systemic mastocytosis.

It is noted that in the present disclosure, any one of the embodiments identified above is intended to include the corresponding sub-embodiments. For example, when embodiment 14 is referred, it is intended to include embodiment 14, embodiment 14-1, embodiment 14-2, embodiment 14-3, and the specific embodiments recited therein.

**Table 1 lists the compounds prepared by the synthetic methods described herein.**

| **No.** | **Chemical Structure** | **Synthetic Procedure Prep No., Example No.** |
|---|---|---|
| 1 | 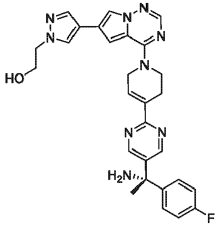 | 1, Ex1 |
| 2 | 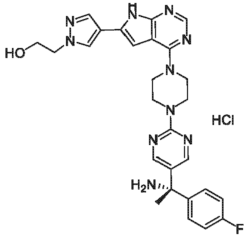 | 2, Ex 2 |
| Comparative 3 | 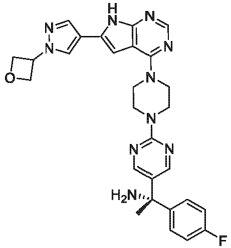 | 2, Comparative Ex 3 |
| 4 | 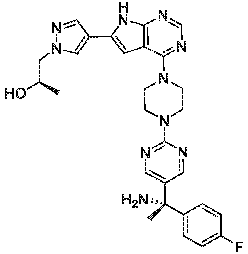 | 2, Ex 4 |
| 5 | 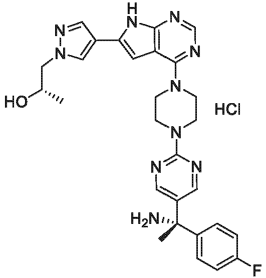 | 2, Ex 5 |
| Comparative 6 | 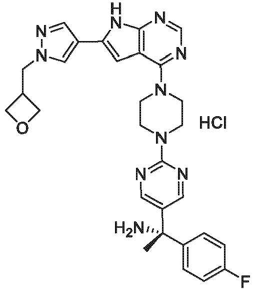 | 2, Comparative Ex 6 |
| 7 | 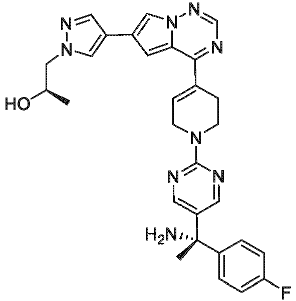 | 3, Ex 4 |
| 8 | 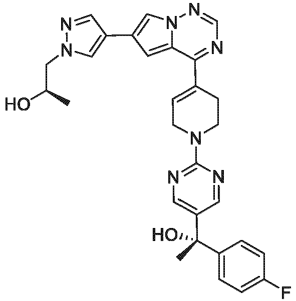 | 3, Ex 4 |
| 9 | 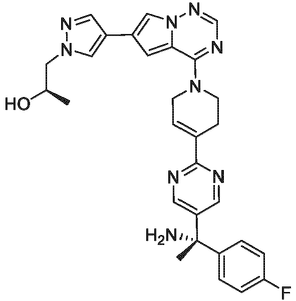 | 1, Ex 4 |
| 10 | 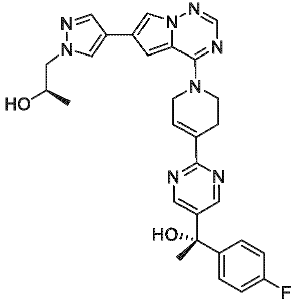 | 1, Ex 4 |
| 11 | 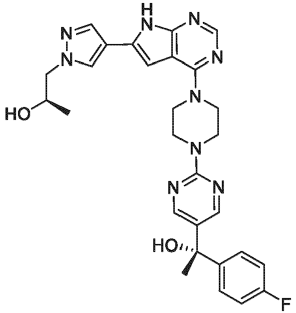 | 2, Ex 4 |
| 12 | 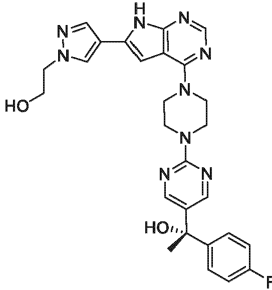 | 2, Ex 2 |
| 13 | 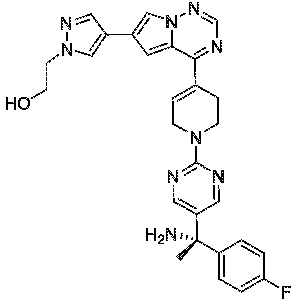 | 3, Ex 2 |
| 14 | 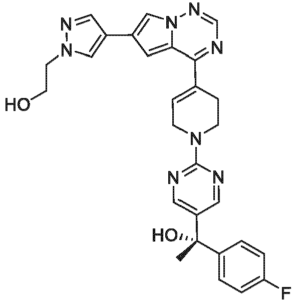 | 3, Ex 2 |
| 14-A | 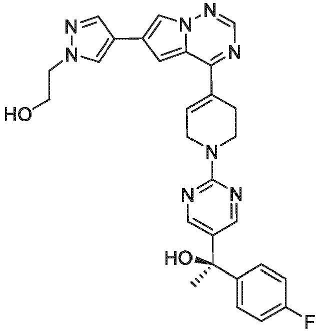 | 3, Ex 2 |
| 15 | 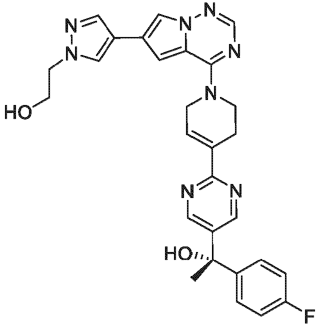 | 1, Ex 2 |
| 16 | 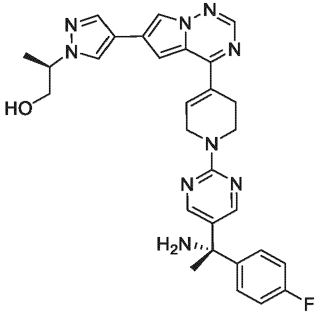 | 3, Ex 9 |
| 17 | 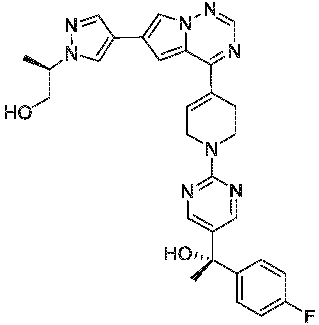 | 3, Ex 9 |
| 18 | 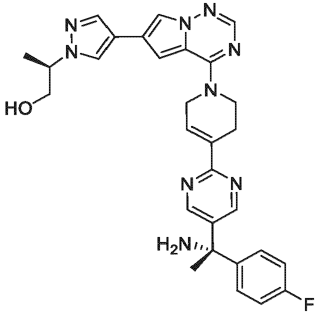 | 1, Ex 9 |
| 19 | 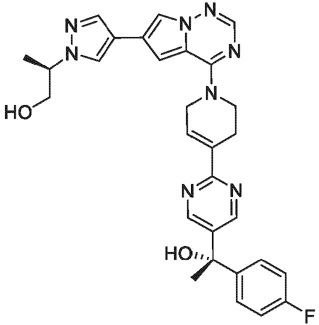 | 1, Ex 9 |
| 20 | 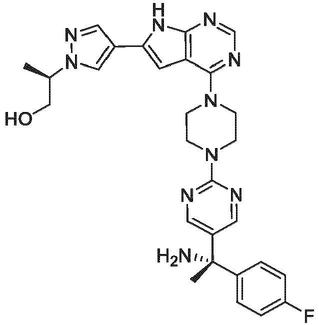 | 2, Ex 9 |
| 21 | 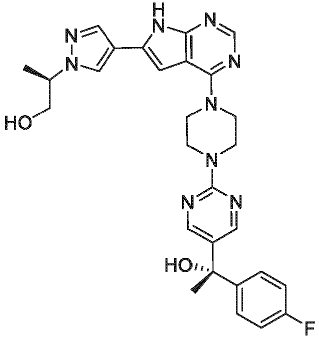 | 2, Ex 9 |
| 22 | 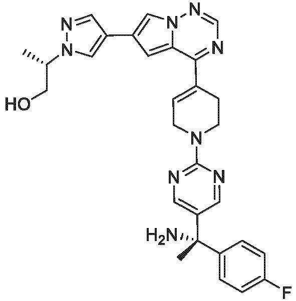 | 3, Ex 10 |
| 23 | 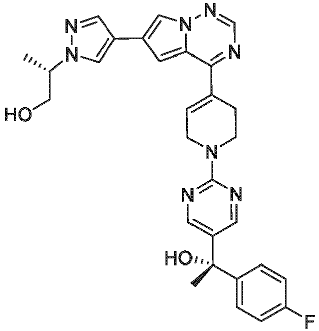 | 3, Ex 10 |
| 24 | 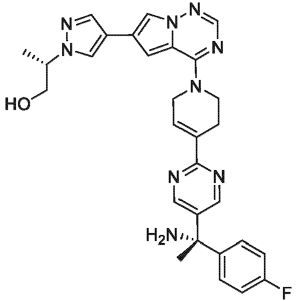 | 1, Ex 10 |
| 25 | 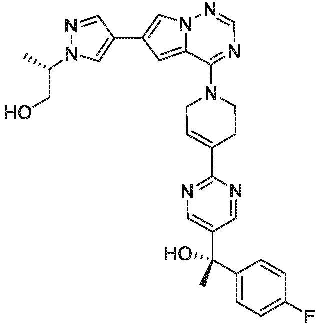 | 1, Ex 10 |
| 26 | 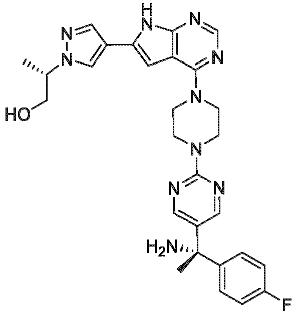 | 2, Ex 10 |
| 27 | 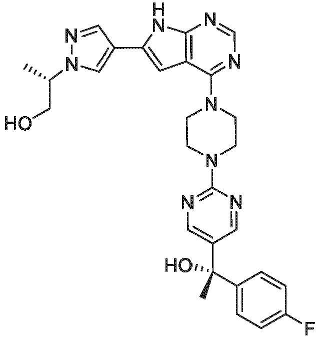 | 2, Ex 10 |
| Comparative 28 | 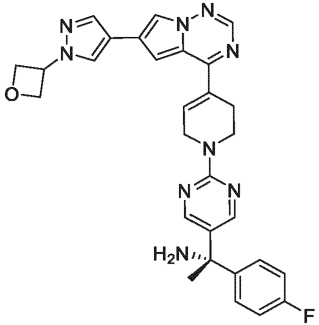 | 3, Comparative Ex 3 |
| Comparative 29 | 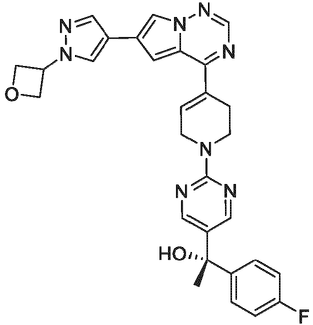 | 3, Comparative Ex 3 |
| Comparative 30 | 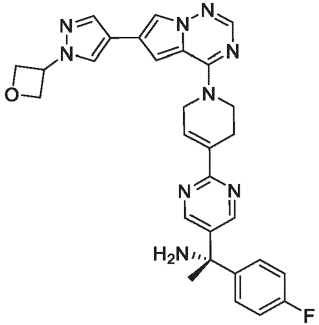 | 1, Comparative Ex 3 |
| Comparative 31 | 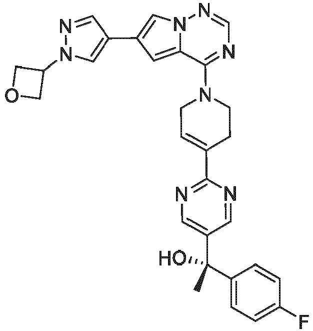 | 1, Comparative Ex 3 |
| Comparative 32 | 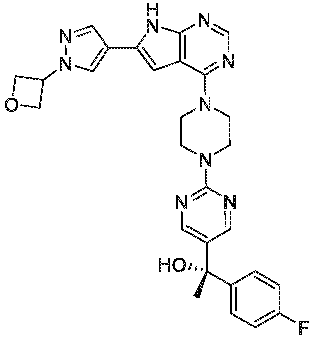 | 2, Comparative Ex 3 |
| Comparative 33 | 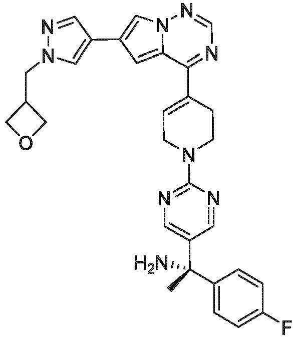 | 3, Comparative Ex 6 |
| Comparative 34 | 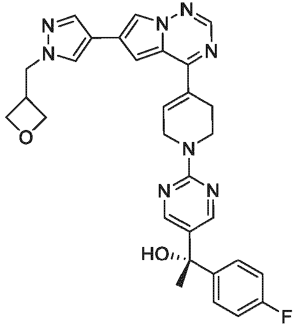 | 3, Comparative Ex 6 |
| Comparative 35 | 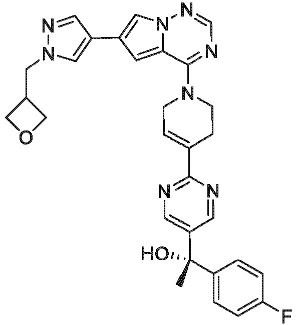 | 1, Comparative Ex 6 |
| Comparative 36 | 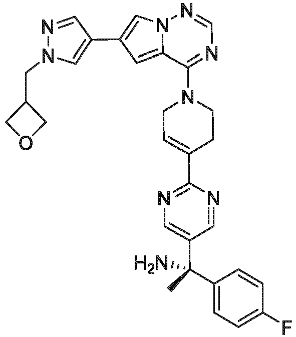 | 1, Comparative Ex 6 |
| Comparative 37 | 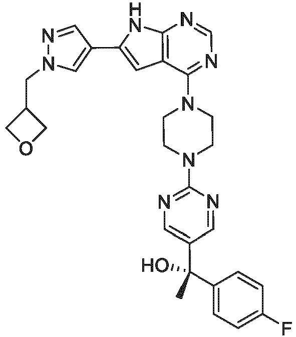 | 2, Comparative Ex 6 |
| 38 | 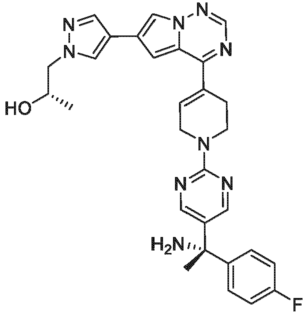 | 3, Ex 5 |
| 39 | 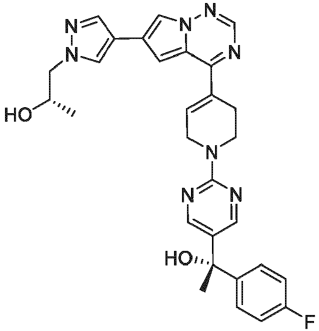 | 3, Ex 5 |
| 40 | 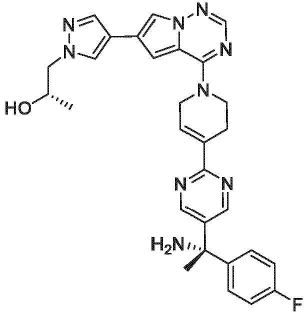 | 1, Ex 5 |
| 41 | 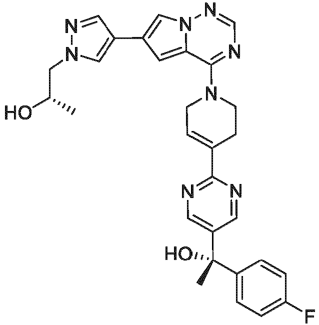 | 1, Ex 5 |
| 42 | 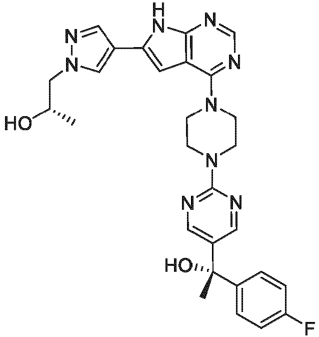 | 2, Ex 5 |
| 43 | Intentionally left blank | |
| 44 | 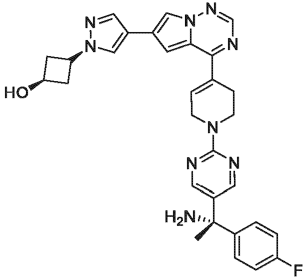 | 3, Ex 11 |
| 45 | 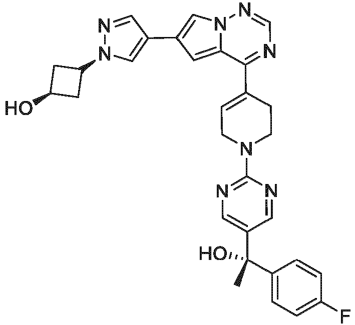 | 3, Ex 11 |
| 46 | 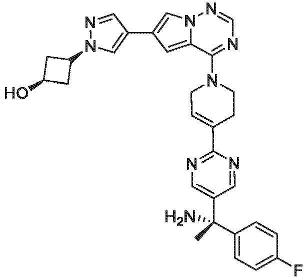 | 1, Ex 11 |
| 47 | 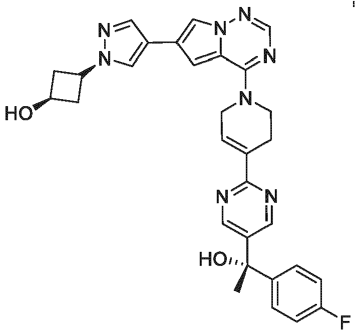 | 1, Ex 11 |
| 48 | 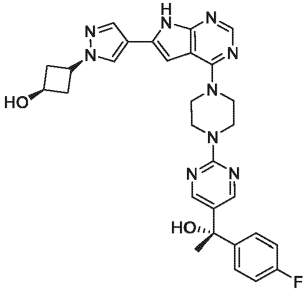 | 2, Ex 11 |
| 49 | 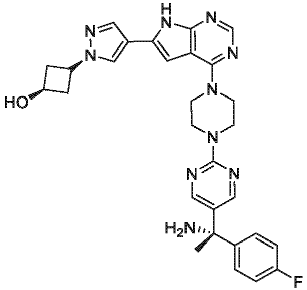 | 2, Ex 11 |
| 50 | 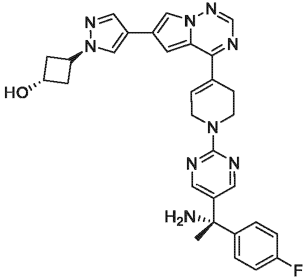 | 1, Ex 12 |
| 51 | 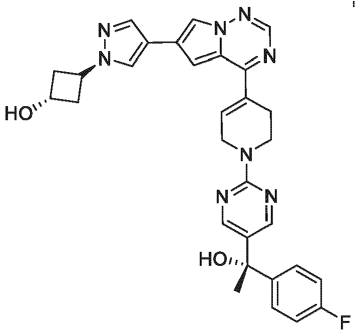 | 1, Ex 12 |
| 52 | 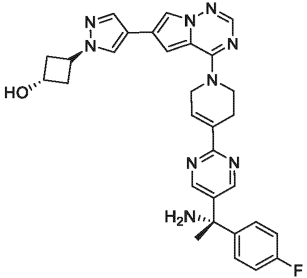 | 1, Ex 12 |
| 53 | 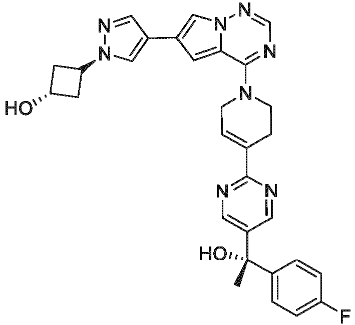 | 1, Ex 12 |
| 54 | 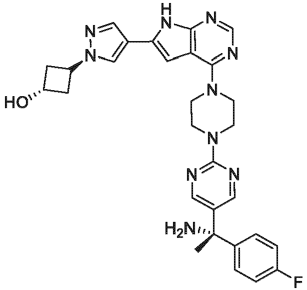 | 2, Ex 12 |
| 55 | 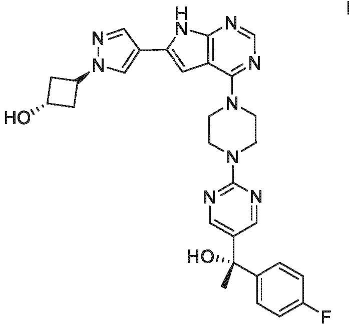 | 2, Ex 12 |
| 56 | 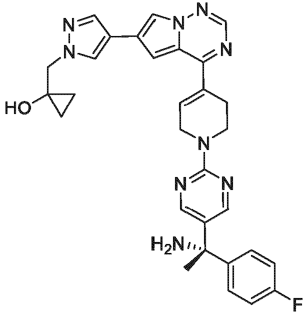 | 3, Ex 13 |
| 57 | 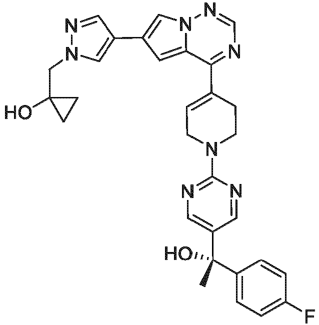 | 3, Ex 13 |
| 58 | 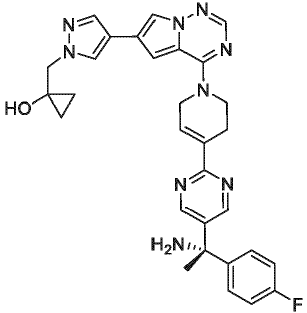 | 1, Ex 13 |
| 59 | Intentionally left blank | |
| 60 | 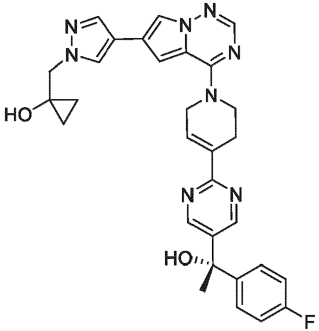 | 1, Ex 13 |
| 61 | 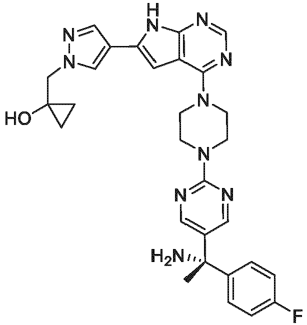 | 2, Ex 13 |
| 62 | 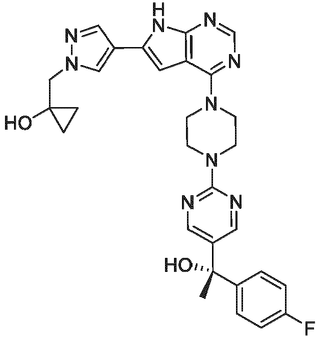 | 2, Ex 13 |
| 63 | 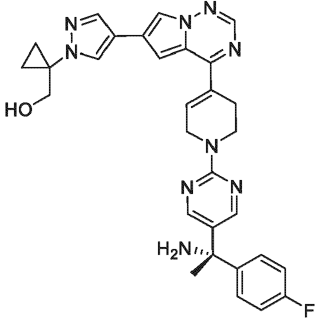 | 3, Ex 14 |
| 64 | 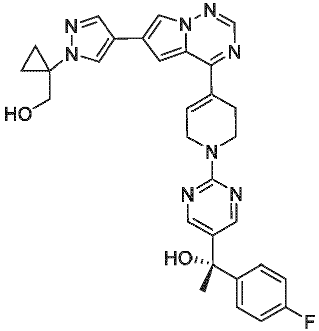 | 3, Ex 14 |
| 65 | 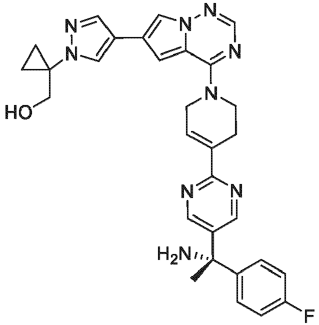 | 1, Ex 14 |
| 66 | 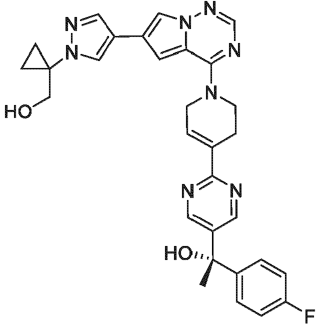 | 1, Ex 14 |
| 67 | 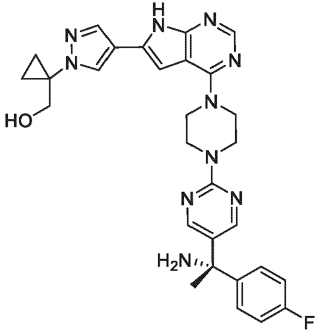 | 2, Ex 14 |
| 68 | 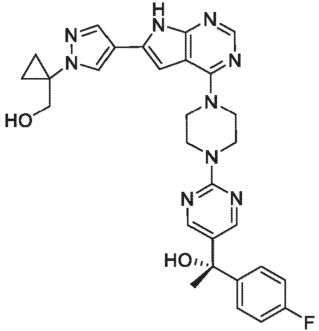 | 2, Ex 14 |
| 69 | 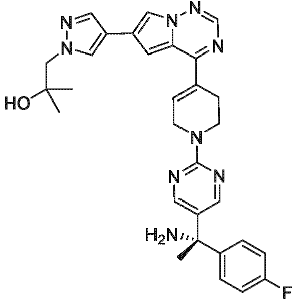 | 3, Ex 7 |
| 70 | 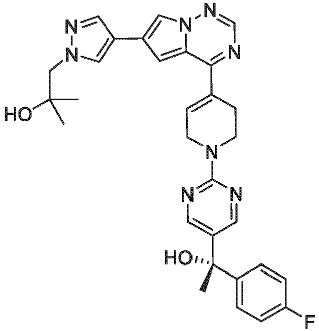 | 3, Ex 7 |
| 71 | 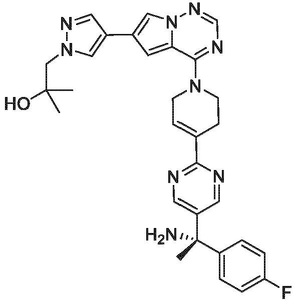 | 1, Ex 7 |
| 72 | 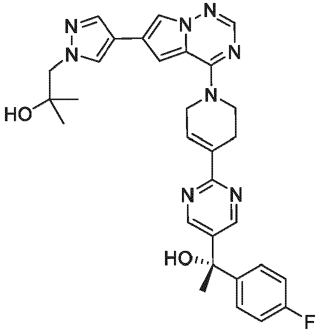 | 1, Ex 7 |
| 73 | 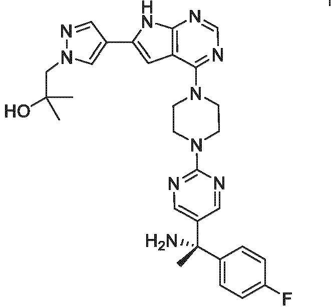 | 2, Ex 7 |
| 74 | 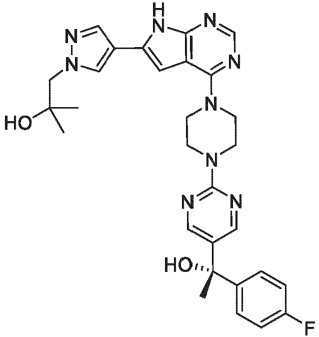 | 2, Ex 7 |
| 75 | 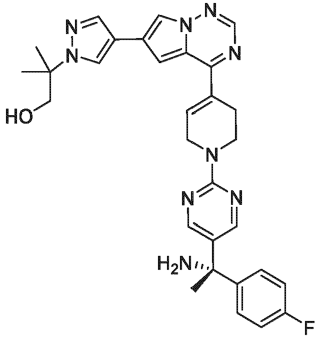 | 3, Ex 8 |
| 76 | 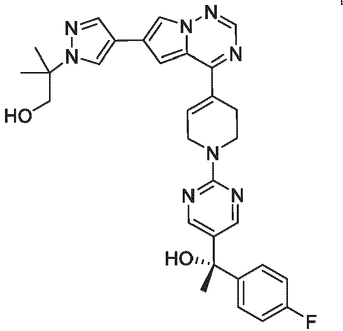 | 3, Ex 8 |
| 77 | 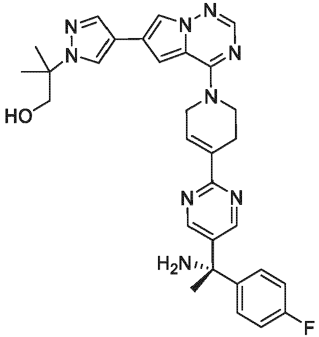 | 1, Ex 8 |
| 78 | 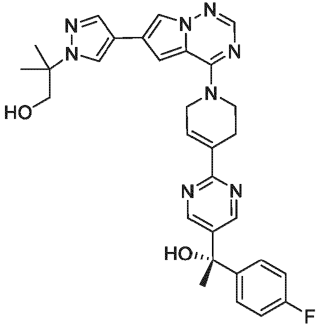 | 1, Ex 8 |
| 79 | 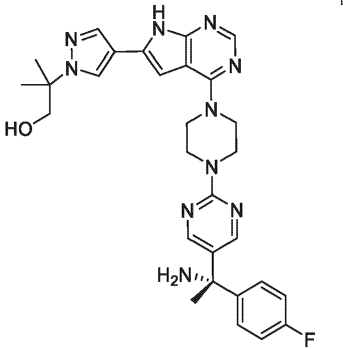 | 2, Ex 8 |
| 80 | 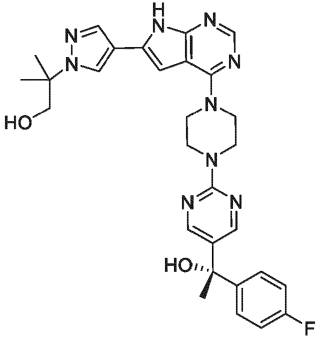 | 2, Ex 8 |
| 81 | 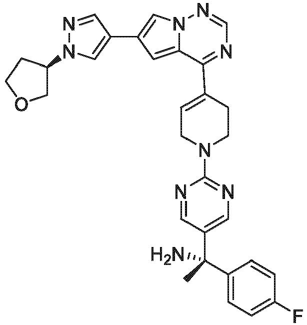 | 3, Ex 15 |
| 82 | 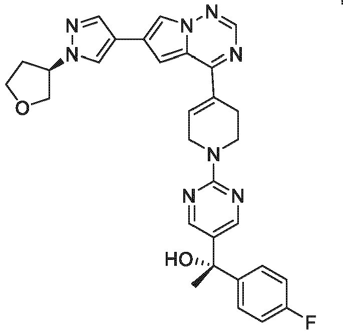 | 3, Ex 15 |
| 83 | 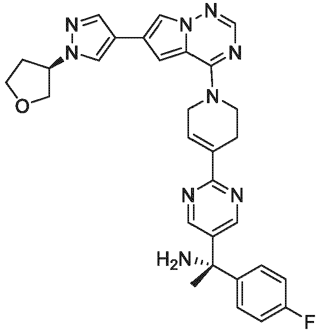 | 1, Ex 15 |
| 84 | 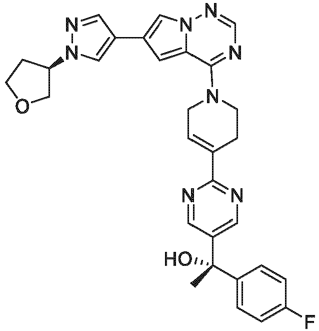 | 1, Ex 15 |
| 85 | 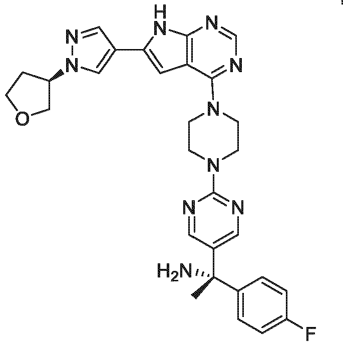 | 2, Ex 15 |
| 86 | 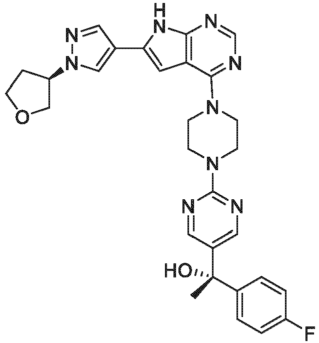 | 2, Ex 15 |
| 87 | 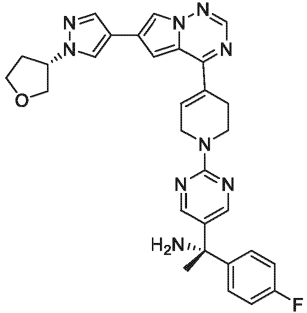 | 3, Ex 16 |
| 88 | 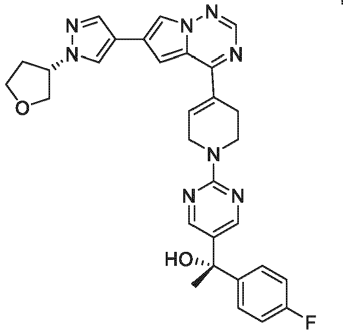 | 3, Ex 16 |
| 89 | 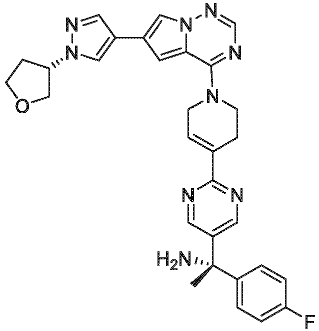 | 1, Ex 16 |
| 90 | 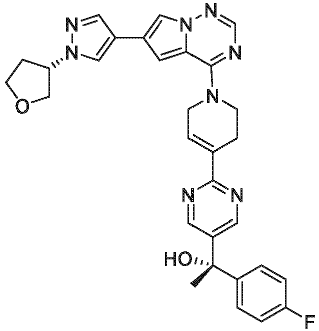 | 1, Ex 16 |
| 91 | 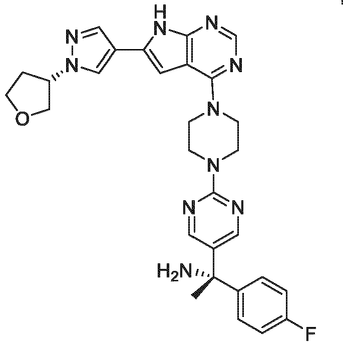 | 2, Ex 16 |
| 92 | 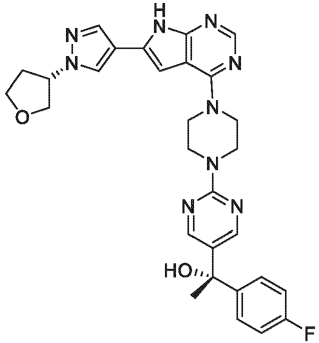 | 2, Ex 16 |
| 93 | 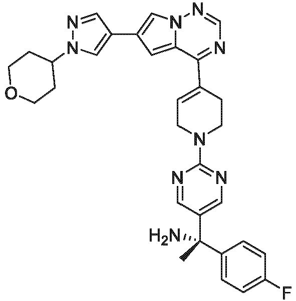 | 3, Ex 17 |
| 94 | 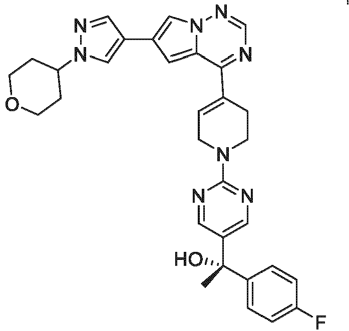 | 3, Ex 17 |
| 95 | 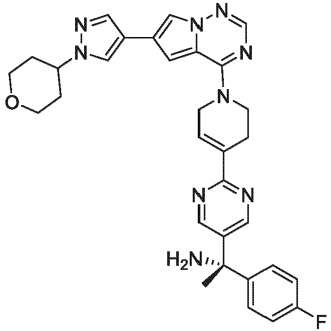 | 1, Ex 17 |
| 96 | 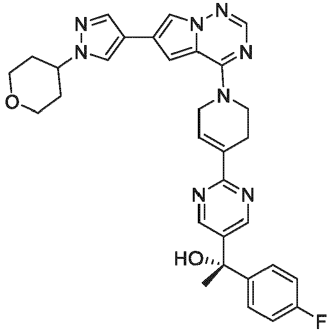 | 1, Ex 17 |
| 97 | 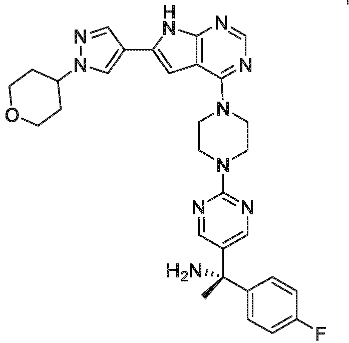 | 2, Ex 17 |
| 98 | 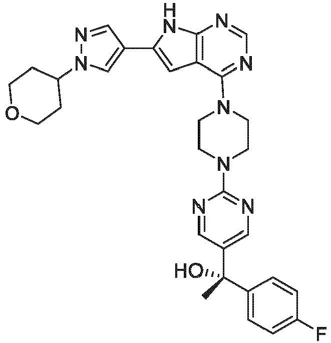 | 2, Ex 17 |
| 99 | 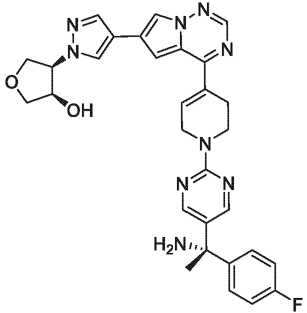 | 3, Ex 18 |
| 100 | 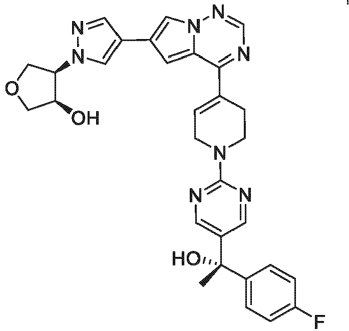 | 3, Ex 18 |
| 101 | 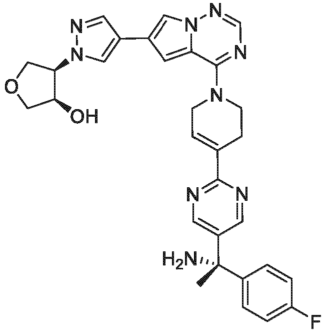 | 1, Ex 18 |
| 102 | 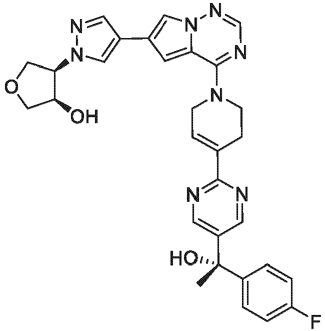 | 1, Ex 18 |
| 103 | 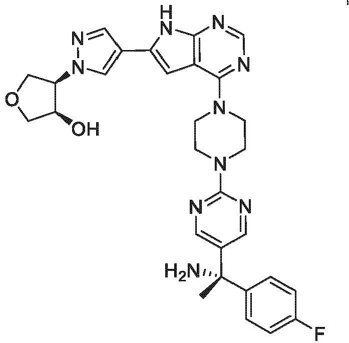 | 2, Ex 18 |
| 104 | 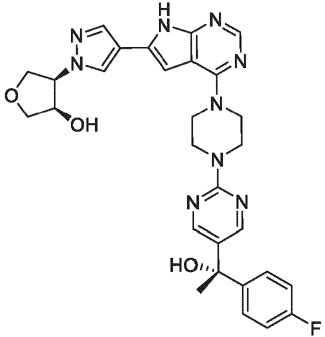 | 2, Ex 18 |
| 105 | 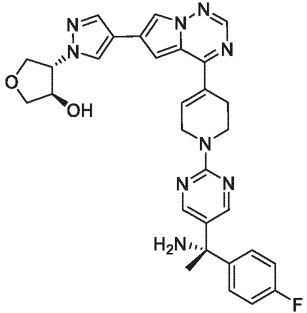 | 3, Ex 19 |
| 106 | 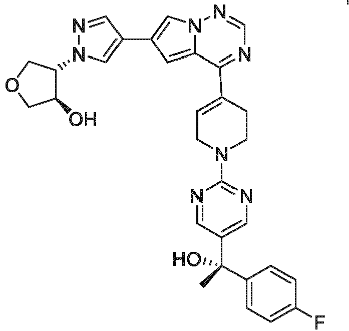 | 3, Ex 19 |
| 107 | 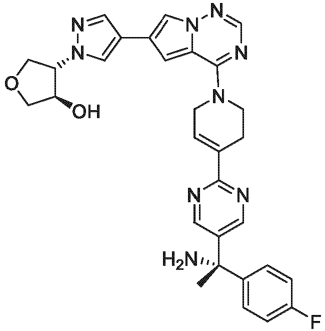 | 1, Ex 19 |
| 108 | 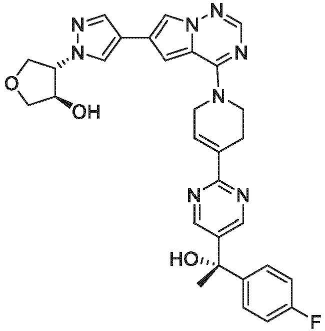 | 1, Ex 19 |
| 109 | 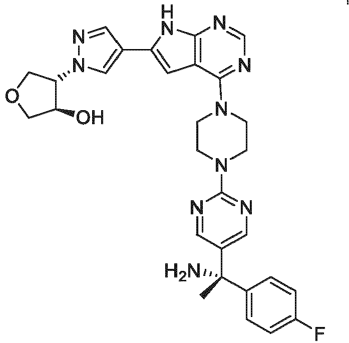 | 2, Ex 19 |
| 110 | 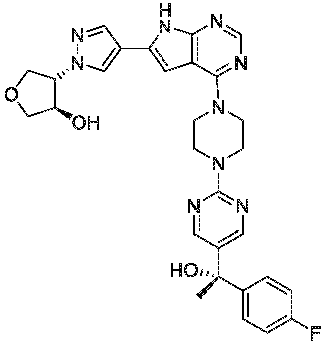 | 2, Ex 19 |
| 111 | 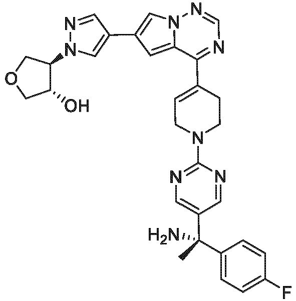 | 3, Ex 20 |
| 112 | 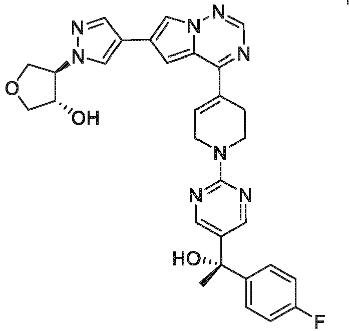 | 3, Ex 20 |
| 113 | 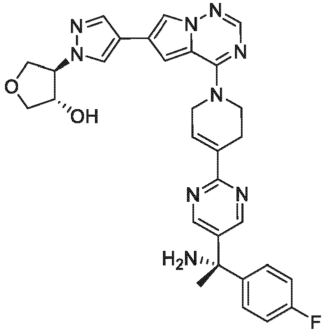 | 1, Ex 20 |
| 114 | 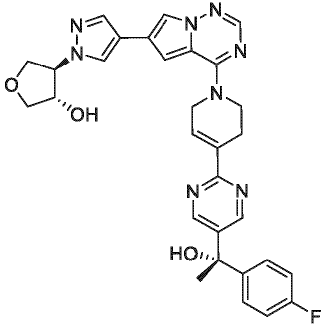 | 1, Ex 20 |
| 115 | 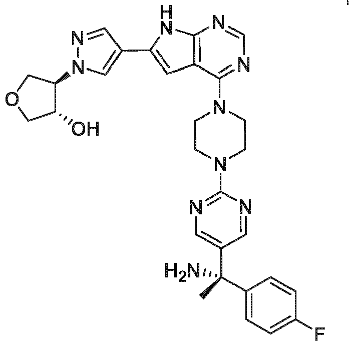 | 2, Ex 20 |
| 116 | 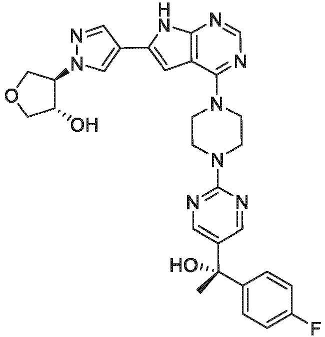 | 2, Ex 20 |
| 117 | 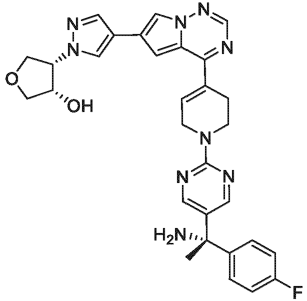 | 3, Ex 21 |
| 118 | 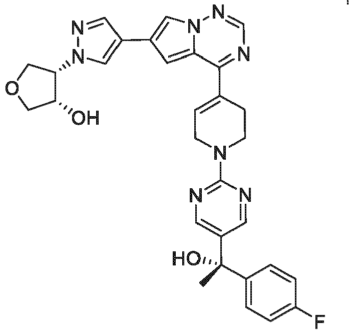 | 3, Ex 21 |
| 119 | 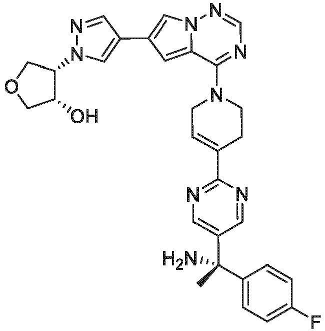 | 1, Ex 21 |
| 120 | 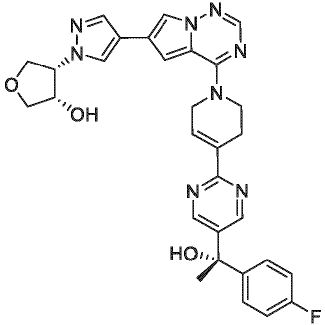 | 1, Ex 21 |
| 121 | 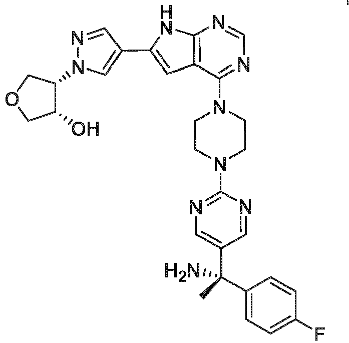 | 2, Ex 21 |
| 122 | 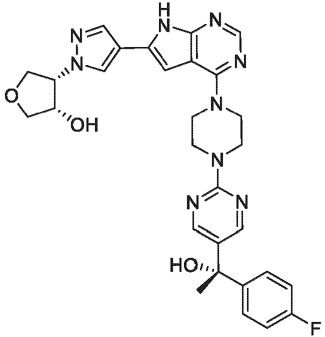 | 2, Ex 21 |
| 123 | 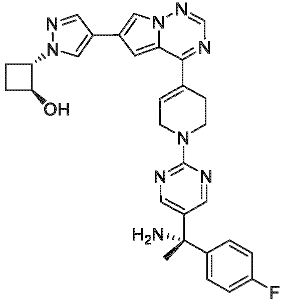 | 3, Ex 22 |
| 124 | 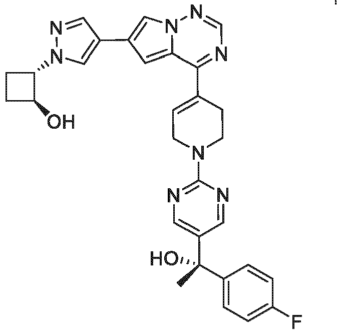 | 3, Ex 22 |
| 125 | 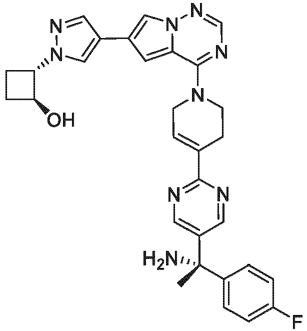 | 1, Ex 22 |
| 126 | 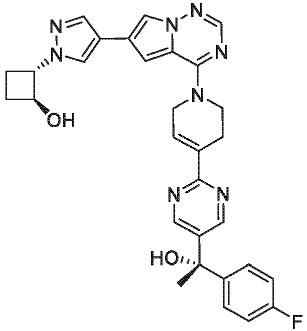 | 1, Ex 22 |
| 127 | 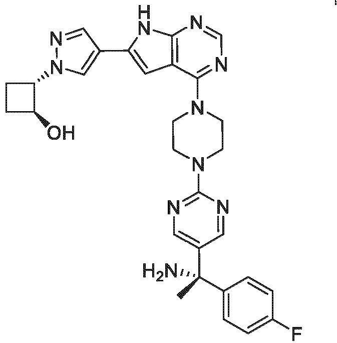 | 2, Ex 22 |
| 128 | 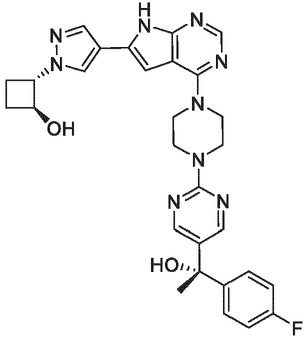 | 2, Ex 22 |
| 129 | 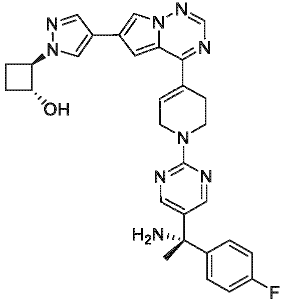 | 3, Ex 23 |
| 130 | 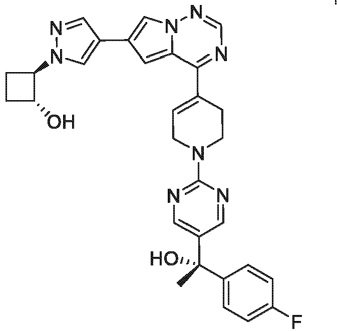 | 3, Ex 23 |
| 131 | 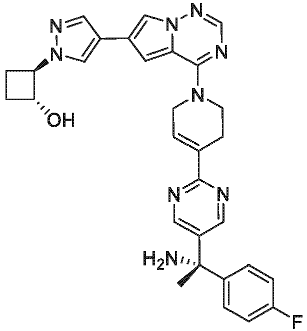 | 1, Ex 23 |
| 132 | 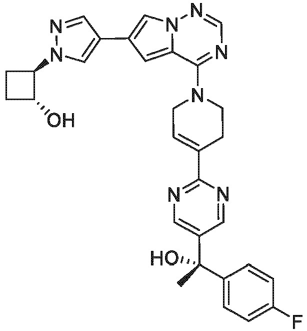 | 1, Ex 23 |
| 133 | 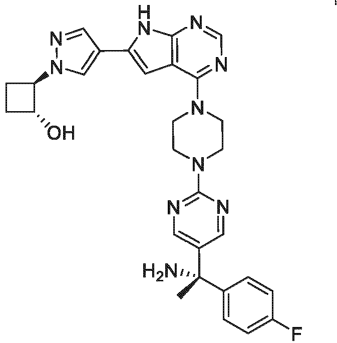 | 2, Ex 23 |
| 134 | 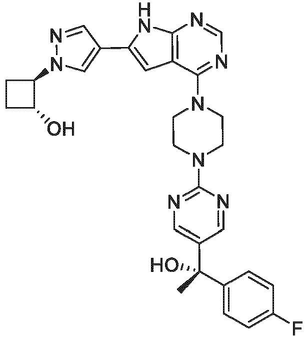 | 2, Ex 23 |

Compounds of the disclosure are selective KIT inhibitors. In some embodiments, compounds of the disclosure are selective D816V KIT inhibitors. Compounds of the disclosure are selective PDGFRα inhibitors. In some embodiments, compounds of the disclosure are selective PDGFRα exon 18 inhibitors. In some embodiments, compounds of the disclosure are selective PDGFRα D842V inhibitors. As used herein, a "selective KIT inhibitor" or a "selective PDGFRα inhibitor" refers to a compound or a pharmaceutically acceptable salt thereof or a solvate of any of the foregoing that selectively inhibits a KIT protein kinase or PDGFRα protein kinase over another protein kinase and exhibits at least a 2-fold selectivity for a KIT protein kinase or a PDGFRα protein kinase over another kinase. For example, a selective KIT inhibitor or a selective PDGFRA inhibitor exhibits at least a 9-fold selectivity, 10-fold selectivity; at least a 15-fold selectivity; at least a 20-fold selectivity; at least a 30-fold selectivity; at least a 40-fold selectivity; at least a 50-fold selectivity; at least a 60-fold selectivity; at least a 70-fold selectivity; at least a 80-fold selectivity; at least a 90-fold selectivity; at least 100-fold, at least 125-fold, at least 150-fold, at least 175-fold, or at least 200-fold selectivity for a KIT protein kinase or a PDGFRα kinase over another kinase. In some embodiments, a selective KIT inhibitor or a selective PDGFRα inhibitor exhibits at least 150-fold selectivity over another kinase, *e.g*., VEGFR2 (vascular endothelial growth factor receptor 2), SRC (Non-receptor protein tyrosine kinase), and FLT3 (Fms-Like Tyrosine kinase 3). In some embodiments, a selective KIT or a selective PDGFRα inhibitor exhibits selectivity over PDGRFβ, CSF1R (colony stimulating factor receptor 1), and FLT3. In some embodiments, a selective KIT or a selective PDGFRα inhibitor exhibits selective over LCK(lymphocyte-specific protein kinase), ABL (nuclear protein tyrosine kinase), never-in-mitosis gene A (NIMA)-related kinase 5 (NEK5), and ROCK1 (rho-associated coil-coil-continuing protein kinase-1). In some embodiments, selectivity for a KIT protein kinase or a PDGFRα protein kinase over another kinase is measured in a cellular assay (*e.g*., a cellular assay). In some embodiments, selectivity for a KIT protein kinase or a PDGFRα protein kinase over another kinase is measured in a biochemical assay (*e.g.*, a biochemical assay).

Compounds of the disclosure are selective over ion channels. In some embodiments, a selective KIT or a selective PDGFRα inhibitor has limited potential to inhibit human voltage-gated sodium channel (hNav 1.2).

Compounds of the disclosure are selective for mutant KIT over wild type KIT. In some embodiments, compounds of the disclosure are selective for exon 17 mutant KIT over wild type KIT.

Compounds of the disclosure can be useful for treating diseases or conditions associated with mutant KIT or mutant PDGFRA activity in humans or non-humans. In some embodiments, compounds of the disclosure are for use as a medicament. In some embodiments, compounds of the disclosure are for use in therapy. In some embodiments, compounds of the disclosure are for use in the manufacture of a medicament. In some embodiments, the disclosure provides methods for treating KIT-driven malignancies, include mastocytosis (SM), GIST (gastrointestinal stromal tumors), AML (acute myeloid leukemia), melanoma, seminoma, intracranial germ cell tumors, and/or mediastinal B-cell lymphoma. In addition, mutations in KIT have been linked to Ewing's sarcoma, DLBCL (diffuse large B cell lymphoma), dysgerminoma, MDS (myelodysplastic syndrome), NKTCL (nasal NK/T-cell lymphoma), CMML (chronic myelomonocytic leukemia), and brain cancers. In some embodiments, the disclosure provides methods for treating Ewing's sarcoma, DLBCL, dysgerminoma, MDS, NKTCL, CMML, and/or brain cancers. KIT mutations have also been found in thyroid cancer, colorectal cancer, endometrial cancer, bladder cancer, NSCLC, and breast cancer (AACR Project GENIE). In some embodiments, compounds of the disclosure can be useful for treating mast cell activation syndrome (MCAS). Compounds of the disclosure can be useful for treating systemic mastocytosis. Compounds of the disclosure can be useful for treating advanced systemic mastocytosis. Compounds of the disclosure can be useful for treating indolent SM and smoldering SM. Compounds of the disclosure can be useful for treating GIST.

Compounds of the disclosure can be useful for treating diseases or conditions associated with the KIT mutations in Exon 9, Exon 11, Exon 14, Exon 17, and/or Exon 18 of the KIT gene sequence. Compounds of the disclosure can be useful for treating diseases or conditions associated with PDGFRA mutations in Exon 12, Exon 14, and/or Exon 18 of the PDGFRA gene sequence. In some embodiments, provided herein are methods for treating a disease or condition associated with at least one KIT mutation in Exon 9, Exon 11, Exon 14, Exon 17, and/or Exon 18 of the KIT gene sequence. In some embodiments, methods for treating a disease or condition associated with at least one PDGFRA mutation in Exon 12, Exon 14, and/or Exon 18 of the PDGFRA gene sequence are provided.

Compounds of the disclosure can be active against one or more KIT protein kinases with mutations in Exon 17 of the KIT gene sequence (*e.g.*, KIT protein mutations D816V, D816Y, D816F, D816K, D816H, D816A, D816G, D816E, D816I, D816F, D820A, D820E, D820G, D820Y, N822K, N822H, V560G, Y823D, and A829P), and much less active against wild-type KIT protein kinase. In some embodiments, provided herein are methods for treating a disease or condition associated with at least one KIT mutation such as those chosen from D816V, D816Y, D816F, D816K, D816H, D816A, D816G, D816E, D816I, D816F, D820A, D820E, D820G, D820Y, N822K, N822H, V560G, Y823D, and A829P. In some embodiments, provided herein are methods for treating a disease or condition associated with at least one KIT mutation such as, *e.g*., those chosen from C809, C809G, D816H, D820A, D820G, N822H, N822K, and Y823D.

Compounds of the disclosure can be active against one or more KIT protein kinases with mutations in Exon 11 of the KIT gene sequence (*e.g*., KIT protein mutations del557-559insF, V559G/D). In some embodiments, provided herein are methods for treating a disease or condition associated with at least one KIT mutation, such as, *e.g*., those chosen from L576P, V559D, V560D, V560G, W557G, Del 554-558EVQWK, del557-559insF, Del EVQWK554-558, Del EVQWKVVEEINGNNYVYI554-571, Del KPMYEVQWK550-558, Del KPMYEVQW550-557FL, Del KV558-559, Del KV558-559N, Del MYEVQW552-557, Del PMYE551-554, Del VV559-560, Del WKVVE557-561, Del WK557-558, Del WKVV557-560C, Del WKVV557-560F, DelYEVQWK553-558, and insertion K558NP.

Compounds of the disclosure can be active against one or more KIT protein kinases with mutations in Exon 11/13 of the KIT gene sequence (*e.g.*, KIT protein mutations V559D/V654A, V560G/D816V, and V560G/822K). In some embodiments, provided here are methods for treating a disease or condition associated with one or more KIT mutations in Exon 11/13).

Compounds of the disclosure can be active against one or more KIT protein kinases with mutations in Exon 9 of the KIT gene sequence. In some embodiments, provided herein are methods for treating a disease or condition associated with at least one KIT mutation in Exon 9.

In some embodiments, compounds of the disclosure are not active against KIT protein kinases with the mutations V654A, N655T, T670I, and/or N680.

Compounds of the disclosure can be active against one or more PDGFRα protein kinases with mutations. In some embodiments, provided herein are methods for treating a disease or condition associated with at least one PDGFRA mutation in Exon 12 of the PDGFRA gene sequence, such as, *e.g*., PDGFRα protein mutations V561D, Del RV560-561, Del RVIES560-564, Ins ER561-562, SPDGHE566-571R, SPDGHE566-571K, or Ins YDSRW582-586. In some embodiments, provided herein are methods for treating a disease or condition associated with at least one PDGFRA mutation in Exon 14 of the PDGFRA gene sequence, such as, *e.g*., PDGFRα protein mutation N659K. In some embodiments, provided herein are methods for treating a disease or condition associated with at least one PDGFRA mutation in Exon 18 of the PDGFRA gene sequence, such as, *e.g.*, PDGFRα protein mutations D842V, D842Y, D842I, DI842-843IM, D846Y, Y849C, Del D842, Del I843, Del RD841-842, Del DIM842-845, Del DIMH842-845, Del IMHD843-846, Del MHDS844-847, RD841-842KI, DIMH842-845A, DIMH842-845V, DIMHD842-846E, DIMHD842-846S, DIMHD842-846N, DIMHD842-846G, IMHDS843-847T, IMHDS8843-847M, or HDSN845-848P.

Compounds of the disclosure can be active against one or more PDGFRα protein kinases with mutations Exon 18 in the PDGFRA gene sequence (*e.g*., protein mutations PDGFRα D842V, PDGFRα D842I, or PDGFRα D842Y). In some embodiments, provided herein are methods for treating a disease or condition associated with at least one PDGFRA mutation in Exon 18, such as, *e.g.*, protein mutation PDGFRα D842V.

Compounds of the disclosure can be useful for treating an eosinophilic disorder. In some embodiments, the eosinophilic disorder is mediated by mutant KIT or PDGFRα. In some embodiments, that eosinophilic disorder is mediated by wild type KIT or PDGFRα. In some embodiments, provided herein are methods for treating an eosinophilic disorder, comprising administering to a subject a therapeutically effective amount of the compounds of the disclosure or a pharmaceutically acceptable salt thereof and/or solvate of any of the foregoing. In one embodiment, the eosinophilic disorder is selected from hypereosinophilic syndrome, eosinophilia, eosinophilic enterogastritis, eosinophilic leukemia, eosinophilic granuloma and Kimura's disease.

In some embodiments, eosinophilic disorder is selected from hypereosinophilic syndrome, eosinophilia, eosinophilic enterogastritis, eosinophilic leukemia, eosinophilic granuloma and Kimura's disease. Other eosinophilic disorders include eosinophilic esophagitis, eosinophilic gastroenteritis, eosinophilic fasciitis, and Churg-Strauss syndrome.

In one embodiment, the eosinophilic disorder is hypereosinophilic syndrome. In a specific embodiment, the hypereosinophilic syndrome is idiopathic hypereosinophilic syndrome. In one embodiment, the eosinophilic disorder is eosinophilic leukemia. In a specific embodiment, the eosinophilic leukemia is chronic eosinophilic leukemia. In another embodiment, the eosinophilic disorder is refractory to treatment with imatinib, sunitinib, and/or regorafenib. In a specific embodiment, the eosinophilic disorder is refractory to treatment with imatinib.

Compounds of the disclosure can be useful for reducing the number of eosinophils in a subject in need thereof. In some embodiments, provided herein are methods for reducing the number of eosinophils in a subject in need thereof comprising administering to the subject a therapeutically effective amount of a compound of the disclosure or a pharmaceutically acceptable salt thereof and/or a solvate of any of the foregoing.

In one embodiment, the disclosed methods reduce the number of eosinophils in the blood, bone marrow, gastrointestinal tract (*e.g.*, esophagus, stomach, small intestine and colon), or lung. In another embodiment, a method disclosed herein reduces the number of blood eosinophils. In a further embodiment, a method disclosed herein reduces the number of lung eosinophils. In still a further embodiment, a method disclosed herein reduces the number of eosinophil precursor cells.

In another embodiment, the disclosed methods reduce (post-administration) the number of eosinophils by at least about 10%, at least about 20%, at least about 30%, at least about 40%, at least about 50%, at least about 60%, at least about 70%, at least about 80%; at least about 90%, at least about 95% or at least about 99%. In a specific embodiment, a method disclosed herein reduces the number of eosinophils below the limit of detection.

In another embodiment, the disclosed methods reduce (post-administration) the number of eosinophil precursors by at least about 10%, at least about 20%, at least about 30%, at least about 40%, at least about 50%, at least about 60%, at least about 70%, at least about 80%, at least about 90%, at least about 95% or at least about 99%. In a specific embodiment, a method disclosed herein reduces the number of eosinophil precursors below the limit of detection.

Compounds of the disclosure can be useful for treating mast cell disorders. Compounds of the disclosure can be useful for treating mastocytosis. Mastocytosis is subdivided into two groups of disorders: (1) cutaneous mastocytosis (CM) describes forms that are limited to the skin; and (2) systemic mastocytosis (SM) describes forms in which mast cells infiltrate extracutaneous organs, with or without skin involvement. SM is further subdivided into five forms: indolent (ISM); smoldering (SSM); aggressive (ASM); SM with associated hematologic non-mast cell lineage disease (SM-AHNMD); and mast cell leukemia (MCL).

Diagnosis of SM is based in part on histological and cytological studies of bone marrow showing infiltration by mast cells of often atypical morphology, which frequently abnormally express non-mast cell markers (CD25 and/or CD2). Diagnosis of SM is confirmed when bone marrow mast cell infiltration occurs in the context of one of the following: (1) abnormal mast cell morphology (spindle-shaped cells); (2) elevated level of serum tryptase above 20 ng/mL; or (3) the presence of the activating KIT protein mutations, such as, *e.g.*, exon 17 mutations such as D816 mutations such as D816V.

Activating mutations at the D816 position are found in the vast majority of mastocytosis cases (90-98%), with the most common mutations being D816V, D816H, and D816Y. The D816V mutation is found in the activation loop of the protein kinase domain and leads to constitutive activation of KIT kinase.

No drugs are approved for the non-advanced forms of systemic mastocytosis, ISM or SSM. Current approaches to management of these chronic diseases include nonspecific symptom-directed therapies that have varying degrees of efficacy and no effect on MC burden. Cytoreductive therapies, such as cladribine and interferon alpha, are occasionally used for intractable symptoms. Based on the current treatment landscape, there remains an unmet medical need in patients with ISM and SSM with moderate-to-severe symptoms that cannot be adequately managed by available symptom-directed therapies.

Compounds of the disclosure can be useful for treating ISM or SSM. In some embodiments, the patient with ISM or SSM has symptoms that are inadequately controlled by at least one, at least two, at least three symptomatic treatments. Symptoms can be assessed using a patient reported outcome (PRO) tool *e.g.* the Indolent Systemic Mastocytosis-Symptom Assessment Form (ISM-SAF) (ISPOR Europe 2019, Copenhagen Denmark, 2-6 Nov 2019). Compounds of the disclosure can be useful for improving symptoms associated with ISM or SSM *e.g.*, reducing or eliminating pruritus, flushing, headaches, and/or GI events, such as vomiting, diarrhea, and abdominal pain. Improvements in symptoms can be assessed using the ISM-SAF.

Compounds of the disclosure can be useful for treating other mast cell disorders, such as mast cell activation syndrome (MCAS), and hereditary alpha tryptasemia (HAT) (Picard Clin. Ther. 2013, May 35(5) 548; Akin J.Allergy Clin. Immuno. 140(2)349 62. Compounds of the disclosure can be useful for treating mast cell disorders associated with KIT and PDGFRα mutations. Compounds of the disclosure can be useful for treating mast cell diseases associated with wild type KIT and PDGFRα.

Compounds of the disclosure can be useful for treating mast cell activation syndrome (MCAS), which is an immunological condition in which mast cells inappropriately and excessively release chemical mediators, resulting in a range of chronic symptoms, sometimes including anaphylaxis or near-anaphylaxis attacks. Unlike mastocytosis, where patients have an abnormally increased number of mast cells, patients with MCAS have a normal number of mast cells that do not function properly and are defined as "hyperresponsive." Types of MCAS include primary MCAS (monoclonal mast cell activation syndrome (MMAS)), secondary MCAS (MCAS that arises from another disease), and idiopathic MCAS (MCAS that rules out primary or secondary MCAS).

Compounds of the disclosure can be useful for treating hereditary alpha tryptasemia (HAT)(overexpression of TPSAB1 causing elevated tryptase)).

Other mast cell diseases include mast cell mediated asthma, anaphylaxis (including idiopathic, Ig-E and non-Ig-E mediated), urticaria (including idiopathic and chronic), atopic dermatitis, swelling (angioedema), irritable bowel syndrome, mastocytic gastroenteritis, mastocytic colitis, pruritus, chronic pruritis, pruritis secondary to chronic kidney failure and heart, vascular, intestinal, brain, kidney, liver, pancreas, muscle, bone and skin conditions associated with mast cells. In some embodiments, the mast cell disease is not associated with mutant KIT or mutant PDGFRα.

KIT and PDGFRA mutations have been extensively studied in GIST. Compounds of the disclosure can be useful for treating GIST associated with KIT mutations. Compounds of the disclosure can be useful for treating unresectable or metastatic GIST. Nearly 80% of metastatic GISTs have a primary activating mutation in either the extracellular region (exon 9) or the juxtamembrane (JM) domain (exon 11) of the KIT gene sequence. Many mutant KIT tumors respond to treatment with targeted therapy such as imatinib, a selective tyrosine kinase inhibitor that specifically inhibits BCR-ABL, KIT, and PDGFRA proteins. However, most GIST patients eventually relapse due to a secondary mutation in KIT that markedly decreases the binding affinity of imatinib. These resistance mutations invariably arise within the adenosine 5-triphosphate (ATP)-binding pocket (exons 13 and 14) or the activation loop (exons 17 and 18) of the kinase gene. Of the currently approved agents for GIST, none are selective targeted agents. Imatinib is currently approved for the treatment of GIST; multikinase inhibitors are used after imatinib. In many cases, these multikinase inhibitors, such as, *e.g*., sunitinib, regorafenib, and midostaurin, only weakly inhibit imatinib resistant mutants and/or the multikinase inhibitors are limited by a more complex safety profile and a small therapeutic window. In some embodiments, compounds of the disclosure can be useful for treating GIST in patients who have been treated with imatinib. Compounds of the disclosure can be useful for treating GIST as first line (1L), second line (2L), third line (3L) or fourth line (4L) therapy.

Compounds of the disclosure can be useful for treating GIST when particular mutations in KIT are absent or present. In some embodiments, compounds of the disclosure are capable of treating GIST when particular mutations in KIT are absent. In certain embodiments, compounds of the disclosure are not capable of treating GIST when particular mutations in KIT are present. In some embodiments, compounds of the disclosure do not provide clinical benefit in patients harboring KIT ATP binding pocket mutations (KIT protein mutations V654A, N655T, and/or T670I).

Compounds of the disclosure can be useful for treating GIST associated with PDGFRA mutations. In 5 to 6% of unresectable of metastatic GIST patients, an activation loop mutation in exon 18 of the gene sequence of PDGFRA at the protein amino acid 842 occurs as the primary mutation.

Compounds of the disclosure can also be useful in treating AML. AML patients also harbor KIT mutations, with the majority of these mutations at the D816 position of the KIT protein.

In some embodiments, the compounds of the disclosure are administered to a subject in need thereof. In some embodiments, the compounds of the disclosure are administered as a pharmaceutical formulation, wherein the compound is combined with one or more pharmaceutically acceptable excipients or carriers. Thus, in some embodiments, disclosed herein are compositions comprising at least one entity chosen from compounds of Formula I and pharmaceutically acceptable salts thereof and/or solvates of any of the foregoing and optionally further comprising at least one pharmaceutically acceptable excipient.

Compounds of the disclosure may be formulated for administration in any convenient way for use in human or veterinary medicine. In some embodiments, the compound included in the pharmaceutical compositions may be active itself, or may be a prodrug, *e.g.*, capable of being converted to an active compound in a physiological setting.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Examples of pharmaceutically acceptable carriers include: (1) sugars, such as, *e.g.,* lactose, glucose, and sucrose; (2) starches, such as, *e.g.,* com starch and potato starch; (3) cellulose and its derivatives, such as, *e.g.*, sodium carboxymethyl cellulose, ethyl cellulose, and cellulose acetate; (4) powdered tragacanth; (5) malt; (6) gelatin; (7) talc; (8) excipients, such as, *e.g.,* cocoa butter and suppository waxes; (9) oils, such as, *e.g.,* peanut oil, cottonseed oil, safflower oil, sesame oil, olive oil, corn oil, and soybean oil; (10) glycols, such as, *e.g.,* propylene glycol; (11) polyols, such as, *e.g.,* glycerin, sorbitol, mannitol, and polyethylene glycol; (12) esters, such as, *e.g.,* ethyl oleate and ethyl laurate; (13) agar; (14) buffering agents, such as, *e.g*., magnesium hydroxide and aluminum hydroxide; (15) alginic acid; (16) pyrogen-free water; (17) isotonic saline; (18) Ringer's solution; (19) ethyl alcohol; (20) phosphate buffer solutions; (21) cyclodextrins, such as, *e.g.,* Captisol^{®}; and (22) other non-toxic compatible substances employed in pharmaceutical formulations.

Examples of pharmaceutically acceptable antioxidants include: (1) water soluble antioxidants, such as, *e.g.*, ascorbic acid, cysteine hydrochloride, sodium bisulfate, sodium metabisulfite, sodium sulfite, and the like; (2) oil-soluble antioxidants, such as, *e.g.*, ascorbyl palmitate, butylated hydroxyanisole (BHA), butylated hydroxytoluene (BHT), lecithin, propyl gallate, alpha-tocopherol, and the like; and (3) metal chelating agents, such as, *e.g.,* citric acid, ethylenediamine tetraacetic acid (EDTA), sorbitol, tartaric acid, phosphoric acid, and the like.

Solid dosage forms (*e.g.*, capsules, tablets, pills, dragees, powders, granules, and the like) can include one or more pharmaceutically acceptable carriers, such as, *e.g.,* sodium citrate or dicalcium phosphate, and/or any of the following: (1) fillers or extenders, such as, *e.g*., starches, lactose, sucrose, glucose, mannitol, and/or silicic acid; (2) binders, such as, *e.g*., carboxymethylcellulose, alginates, gelatin, polyvinyl pyrrolidone, sucrose, and/or acacia; (3) humectants, such as, *e.g.,* glycerol; (4) disintegrating agents, such as, *e.g.,* agar-agar, calcium carbonate, potato or tapioca starch, alginic acid, certain silicates, and sodium carbonate; (5) solution retarding agents, such as, *e.g*., paraffin; (6) absorption accelerators, such as, *e.g.,* quaternary ammonium compounds; (7) wetting agents, such as, *e.g.,* cetyl alcohol and glycerol monostearate; (8) absorbents, such as, *e.g.*, kaolin and bentonite clay; (9) lubricants, such as, *e.g.,* talc, calcium stearate, magnesium stearate, solid polyethylene glycols, sodium lauryl sulfate, and mixtures thereof; and (10) coloring agents.

Liquid dosage forms can include pharmaceutically acceptable emulsions, microemulsions, solutions, suspensions, syrups, and elixirs. In addition to the active ingredient, the liquid dosage forms may contain inert diluents commonly used in the art, such as, *e.g.,* water or other solvents, solubilizing agents, and emulsifiers, such as, *e.g.,* ethyl alcohol, isopropyl alcohol, ethyl carbonate, ethyl acetate, benzyl alcohol, benzyl benzoate, propylene glycol, 1,3-butylene glycol, oils (such as, *e.g*., cottonseed, groundnut, corn, germ, olive, castor, and sesame oils), glycerol, tetrahydrofurfuryl alcohol, polyethylene glycols and fatty acid esters of sorbitan, and mixtures thereof.

Suspensions, in addition to the active compounds, may contain suspending agents as, *e.g.*, ethoxylated isostearyl alcohols, polyoxyethylene sorbitol and sorbitan esters, microcrystalline cellulose, aluminum metahydroxide, bentonite, agar-agar, tragacanth, and mixtures thereof.

Ointments, pastes, creams and gels may contain, in addition to an active compound, excipients, such as, *e.g.*, animal and vegetable fats, oils, waxes, paraffins, starch, tragacanth, cellulose derivatives, polyethylene glycols, silicones, bentonites, silicic acid, talc, zinc oxide, or mixtures thereof.

Powders and sprays can contain, in addition to an active compound, excipients such as, *e.g.*, lactose, talc, silicic acid, aluminum hydroxide, calcium silicates, and polyamide powder, or mixtures of these substances. Sprays can additionally contain customary propellants, such as, *e.g*., chlorofluorohydrocarbons and volatile unsubstituted hydrocarbons, such as, *e.g*., butane and propane.

Non-limiting examples of dosage forms for the topical or transdermal administration of compounds of the disclosure include powders, sprays, ointments, pastes, creams, lotions, gels, solutions, patches, and inhalants. The active compound may be mixed under sterile conditions with a pharmaceutically acceptable carrier, and with any preservatives, buffers, or propellants that may be required.

When a compound of the disclosure is administered as a pharmaceutical to humans and animals, the compound can be given per se or as a pharmaceutical composition containing, for example, 0.1 to 99.5% (such as 0.5 to 90%) of active ingredient in combination with a pharmaceutically acceptable carrier.

The formulations can be administered topically, orally, transdermally, rectally, vaginally, parentally, intranasally, intrapulmonary, intraocularly, intravenously, intramuscularly, intraarterially, intrathecally, intracapsularly, intradermally, intraperitoneally, subcutaneously, subcuticularly, or by inhalation.

In addition, compounds of the disclosure can be administered alone or in combination with other compounds, including other KIT- or PDGFRα modulating compounds, or other therapeutic agents. In some embodiments, a compound of the disclosure can be administered in combination with ripretinib. In some embodiments, a compound of the disclosure can be administered in combination with one or more compounds selected from imatinib, sunitinib, regorafenib, cabozantinib, crenolanib, midostaurin, brentuximab vedotin, and mastitinib for treating a disease or condition disclosed herein.

Compounds of the disclosure can be administered to a patient, who has had prior treatment with another compound or compounds. Compounds of the disclosure can be useful as first line (1L), second line (2L), third line (3L), or fourth line (4L) therapy.

In some embodiments, a compound of the disclosure is administered after prior treatment with imatinib.

Compounds of the disclosure can be administered to a patient who has had no prior treatment with midostaurin. In some embodiments, compounds of the disclosure can be administered to a patient who has had prior treatment with midostaurin.

### EXAMPLES

### Definitions

- C: Celsius
- Cs₂CO₃: cesium carbonate
- DCM: dichloromethane
- DIPEA: diisopropylamine
- DMF: dimethyl formamide
- DMSO: dimethylsulfoxide
- EtOAc: ethyl acetate
- EDCI: 1-ethyl-3-(3-dimethylaminopropyl)carbodiimide
- h: hours
- H₂: hydrogen gas
- H₂O: water
- HCl: hydrochloric acid
- HOAc: acetic acid
- HOBT: hydroxybenzotriazole
- HPLC: high performance liquid chromatography
- IC50: inhibitory concentration 50%
- IPA: isopropyl alcohol
- K₂CO₃: potassium carbonate
- KOAc: potassium acetate
- LCMS: liquid chromatography mass spectrometry
- LiAlH₄: lithium aluminum hydride
- min: minutes
- MsCl: mesyl chloride
- MTBE: methyl tert-butyl ether
- MeOH: methanol
- N₂: nitrogen gas
- NaOH: sodium hydroxide
- Na₂SO₄: sodium sulfate
- NH₄HCO₃: ammonium formate
- NMP: N-methylpyrrolidinone
- Pd/C: palladium on carbon
- Pd(dppf)Cl₂: [1,1'-bis(diphenylphosphino)ferrocene]dichloropalladium(II)
- PE: petroleum ether
- RT: room temperature
- TEA: triethylamine
- THF: tetrahydrofuran
- TsCl: tosyl chloride

Methods for preparing compounds of the disclosure can be carried out in suitable solvents which can be readily selected by one of skill in the art of organic synthesis. Suitable solvents can be substantially non-reactive with the starting materials (reactants), intermediates, or products at the temperatures at which the reactions are carried out, *e.g*., temperatures which can range from the solvent's freezing temperature to the solvent's boiling temperature. A given reaction can be carried out in one solvent or a mixture of more than one solvent. Depending on the particular reaction step, suitable solvents for a particular reaction step can be selected by the skilled artisan.

Preparation of compounds of the disclosure can involve the protection and deprotection of various chemical groups. The need for protection and deprotection, and the selection of appropriate protecting groups, can be readily determined by one skilled in the art. The chemistry of protecting groups can be found, for example, in Wuts and Greene, Protective Groups in Organic Synthesis, 5th ed., John Wiley & Sons: New Jersey, (2014).

Reactions can be monitored according to any suitable method known in the art. For example, product formation can be monitored by spectroscopic means, such as nuclear magnetic resonance (NMR) spectroscopy (*e.g*., ¹H or ¹³C), infrared (IR) spectroscopy, spectrophotometry (*e.g*., UV-visible), mass spectrometry (MS), or by chromatographic methods such as high performance liquid chromatography (HPLC) or thin layer chromatography (TLC). Analytical instruments and methods for compound characterization:

LC-MS: Unless otherwise indicated, all liquid chromatography-mass spectrometry (LC-MS) data (sample analyzed for purity and identity) were obtained with an Agilent model-1260 LC system using an Agilent model 6120 mass spectrometer utilizing ES-API ionization fitted with an Agilent Poroshel 120 (EC-C18, 2.7 um particle size, 3.0 x 50mm dimensions) reverse-phase column at 22.4 degrees Celsius. The mobile phase consisted of a mixture of solvent 0.1% formic acid in H2O and 0.1% formic acid in acetonitrile. A constant gradient from 95% aqueous/5% organic to 5% aqueous/95% organic mobile phase over the course of 4 minutes was utilized. The flow rate was constant at 1 mL/min.

Silica gel chromatography: Silica gel chromatography was performed on either a Teledyne Isco CombiFlash^{®} Rf unit or a Biotage^{®} Isolera Four unit.

Proton NMR: Unless otherwise indicated, all 1H NMR spectra were obtained with a Varian 400MHz Unity Inova 400 MHz NMR instrument (acquisition time = 3.5 seconds with a 1 second delay; 16 to 64 scans). Where characterized, all protons were reported in DMSO-d6 solvent as parts-per million (ppm) with respect to residual DMSO (2.50 ppm).

One of ordinary skill in the art will recognize that modifications of the gradient, column length, and flow rate are possible and that some conditions may be more suitable for compound characterization than others, depending on the chemical species being analyzed.

### EXAMPLES

### Synthetic Preparations

### Preparation of Intermediates

### Preparation 1: N-((S)-1-(2-(1-(6-bromopyrrolo[2,1-f][1,2,4]triazin-4-yl)-1,2,3,6-tetrahydropyridin-4-yl)pyrimidin-5-yl)-1-(4-fluorophenyl)ethyl)-2-methylpropane-2-sulfinamide (I-1)

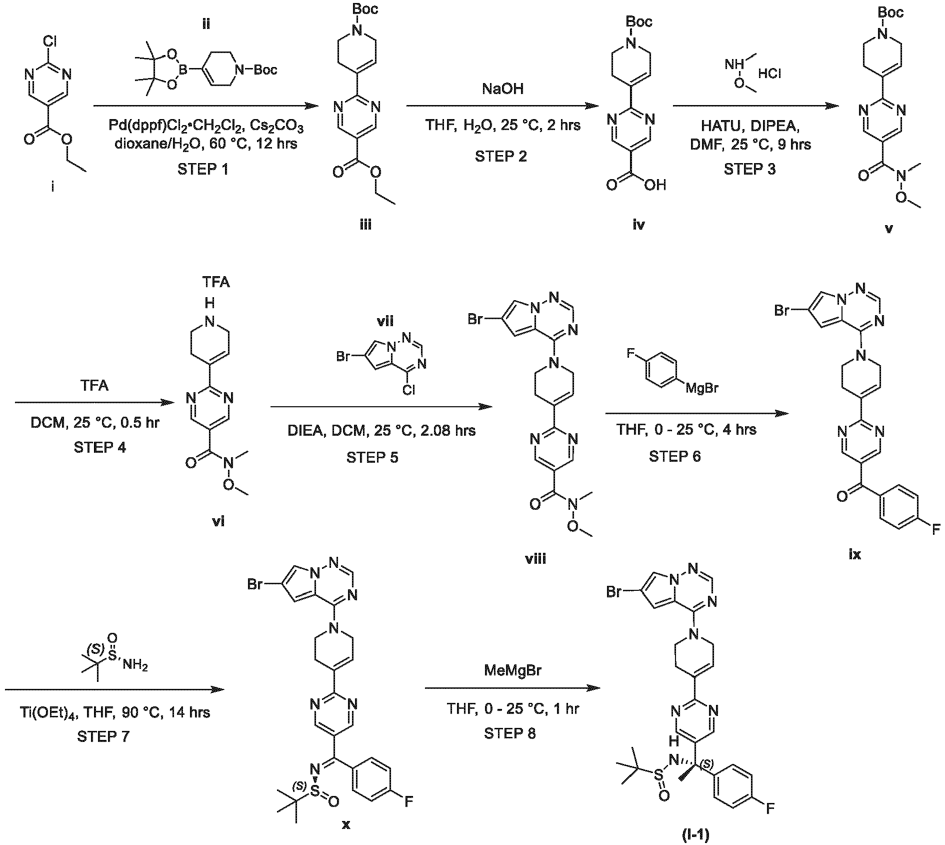

Step 1: Synthesis of ethyl 2-(1-(tert-butoxycarbonyl)-1,2,3,6-tetrahydropyridin-4-yl)pyrimidine-5-carboxylate (iii): A mixture of ethyl 2-chloropyrimidine-5-carboxylate (5.00 g, 26.8 mmol, 1.00 eq), tert-butyl 4-(4,4,5,5-tetramethyl-1,3,2-dioxaborolan-2-yl)-3,6-dihydropyridine-1(2H)-carboxylate (9.11 g, 29.5 mmol, 1.10 eq), Pd(dppf)Cl₂•CH₂Cl₂ (2.63 g, 3.22 mmol, 0.12 eq), Cs₂CO₃ (17.5 g, 53.6 mmol, 2.00 eq) in dioxane (150 mL) and H₂O (15.0 mL) was degassed and purged with N₂ (g) three times, and then the mixture was stirred at 60 °C for 12 h under N₂ (g) atmosphere. The reaction mixture was filtered, then diluted with H₂O (300 mL) and extracted with EtOAc (400 mL x 3). The combined organic layers were washed with brine (500 mL x 2), dried over Na₂SO₄, filtered and concentrated under reduced pressure to give a residue. The residue was purified by column chromatography (silica gel, petroleum ether: EtOAc = 50 : 1 to 10 : 1, plate 1, Rf = 0.71) to give the title compound (4.90 g, 54.9% yield) as a light yellow solid. LCMS: RT = 0.977 min, m/z = 278.2 (M-56+H)+.

Step 2: Synthesis of 2-(1-(tert-butoxycarbonyl)-1,2,3,6-tetrahydropyridin-4-yl)pyrimidine-5-carboxylic acid (iv): To a solution of ethyl 2-(1-(tert-butoxycarbonyl)-1,2,3,6-tetrahydropyridin-4-yl)pyrimidine-5-carboxylate (3.00 g, 9.00 mmol, 1.00 eq) in THF (30.0 mL) was added the mixture of NaOH (803 mg, 20.1 mmol, 2.23 eq) in H₂O (20.1 mL). Then the mixture was stirred at 25 °C for 2 hrs. The mixture was concentrated under vacuum to remove THF, then adjusted pH to 2-3 with 1M HCl and filtered. The filter cake was concentrated under vacuum to give the title compound (2.49 g, 90.6% yield) as a light-yellow solid that was used in the next step without further purification. 1H NMR (400 MHz, d6-DMSO) δ 9.11 (s, 2H), 7.28 (br s, 1H), 4.12 (br s, 3H), 3.52 - 3.55 (br t, J = 5.2 Hz, 2H), 2.63 (s, 2H), 1.43 (s, 9H).

Step 3: Synthesis of tert-butyl 4-(5-(methoxy(methyl)carbamoyl)pyrimidin-2-yl)-3,6-dihydropyridine-1(2H)-carboxylate (v): To a solution of 2-(1-(tert-butoxycarbonyl)-1,2,3,6-tetrahydropyridin-4-yl)pyrimidine-5-carboxylic acid (2.49 g, 8.16 mmol, 1.00 eq) in DMF (50.0 mL) was added HATU (6.20 g, 16.3 mmol, 2.00 eq), DIPEA (5.27 g, 40.8 mmol, 7.10 mL, 5.00 eq), N,O-dimethylhydroxylamine hydrochloride (1.19 g, 12.2 mmol, 1.50 eq). Then the mixture was stirred at 25 °C for 9 h. The mixture was diluted with EtOAc (50.0 mL), washed with water (100 mL x 2), the water layers was extracted with EtOAc (50.0 mL x 2), then combined the organic layers and washed in sequence with 1M HCl (40.0 mL), saturated NaHCO₃ (40.0 mL), brine (50.0 mL). The organic layer was dried with Na₂SO₄, filtered and concentrated under vacuum. The residue was purified by column chromatography (SiO₂, petroleum ether : EtOAc = 5 : 1 to 10 : 3, petroleum ether : EtOAc = 1 : 1, Rf = 0.60) to give the title compound (1.89 g, 66.5% yield) as a light yellow solid: LCMS: RT = 0.888 min, m/z = 293.3 (M-56+H)+. 1H NMR: (400 MHz, d6-DMSO) δ 9.00 (s, 2H), 7.30 (br s, 1H), 4.13 (br s, 2H), 3.60 (s, 3H), 3.54 (br t, J = 5.6 Hz, 2H). 3.31 (s, 3H), 2.69 (s, 1H), 2.63 (br d, J = 1.6 Hz, 2H), 1.43 (s, 9H).

Step 4: Synthesis of N-methoxy-N-methyl-2-(1,2,3,6-tetrahydropyridin-4-yl)pyrimidine-5-carboxamide trifluoroacetate (vi): To a solution of tert-butyl 4-(5-(methoxy(methyl)carbamoyl)pyrimidin-2-yl)-3,6-dihydropyridine-1(2H)-carboxylate (1.89 g, 5.42 mmol, 1.00 eq) in DCM (18.9 mL) was added TFA (7.48 g, 65.6 mmol, 4.86 mL, 12.1 eq). The mixture was stirred at 25 °C for 0.5 h, then was concentrated under vacuum to give the title compound (1.97 g, crude) as a yellow oil that was used in the next step without further purification.

Step 5: Synthesis of 2-(1-(6-bromopyrrolo[2,1-f][1,2,4]triazin-4-yl)-1,2,3,6-tetrahydropyridin-4-yl)-N-methoxy-N-methylpyrimidine-5-carboxamide (viii): To a solution of N-methoxy-N-methyl-2-(1,2,3,6-tetrahydropyridin-4-yl)pyrimidine-5-carboxamide trifluoroacetate (1.97 g, 5.44 mmol, 1.00 eq) in DCM (20.0 mL) was added DIPEA (4.22 g, 32.6 mmol, 5.68 mL, 6.00 eq), and the resulting mixture was stirred at 25 °C for 5 mins. 6-Bromo-4-chloropyrrolo[2,1-f][1,2,4]triazine (1.33 g, 5.71 mmol, 1.05 eq) was added, and the mixture was stirred at 25 °C for another 2 h. The reaction mixture was concentrated under vacuum, diluted with isopropanol (20.0 mL) and water (5.00 mL), stirred for 16 h, and filtered. The filter cake was washed with isopropanol (10.0 mL x 3), washed with petroleum ether (20.0 mL x 2), and concentrated under vacuum to the title compound (1.99 g, 82.4% yield) was obtained as a yellow solid that was used in the next step without further purification: LCMS: RT = 0.897 min, m/z = 446.2 (M+H)+. 1H NMR: (400 MHz, d6-DMSO) δ 9.03 (s, 2H), 7.96 (d, J = 1.6 Hz, 1H), 7.93 (s, 1H), 7.40 (br s, 1H), 7.24 (d, J = 1.6 Hz, 1H), 4.75 (br s, 2H), 4.14 (t, J = 5.6 Hz, 2H), 3.61 (s, 3H), 3.32 (s, 3H), 2.83 (br s, 2H).

Step 6: Synthesis of (2-(1-(6-bromopyrrolo[2,1-f][1,2,4]triazin-4-yl)-1,2,3,6-tetrahydropyridin-4-yl)pyrimidin-5-yl)(4-fluorophenyl)methanone (ix): To a cooled solution (0 °C) of 2-(1-(6-bromopyrrolo[2,1-f][1,2,4]triazin-4-yl)-1,2,3,6-tetrahydropyridin-4-yl)-N-methoxy-N-methylpyrimidine-5-carboxamide (1.78 g, 4.01 mmol, 1.00 eq) in THF (110 mL) was added (4-fluorophenyl)magnesium bromide (1.00 M solution in THF, 28.1 mL, 7.00 eq) drop-wise under N₂ (g). The resulting mixture was stirred at 25 °C for 4 h. The mixture was poured into saturated aq. NH₄Cl solution (50.0 mL) slowly, then diluted with EtOAc (50.0 mL). The water layer was extracted with EtOAc (30.0 mL x 3), combined the organic layers and washed with brine (50.0 mL), dried with Na₂SO₄ and concentrated. The crude product was triturated with isopropanol (30.0 mL) at 25 °C for 3 h, and stirred for 12 h. The mixture was filtered with isopropanol (3.00 mL x 3), and the filter cake was washed with petroleum ether (3.00 mL) and dried under vacuum to give the title compound (1.36 g, 69.6% yield, 98.3% purity) as a light yellow solid: LCMS: RT = 1.024 mins, m/z = 479.2 (M+H)+. 1H NMR (400 MHz, d6-DMSO) δ 9.09 (s, 2H), 7.92 - 7.98 (m, 4H), 7.49 (br s, 1H), 7.43 (t, J = 8.8 Hz, 2H), 7.25 (d, J= 1.6 Hz, 1H), 4.78 (br s, 2H), 4.16 (t, J = 5.6 Hz, 2H), 2.87 (br s, 2H).

Step 7: Synthesis of (S,E)-N-((2-(1-(6-bromopyrrolo[2,1-f][1,2,4]triazin-4-yl)-1,2,3,6-tetrahydropyridin-4-yl)pyrimidin-5-yl)(4-fluorophenyl)methylene)-2-methylpropane-2-sulfinamide (x): To a solution of (2-(1-(6-bromopyrrolo[2,1-f][1,2,4]triazin-4-yl)-1,2,3,6-tetrahydropyridin-4-yl)pyrimidin-5-yl)(4-fluorophenyl)methanone (1.06 g, 2.17 mmol, 1.00 eq) in THF (60.0 mL) was added (S)-2-methylpropane-2-sulfinamide (1.05 g, 8.70 mmol, 4.00 eq), Ti(OEt)₄ (22.0 g, 96.5 mmol, 20.0 mL, 44.4 eq). Then the mixture was stirred at 90 °C for 14 hrs. The reaction mixture was cooled to room temperature, quenched by water (100 mL), and filtered. The filter cake was washed with EtOAc (30.0 mL x 3), then the filtrate was washed with brine (50.0 mL), dried over Na₂SO₄, filtered, and concentrated under reduced pressure. The residue was purified by column chromatography (silica gel, petroleum ether : EtOAc = 3 : 1 to 0 : 1) to give the title compound (1.07 g, 82.1% yield) as a light yellow solid: LCMS: RT = 2.749 mins, m/z = 584.3 (M+H)+. 1H NMR: (400 MHz, d6-DMSO) δ 8.23 (s, 2H), 7.97 (d, J = 1.6 Hz, 1H), 7.95 (s, 1H), 7.58 - 7.82 (m, 2H), 7.43 (br s, 1H), 7.37 (br t, J = 8.8 Hz, 2H), 7.26 (d, J = 1.2 Hz, 1H), 4.78 (br s, 2H), 4.17 (br t, J = 5.6 Hz, 2H), 2.86 (br s, 2H), 1.26 (s, 9H).

Step 8: Synthesis of N-((S)-1-(2-(1-(6-bromopyrrolo[2,1-f][1,2,4]triazin-4-yl)-1,2,3,6-tetrahydropyridin-4-yl)pyrimidin-5-yl)-1-(4-fluorophenyl)ethyl)-2-methylpropane-2-sulfinamide (I-1): To a cooled (0 °C) solution of (S,E)-N-((2-(1-(6-bromopyrrolo[2,1-f][1,2,4]triazin-4-yl)-1,2,3,6-tetrahydropyridin-4-yl)pyrimidin-5-yl)(4-fluorophenyl)methylene)-2-methylpropane-2-sulfinamide (600 mg, 1.00 mmol, 1.00 eq) in THF (15.0 mL) was added MeMgBr (3.00 M solution in ether, 3.34 mL, 10.0 eq) under N₂ (g). Then the mixture was stirred at 25 °C for 1 h. The mixture was carefully quenched with saturated aq. NH₄Cl (30.0 mL) and extracted with EtOAc (20.0 mL x 3). The organic extracts were washed with brine (20.0 mL), dried with Na2SO4, and concentrated under vacuum. The residue was purified by prep-TLC (SiO2, petroleum ether: EtOAc = 1 : 1, Rf = 0.35, 0.30) to give the title compound **I-1** (632 mg, 89.3% yield, 84.7% purity) as a yellow solid: LCMS: RT = 1.024 mins, m/z = 598.2 (M+H)+.

In some preparations, **I-1** is mixed with MeOH (7 vol.) and 4 M HCl in dioxane (6.0 equiv) and heated to 40 °C. After reaction completion, the mixture is cooled to rt and charged with MTBE (10 vol.). The mixture is filtered and washed with MTBE and dried under vacuum to afford the amine **(I-1a):**

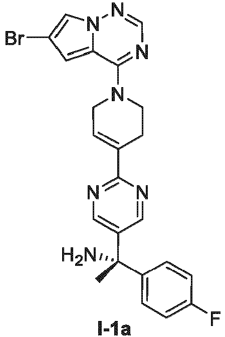

The amine I-1a (2.0 mmol), 4,4,4',4',5,5,5',5'-octamethyl-2,2'-bi(1,3,2-dioxaborolane) (3.1 mmol), Pd(dppf)Cl₂ (200 µmol), dppf (300 µmol) and KOAc (4.0 mmol) in 1,4-dioxane (30 mL) is purged with N₂ (g) for 10 min and stirred at 80 °C for 16 h. After that, the solution is diluted with EA, washed with H2O and brine, and concentrated. The residue is purified by flash column chromatography on silica gel (DCM/MeOH = 15/1) to afford the compound **(I-1A):**

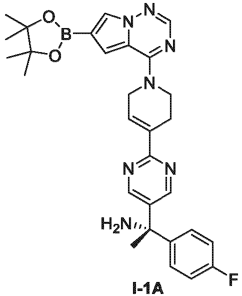

In some preparations, **I-1A** is mixed with 4-(4,4,5,5-tetramethyl-1,3,2-dioxaborolan-2-yl)-1H-pyrazole (6.0 mmol), Pd(dppf)Cl₂ (605 µmol) and K₂CO₃ (18.2 mmol) in DMF/H₂O (40 mL/10 mL) and purged with N₂ (g) for 10 mins and stirred at 70 °C for 16 h under N₂. After that, the solution is diluted with EA, washed with H₂O and brine, and concentrated. The residue is purified by flash column chromatography on silica gel (DCM/MeOH = 10/1) to afford the compound **(I-1B):** 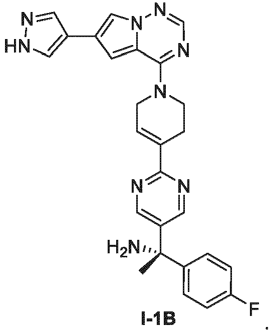 The corresponding (S)-hydroxy intermediates: **(I-8), (I-8A),** and **(I-8B):** 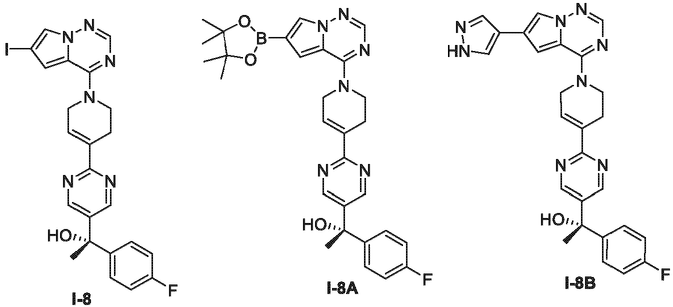 are prepared by treating intermediate ix (resulting from Step 6 above) with methyl magnesium Grinard in THF at 0 °C to rt. After completion of the reaction, the mixture is quenched in NH₄Cl soluation and extracted with EA. The combined organic layers are washed with H₂O and brine, dried over sodium sulfate, filtered, and concentrated. The residue is purified by column chromatography to afford the racemate. The enantiomers are separated by chiral HPLC to afford (R)- and (S)-**I-8.** The (S)-hydroxy compounds I-8A and I-8B are prepared by substituting **I-8** in for **I-1a** to the methods of Preparation 1.

### Preparation 2: (S)-1-(4-fluorophenyl)-1-(2-(4-(6-iodo-7H-pyrrolo[2,3-d]pyrimidin-4-yl)piperazin-1-yl)pyrimidin-5-yl)ethan-1-amine hydrochloride (I-2).

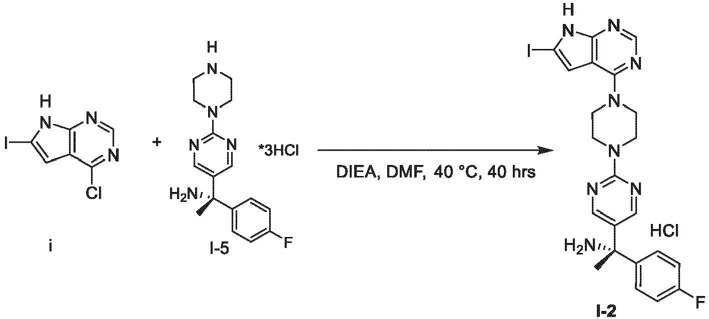

To a suspension of compound I-5 (see Preparation 6)(1.32 g, 3.22 mmol, 1.00 eq, 3 HCl) in DMF (23.2 mL) was added DIEA (1.66 g, 12.9 mmol, 2.24 mL, 4.00 eq) and 4-chloro-6-iodo-7H-pyrrolo[2,3-d]pyrimidine (900 mg, 3.22 mmol, 1.00 eq). The mixture was stirred at 40 °C for 60 h. The mixture was poured into H₂O (150 mL) drop-wise, and the resulting suspension was filtered. The filter cake was washed with H₂O (20.0 mL x 3). The crude product was purified by prep-HPLC (column: Phenomenex Synergi Max-RP 250 x 50 mm x 10 mm; mobile phase: [water (0.05% HCl) - CH₃CN]; B%: 10% - 35%, 20 min) to give the title compound (1.20 g, 64.0% yield, 99.8% purity) as a yellow solid: LCMS: RT = 0.882 min, m/z = 528.3 (M-16)+.

In some preparations, a mixture of **I-2** (2.0 mmol), 4,4,4',4',5,5,5',5'-octamethyl-2,2'-bi(1,3,2-dioxaborolane) (3.1 mmol), Pd(dppf)Cl₂ (200 µmol), dppf (300 µmol) and KOAc (4.0 mmol) in 1,4-dioxane (30 mL) is purged with N₂ (g) for 10 min and stirred at 80 °C for 16 h. After that, the solution is diluted with EA, washed with H2O and brine, and concentrated. The residue is purified by flash column chromatography on silica gel (DCM/MeOH = 15/1) to afford the compound **(I-2A):**

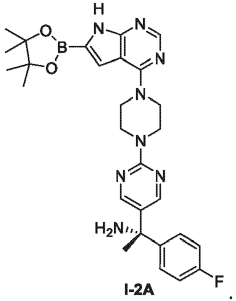

In some preparations, **I-2** is mixed with 4-(4,4,5,5-tetramethyl-1,3,2-dioxaborolan-2-yl)-1H-pyrazole (6.0 mmol), Pd(dppf)Cl₂ (605 µmol) and K₂CO₃ (18.2 mmol) in DMF/H₂O (40 mL/10 mL) and purged with N₂ (g) for 10 mins and stirred at 70 °C for 16 h under N₂. After that, the solution is diluted with EA, washed with H₂O and brine, and concentrated. The residue is purified by flash column chromatography on silica gel (DCM/MeOH = 10/1) to afford the compound **(I-2B):** 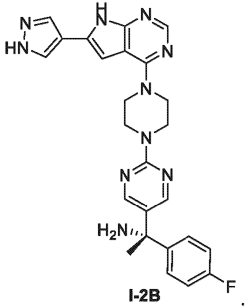 The corresponding (S)-hydroxy intermediates: (I-7), (I-7A), and (I-7B): 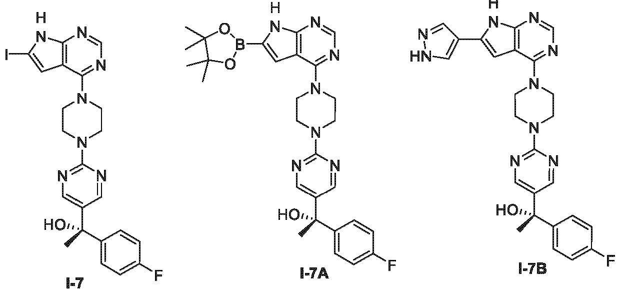 are prepared by substituting intermediate **I-6** (See Preparation 6) for **I-5** in the methods of Preparation 2.

### Preparation 3: 1-(2-(1-(6-bromopyrrolo[2,1-f][1,2,4]triazin-4-yl)-1,2,3,6-tetrahydropyridin-4-yl)pyrimidin-5-yl)-1-(4-fluorophenyl)ethan-1-ol (1k):

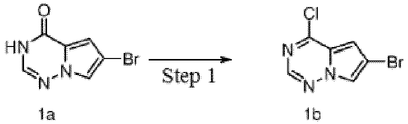

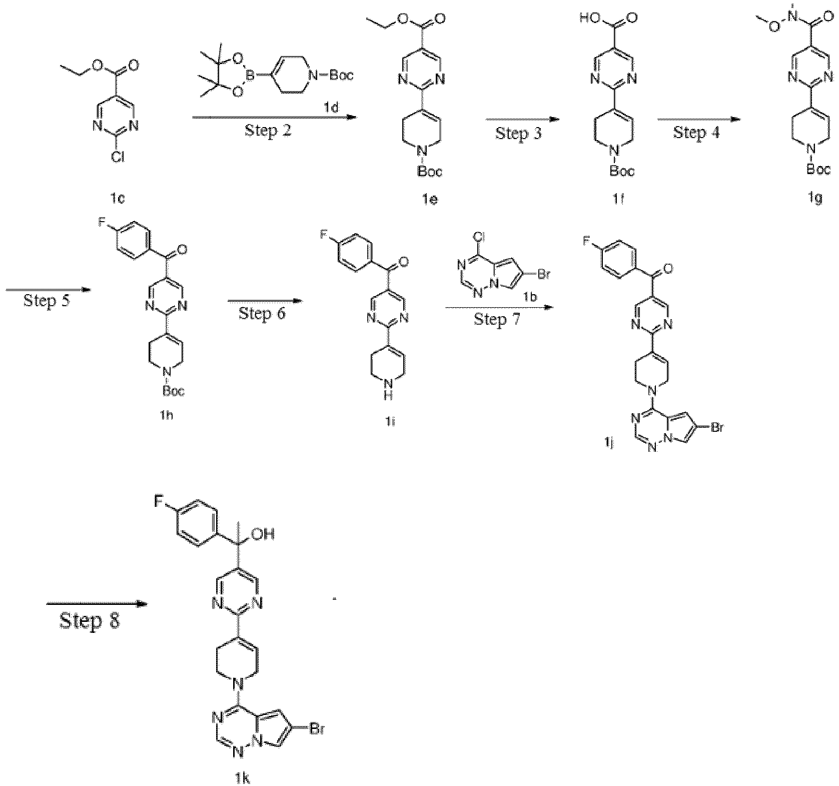

Step 1: 6-bromopyrrolo[2,1-f][1,2,4]triazin-4(3H)-one 1a (22 mmol) is dissolved in phosphorus oxychloride (100 mL), and reacted for 3 h at 130°C. The reaction solution is concentrated under reduced pressure. The resulting residue is added with a saturated aqueous solution of glacial sodium hydrogen carbonate (100 mL) and extracted with DCM. The organic phases are combined and washed with a saturated aqueous NaCl solution (100 mL). The organic phase is dried over anhydrous Na₂SO₄, and concentrated under reduced pressure, to obtain 6-bromo-4-chloropyrrolo[2,1-f][1,2,4]triazine 1b.

Step 2: Under argon atmosphere, ethyl 2-chloropyrimidin-5-carboxylate 1c (10 mmol), t-butyl 4-(4,4,5,5-tetramethyl-1,3,2-dioxaborolan-2-yl)-3,6-dihydropyridine-1(2H)-carboxylate 1d (11 mmol), [1,1'-bis(diphenylphosphino)ferrocene]palladium dichloride (1.2 mmol), and cesium carbonate (20 mmol) are dissolved in 1,4-dioxane/H₂O(66 mL, V/V=10/1), and reacted for 5 h at 60°C. The reaction solution is diluted by adding EA (150 mL) and washed sequentially with water (30 mL x 2), and a saturated aqueous NaCl solution (30 mL). The organic phase is dried over anhydrous sodium sulfate and concentrated under reduced pressure. The resulting residue is purified by column chromatography on silica gel to obtain ethyl 2-(1-(t-butoxycarbonyl)-1,2,3,6-tetrahydropyridin-4-yl)pyrimidin-5-carboxylate 1e.

Step 3: Ethyl 2-(1-(t-butoxycarbonyl)-1,2,3,6-tetrahydropyridin-4-yl)pyrimidin-5-carboxylate 1e (5.4 mmol) is dissolved in THF (15 mL), and an aqueous NaOH solution (10 mL, 1 M) is added dropwise, and reacted for 5 h at rt. The reaction solution is concentrated under reduced pressure to remove THF, H₂O (20 mL) is added, and the solution is adjusted to about pH 2-3 with a 1 M aqueous HCl solution. A large amount of white solid product is precipitated out, filtered and dried to obtain crude 2-(1-(t-butoxycarbonyl)-1,2,3,6-tetrahydropyridin-4-yl)pyrimidin-5-carboxylic acid 1f.

Step 4: 2-(1-(t-butoxycarbonyl)-1,2,3,6-tetrahydropyridin-4-yl)pyrimidin-5-carboxylic acid 1f (4.86 mmol) is dissolved in DCM (50 mL). N,N-diisopropylethyl amine (24 mmol), 2-(7-azabenzotriazole)tetramethyluronium hexafluorophosphate (9.7 mmol) and N,O-dimethylhydroxylamine hydrochloride (7.3 mmol) are added in sequence, and reacted at rt for 6 h. The reaction solution is diluted by adding DCM (150 mL), and washed sequentially with H₂O (20 mL x 2), a 1 M aqueous HCl solution (20 mL), a saturated aqueous sodium hydrogen carbonate solution (20 mL), and a saturated aqueous NaCl solution (20 mL). The organic phase is dried over anhydrous Na₂SO₄ and concentrated under reduced pressure. The resulting residue is purified by column chromatography on silica gel to obtain t-butyl 4-(5-(methoxy(methyl)carbamoyl)pyrimidin-2-yl)-3,6-dihydropyridine-1(2H)-carboxylate 1g.

Step 5: Under argon atmosphere, t-butyl 4-(5-(methoxy(methyl)carbamoyl)pyrimidin-2-yl)-3,6-dihydropyridine-1(2H)-carboxylate 1g (3.4 mmol) is dissolved in THF (15 mL). The reaction solution is cooled to 0°C in an ice water bath, and 4-fluorophenyl magnesium bromide (14 mL,1M/THF) is added dropwise, and reacted for 4 h at rt. The reaction is quenched by adding a saturated aqueous ammonium chloride solution. The reaction solution is diluted by adding EA (100 mL) and washed sequentially with H₂O (20 mL x 2), and a saturated aqueous NaCl solution (20 mL). The organic phase is dried over anhydrous sodium sulfate and concentrated under reduced pressure. The resulting residue is purified by column chromatography on silica gel to obtain t-butyl 4-(5-(4-fluorobenzoyl)pyrimidin-2-yl)-3,6-dihydropyridine-1(2H)-carboxylate 1h.

Step 6: t-butyl 4-(5-(4-fluorobenzoyl)pyrimidin-2-yl)-3,6-dihydropyridine-1(2H)-carboxylate 1h (2.4 mmol) is dissolved in DCM (10 mL), and trifluoroacetic acid (2 mL) is added and reacted at rt for 2 h. The reaction solution is concentrated under reduced pressure, to obtain crude (4-fluorophenyl)(2-(1,2,3,6-tetrahydropyridin-4-yl)pyrimidin-5-yl)methanone 1i, which is directly used in the next step.

Step 7: (4-fluorophenyl)(2-(1,2,3,6-tetrahydropyridin-4-yl)pyrimidin-5-yl)methanone 1i (2.4 mmol) is dissolved in DCM (15 mL). DIPEA (9.5 mmol) is added dropwise, and stirred for 5 min at rt. Then 6-bromo-4-chloropyrrolo[2,1-f][1,2,4]triazine 1b (2.8 mmol) is added, and reacted at rt for 12 h. The reaction solution is concentrated under reduced pressure to remove DCM. The residue is diluted by adding EA (70 mL), and washed sequentially with H₂O (10 mL), 1 M aqueous HCl solution (10 mL) and a saturated aqueous NaCl solution (20 mL). The organic phase is dried over anhydrous sodium sulfate and concentrated under reduced pressure. The resulting residue is purified by column chromatography on silica gel to obtain (2-(1-(6-bromopyrrolo[2,1-f][1,2,4]triazin-4-yl)-1,2,3,6-tetrahydropyridin-4-yl)pyrimidin-5-yl)(4-fluorophenyl)methanone 1j.

Step 8: Under argon atmosphere, (2-(1-(6-bromopyrrolo[2,1-f][1,2,4]triazin-4-yl)-1,2,3,6-tetrahydropyridin-4-yl)pyrimidin-5-yl)(4-fluorophenyl)methanone 1j (0.4 mmol) is dissolved in THF (10 mL). The reaction solution is cooled to 0°C in an ice water bath, and methyl magnesium bromide (4.2 mL, 1 M/THF) is added dropwise, and reacted at rt for 3 h. At 0°C, the reaction is quenched by adding a saturated aqueous ammonium chloride solution (10 mL). The reaction solution is distilled under reduced pressure to remove THF. The reaction solution is diluted by adding EA (50 mL) to the residue. The aqueous layer is separated, and the organic phase is washed sequentially with water (10 mL x 2) and a saturated aqueous NaCl solution (20 mL), dried over anhydrous sodium sulfate, and concentrated under reduced pressure. The resulting residue is purified by column chromatography on silica gel to obtain 1-(2-(1-(6-bromopyrrolo[2,1-f][1,2,4]triazin-4-yl)-1,2,3,6-tetrahydropyridin-4-yl)pyrimidin-5-yl)-1-(4-fluorophenyl)ethan-1-ol **(1k).**

In some preparations, a mixture of **1k** (2.0 mmol), 4,4,4',4',5,5,5',5'-octamethyl-2,2'-bi(1,3,2-dioxaborolane) (3.1 mmol), Pd(dppf)Cl₂ (200 µmol), dppf (300 µmol) and KOAc (4.0 mmol) in 1,4-dioxane (30 mL) is purged with N₂ (g) for 10 min and stirred at 80 °C for 16 h. After that, the solution is diluted with EA, washed with H₂O and brine, and concentrated. The residue is purified by flash column chromatography on silica gel (DCM/MeOH) to afford the compound **(1k-A):**

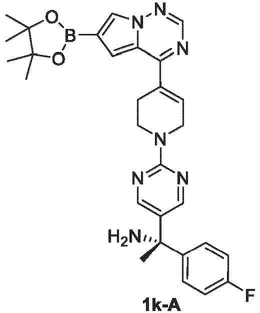

In some preparations, **1k-A** mixed with 4-(4,4,5,5-tetramethyl-1,3,2-dioxaborolan-2-yl)-1H-pyrazole (6.0 mmol), Pd(dppf)Cl₂ (605 µmol) and K₂CO₃ (18.2 mmol) in DMF/H₂O (40 mL/10 mL) and purged with N₂ (g) for 10 mins and stirred at 70 °C for 16 h under N₂. After that, the solution is diluted with EA, washed with H₂O and brine, and concentrated. The residue is purified by flash column chromatography on silica gel (DCM/MeOH) to afford the compound **(1k-B):** 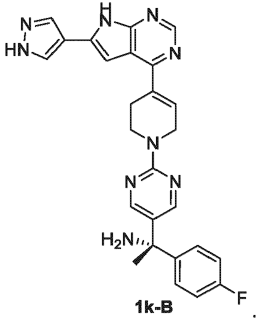 The corresponding (S)-hydroxy intermediates: **(I-9), (I-9A),** and **(I-9B):** 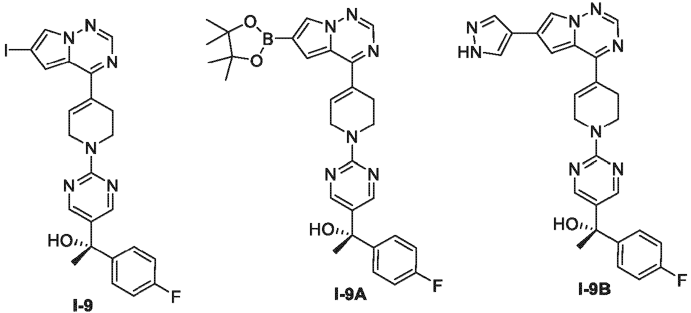 are prepared by treating intermediate 1j (resulting from Step 7 above) with (S)-2-Methylpropane-2-sulfinamide (0.908 mmol) and ethyl orthotitanate (0.715 mmol) and stirring in THF (3.2 mL) at 70 °C until the reaction is complete by tlc. Room temperature is attained, water is added, and the products are extracted into EA. The combined organic extracts are washed with brine, dried over Na₂SO₄, filtered, and concentrated in vacuo while loading onto Celite. Purification of the residue by MPLC (0-10% MeOH-EtOAc) affords the sulfonamide. The resulting sulfonamide is taken up in THF and cooled to 0 °C. Methylmagnesium bromide (3 M solution in diethyl ether, 1.5 mmol) is added and the resulting mixture stirred at 0 °C until completion. Additional methylmagnesium bromide is added, if needed. Saturated ammonium chloride is added and the products are extracted into EA. The combined organic extracts are washed with brine, dried over Na₂SO₄, filtered, and concentrated in vacuo while loading onto Celite. Purification of the residue by MPLC (0-10% MeOH-EtOAc) methyl-sulfinamide, which is deprotected by stirring in 4 M HCl in 1,4-dioxane (1.5 mL)/MeOH (1.5 mL) at rt. The solvent is removed in vacuo and the residue triturated in EAc to give the racemate. The enantiomers are separated by chiral HPLC to afford (R)- and (S)-**1-9.**

### Preparation 4: (R)-1-(4-(4,4,5,5-tetramethyl-1,3,2-dioxaborolan-2-yl)-1H-pyrazol-1-yl)propan-2-ol (I-3)

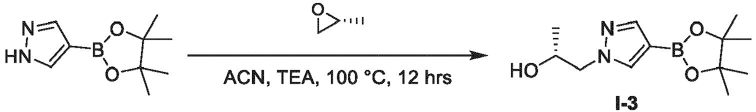

To a solution of 4-(4,4,5,5-tetramethyl-1,3,2-dioxaborolan-2-yl)-1H-pyrazole (2.00 g, 10.3 mmol, 1.00 eq) and (R)-2-methyloxirane (1.50 g, 25.8 mmol, 1.81 mL, 2.50 eq) in acetonitrile (6.70 mL) was added triethylamine (4.38 g, 43.3 mmol, 6.03 mL, 4.20 eq). The mixture was stirred at 100 °C for 12 hrs, then was cooled and concentrated in vacuo. The residue was purified by column chromatography (SiO₂, Petroleum ether:EA = 2:1) to give the title compound (2.05 g, 78.9% yield) as a white oil. LCMS: RT = 0.759 min, m/z = 253.2 (M+H)+. 1H NMR: (400 MHz, d6-DMSO) δ 7.85 (s 1H), 7.56 (s, 1H), 7.88 (d, J = 4.8 Hz 1H), 4.00 - 4.02 (m, 2H), 3.93 - 3.96 (m, 1H), 1.24 (s, 12H), 1.00 - 1.01 (m, 3H).

### Preparation 5: (S)-1-(4-(4,4,5,5-tetramethyl-1,3,2-dioxaborolan-2-yl)-1H-pyrazol-1-yl)propan-2-ol (I-4).

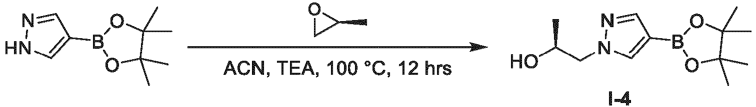

To a solution of 4-(4,4,5,5-tetramethyl-1,3,2-dioxaborolan-2-yl)-1H-pyrazole (2.00 g, 10.3 mmol, 1.00 eq) and (S)-2-methyloxirane (1.80 g, 30.9 mmol, 2.17 mL, 3.00 eq) in acetonitrile (6.70 mL) was added triethylamine (4.38 g, 43.3 mmol, 6.03 mL, 4.20 eq). The mixture was stirred at 100 °C for 12 hrs, then was cooled and concentrated under in vacuo. The residue was purified by column chromatography (SiO₂, Petroleum ether: EtOAc = 2:1) to give the title compound (2.13 g, 72.4% yield, 88.4% purity) as a white oil: LCMS: RT = 0.767 min, m/z = 253.2 (M+H)+. 1H NMR (400 MHz, d6-DMSO) δ 7.85 (s 1H), 7.56 (s, 1H), 7.88 (d, J = 4.8 Hz 1H), 4.00 - 4.02 (m, 2H), 3.93 - 3.96 (m, 1H), 1.24 (s, 12H), 1.01 (d, J = 6.0 Hz, 3H).

### Preparation 6: (S)-1-(4-fluorophenyl)-1-(2-(piperazin-1-yl)pyrimidin-5-yl)ethan-1-amine hydrochloride (I-5A)

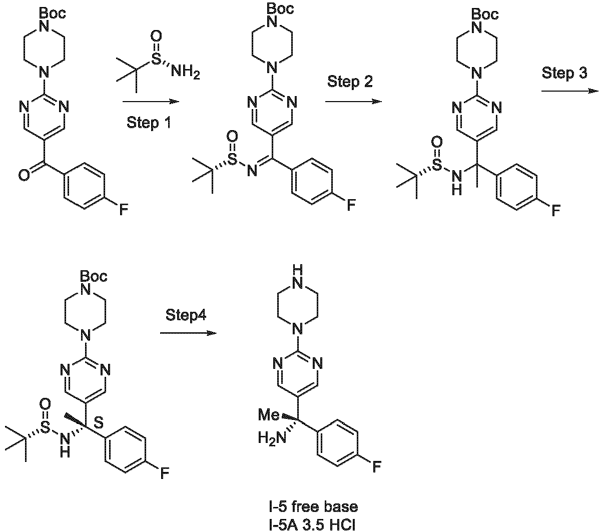

Step 1: Synthesis of *tert*-Butyl (S,Z)-4-(5-(((*tert*-butylsulfinyl)imino)(4-fluorophenyl)methyl)- pyrimidin-2-yl)piperazine-1-carboxylate tert-Butyl 4-(5-(4-fluorobenzoyl)pyrimidin-2-yl)piperazine-1-carboxylate (20.0 g, 1.0 eq.), (S)-(-)-2-methyl-2-propanesulfinamide (9.43 g, 1.5 eq), and LiOH (0.64 g, 0.5 eq.) were added to a reaction vessel with toluene (160 mL). To this mixture, titanium(IV)isopropoxide (18.42 g, 1.25 eq.) was added and the reaction agitated at 50-60 °C for 1 h. The reaction was then distilled to remove 80 mL while charging additional toluene (80 mL) at 40-60 °C. The reaction mixture was cooled to 20-30 °C and then added to a monosodium citrate solution (80 mL, 30%-w/w citric acid at pH 3-4). The mixture was agitated for 1.5 hrs at 45-55 °C and then the phases separated. The organic phase was washed with potassium bicarbonate (40 mL, 25%-w/w aqueous) and the organic phase distilled to remove 40 mL. The product solution was diluted with tetrahydrofuran (30 mL) before being used in the next step directly as a solution (approx. 15% w/w of *tert*-Butyl (S,Z)-4-(5-(((*tert*-butylsulfinyl)imino)(4-fluorophenyl)methyl)- pyrimidin-2-yl)piperazine-1-carboxylate).

Step 2: Synthesis of *tert*-Butyl 4-(5-((S)-1-(((S)-*tert*-butylsulfinyl)amino)-1-(4-fluorophenyl)ethyl)pyrimidin-2-yl)piperazine-1-carboxylate by isolation Methyl magnesium chloride (27.8 g, 22%-w/w in THF, 2.0 eq.) was added to the *tert-*butyl (S,Z)-4-(5-(((*tert*-butylsulfinyl)imino)(4-fluorophenyl)methyl)-pyrimidin-2-yl)piperazine-1-carboxylate reaction solution in toluene/THF (120 g corresponding to 20 g input material) at 10 °C over 2 to 3 hrs. The reaction mixture was allowed to agitate for 1.5 hrs to reach reaction completion. The reaction mixture was quenched by the addition of methanol (40 mL) followed by water (10 mL). The mixture was distilled to remove 100-110 mL distillate and then washed with ammonium chloride (80 mL, 20% w/w in water). The organic phase was washed with water (80 mL), diluted with toluene (60 mL), and distilled to remove 60-80 mL distillate. The solution of *tert*-Butyl 4-(5-((S)-1-(((S)-*tert*-butylsulfinyl)amino)-1-(4-fluorophenyl)ethyl)pyrimidin-2-yl)piperazine-1-carboxylate at 50-60 °C was charged with n-heptane (80 mL) and then cooled to 42 °C, at which time seeds were added (25-50 mg). The solution was held 30 min and then cooled to 0-10 °C for 30 min. The solids were isolated by filtration, washed with n-heptane and toluene mixture (1:1, 30 mL) followed by n-heptane (30 mL). The product was dried to yield 9 g (40-45%) of crude *tert*-Butyl 4-(5-((S)-1-(((S)-*tert*-butylsulfinyl)amino)-1-(4-fluorophenyl)ethyl)pyrimidin-2-yl)piperazine-1-carboxylate (96.4 to 97.2% de).

Step 3: Recrystallization of *tert*-Butyl 4-(5-((S)-1-(((S)-*tert*-butylsulfinyl)amino)-1-(4-fluorophenyl)ethyl)pyrimidin-2-yl)piperazine-1-carboxylate.

*tert*-Butyl 4-(5-((S)-1-(((S)-*tert*-butylsulfinyl)amino)-1-(4-fluorophenyl)ethyl)pyrimidin-2-yl)piperazine-1-carboxylate (10.0 g) was dissolved in isopropanol (100 mL) and heated to 40-60 °C, then passed through a clarifying filter with washing/rinsing with isopropanol (20 mL). The resulting solution was vacuum distilled at 40-60 °C to remove 60-70 mL distillate. The mixture was diluted with water (45 mL) at 50-60 °C and then cooled to 40 °C, at which time it was seeded with 25-50 mg. The mixture was further cooled to 20-25 °C and water (20 mL) was added. The solids were isolated by filtration, washed with isopropropanol/water mixture (1:1, 20 mL) and then slurry washed with isopropanol/water (1:2, 30 mL). Drying yielded 8.5 g (85%) of product of *tert*-Butyl-4-(5-((S)-1-(((S)-*tert*-butylsulfinyl)amino)-1-(4-fluorophenyl)ethyl)pyrimidin-2-yl)piperazine-1-carboxylate (>99.8% de).

### Step 4: Synthesis of (S)-1-(4-fluorophenyl)-1-(2-(piperazin-1-yl)pyrimidin-5-yl)ethan-1-amine hydrochloride (I-5A)

*tert-*Butyl 4-(5-((S)-1-(((S)-*tert*-butylsulfinyl)amino)-1-(4-fluorophenyl)ethyl)pyrimidin-2-yl)piperazine-1-carboxylate (7) (9.0 g) was heated to 45-55 °C in acetonitrile (40 mL) with hydrochloric acid (33%, 8.14 g, 4.1 eq.) for 1 h to afford (S)-1-(4-fluorophenyl)-1-(2-(piperazin-1-yl)pyrimidin-5-yl)ethan-1-amine hydrochloride (I-5A). Preparation of (S)-1-(4-fluorophenyl)-1-(2-(piperazin-1-yl)pyrimidin-5-yl)ethanol **(I-6):** 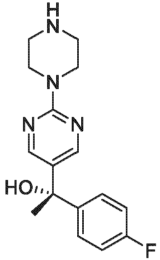 **(I-6)** and its R-enantiomer are described in WO2015/057873.

### Preparation of Compounds

### Example 1: Synthesis of (S)-2-(4-(4-(4-(5-(1-amino-1-(4-fluorophenyl)ethyl)pyrimidin-2-yl)-3,6-dihydropyridin-1(2H)-yl)pyrrolo[2,1-f][1,2,4]triazin-6-yl)-1H-pyrazol-1-yl)ethan-1-ol (1)

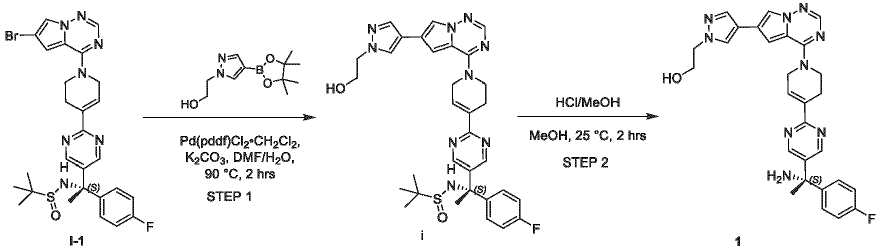

Step 1: Synthesis of N-((S)-1-(4-fluorophenyl)-1-(2-(1-(6-(1-(2-hydroxyethyl)-1H-pyrazol-4-yl)pyrrolo[2,1-f][1,2,4]triazin-4-yl)-1,2,3,6-tetrahydropyridin-4-yl)pyrimidin-5-yl)ethyl)-2-methylpropane-2-sulfinamide (i): To a solution of I-1 (300 mg, 424 umol, 1.00 eq), 2-(4-(4,4,5,5-tetramethyl-1,3,2-dioxaborolan-2-yl)-1H-pyrazol-1-yl)ethan-1-ol (142 mg, 594 mmol, 1.40 eq), K₂CO₃ (117 mg, 849 umol, 2.00 eq) in DMF (5.00 mL) and H₂O (1.00 mL) was added Pd(dppf)Cl₂•CH₂Cl₂ (52.0 mg, 63.7 umol, 0.15 eq) under N₂ (g). Then the mixture was stirred at 90 °C for 2 hrs. The mixture was diluted with EtOAc (50.0 mL), then washed in sequence with water (10.0 mL) and brine (35.0 mL x 3). The water layer was extracted with EtOAc (20.0 mL x 2), and the combined the organic layers were dried with Na₂SO₄, filtered, and concentrated. The residue was purified by prep-HPLC (column: 3_Phenomenex Luna C18 75 x 30 mm x 3 mm; mobile phase: [water (0.05%HCl) - CH3CN]; B%: 29% - 49%, 6.5 min). Fractions containing product were combined and adjusted pH to 9 with saturated aq. NaHCO₃, extracted with EtOAc (20.0 mL x 3), and concentrated under vacuum to give the title compound (66.0 mg, 105 µmol, 24.7% yield) as a light yellow solid: LCMS: RT = 0.877 min, m/z = 630.3 (M+H)+.

Step 2: Synthesis of (S)-2-(4-(4-(4-(5-(1-amino-1-(4-fluorophenyl)ethyl)pyrimidin-2-yl)-3,6-dihydropyridin-1(2H)-yl)pyrrolo[2,1-f][1,2,4]triazin-6-yl)-1H-pyrazol-1-yl)ethan-1-ol (1): To a solution of N-((S)-1-(4-fluorophenyl)-1-(2-(1-(6-(1-(2-hydroxyethyl)-1H-pyrazol-4-yl)pyrrolo[2,1-f][1,2,4]triazin-4-yl)-1,2,3,6-tetrahydropyridin-4-yl)pyrimidin-5-yl)ethyl)-2-methylpropane-2-sulfinamide (66.0 mg, 105 mmol, 1.00 eq) in MeOH (2.00 mL) was added HCl/MeOH (4.00 M, 0.50 mL, 19.1 eq). Then the mixture was stirred at 25 °C for 2 hrs. The mixture was adjusted pH to 5 and concentrated under vacuum. The residue was diluted with DMF (1.00 mL) and purified by prep-HPLC (column: 3_Phenomenex Luna C18 75 x 30 mm x 3 mm; mobile phase: [water (0.05% HCl) - CH3CN]; B%: 14% - 34%, 6.5 min). Fractions containing product were combined and adjusted pH to 8 with saturated aq. NaHCO₃, extracted with EtOAc (20.0 mL x 3), and the combined organic layers was washed with brine (20.0 mL), dried with Na₂SO₄, and concentrated to give the title compound (18.0 mg, 31.7% yield) as yellow gum: LCMS: RT = 2.178 mins, m/z = 526.5 (M+H)+. 1H NMR (400 MHz, d6-DMSO) δ 8.79 (s, 2H), 8.08 (s, 1H), 7.99 (d, J = 1.6 Hz, 1H), 7.87 (s, 1H), 7.85 (s, 1H), 7.44 - 7.51 (m, 2H), 7.23 - 7.30 (m, 2H), 7.08 - 7.16 (m, 2H), 4.94 (t, J = 5.6 Hz, 1H), 4.74 (br s, 2H), 4.12 - 4.19 (m, 4H), 3.72 - 3.79 (m, 2H), 2.82 (br s, 2H), 1.80 (s, 3H).

### Example 2: Synthesis of (S)-2-(4-(4-(4-(5-(1-amino-1-(4-fluorophenyl)ethyl)pyrimidin-2-yl)piperazin-1-yl)-7H-pyrrolo[2,3-d]pyrimidin-6-yl)-1H-pyrazol-1-yl)ethan-1-ol hydrochloride (2)

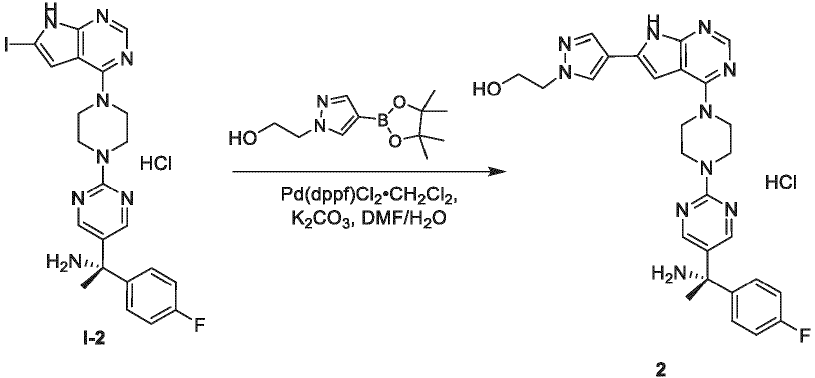

To a solution of I-2 (90.0 mg, 152 mmol, 98.3% purity, 1.00 eq), 2-(4-(4,4,5,5-tetramethyl-1,3,2-dioxaborolan-2-yl)-1H-pyrazol-1-yl)ethan-1-ol (43.5 mg, 183 mmol, 1.20 eq) in DMF (2.00 mL) was added Pd(dppf)Cl₂•CH₂Cl₂ (45.0 mg, 55.1 mmol) and K₂CO₃ (63.1 mg, 457 mmol, 3.00 eq), H₂O (0.50 mL). The mixture was degassed with N₂ (g) three times, then stirred at 95 °C for 5 hrs. The mixture was diluted with EtOAc (20.0 mL), washed with water (10.0 mL x 3), and washed with brine (10.0 mL). The aqueous layer was extracted with EtOAc (20 mL). The combined organic layers were dried over Na₂SO₄ and concentrated under vacuum. The residue was purified by prep-HPLC (column: 3_Phenomenex Luna C18 75 x 30 mm x 3 mm; mobile phase: [water (0.05% HCl) - CH3CN]; B%: 9% - 29%, 7 min). Fractions containing product were lyophilized to give the title compound (30.3 mg, 34.4% yield) as a light yellow solid: LCMS: RT = 0.711 min, m/z = 512.4 (M-16)+. 1H NMR (400 MHz, d6-DMSO) δ13.21 - 12.41 (m, 1H), 9.63 - 8.94 (m, 3H), 8.42 (s, 2H), 8.30 (s, 1H), 8.22 (s, 1H), 8.00 (s, 1H), 7.53 - 7.42 (m, 2H), 7.31 (t, J = 8.8 Hz, 2H), 7.12 (br d, J = 1.2 Hz, 1H), 4.19 (br t, J = 5.2 Hz, 2H), 4.13 - 4.06 (m, 4H), 4.03 - 3.97 (m, 4H), 3.76 (t, J = 5.2 Hz, 2H), 2.02 (s, 3H). Compounds 12, 13, 14, 14A, and 15 are prepared using the same procedure and substituting I-2 with the appropriate intermediate.

### Comparative Example 3: Synthesis of (S)-1-(4-fluorophenyl)-1-(2-(4-(6-(1-(oxetan-3-yl)-1H-pyrazol-4-yl)-7H-pyrrolo[2,3-d]pyrimidin-4-yl)piperazin-1-yl)pyrimidin-5-yl)ethan-1-amine (3)

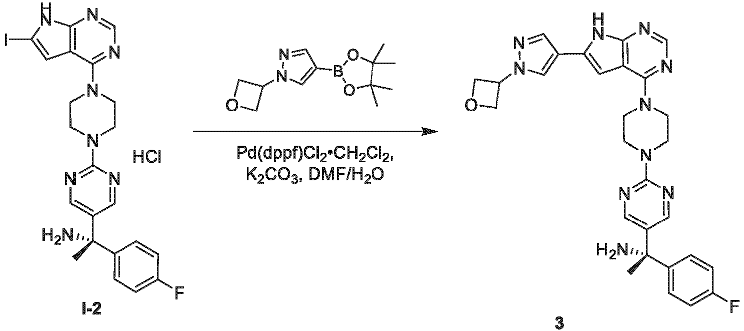

The title compound was prepared as described for example 2, except that 1-(oxetan-3-yl)-4-(4,4,5,5-tetramethyl-1,3,2-dioxaborolan-2-yl)-1H-pyrazole was used in place of 2-(4-(4,4,5,5-tetramethyl-1,3,2-dioxaborolan-2-yl)-1H-pyrazol-1-yl)ethan-1-ol. The crude product was purified by prep-HPLC (column: 3_Phenomenex Luna C18 75 x 30 mm x 3 mm; mobile phase: [water (0.05% HCl) - CH3CN]; B%: 11% - 31%, 6.5 min). Fractions containing product were combined, adjusted pH to 8~9 with solid sodium carbonate, and extracted with EtOAc (20.0 mL x 3). The organic layer was washed with brine (10.0 mL), dried over Na₂SO₄, filtered and concentrated under vacuum to give the title compound (97.5 mg, 50.8% yield) as an off-white solid: LCMS: EW25770-9-P1C, product: RT = 0.876 min, m/z = 524.4 (M-16)+. 1H NMR: EW25770-9-P1A 400 MHz, d6-DMSO) δ 12.0 (s, 1H), 8.40 (s, 2H), 8.34 (s, 1H), 8.15 (s, 1H), 8.09 (s, 1H), 7.41 - 7.51 (m, 2H), 7.05 - 7.15 (m, 2H), 6.92 (d, J = 1.2 Hz, 1H), 5.61 (q, J = 6.4 Hz, 1H), 4.93 - 5.00 (m, 2H), 4.84 - 4.92 (m, 2H), 3.95 - 3.98 (m, 4H), 3.87 - 3.88 (m, 4H), 1.73 (s, 3H). Compounds 28, 29, 30, 31, and 32 are prepared using the same procedure and substituting I-2 with the appropriate intermediate.

### Example 4: Synthesis of (R)-1-(4-(4-(4-(5-((S)-1-amino-1-(4-fluorophenyl)ethyl)pyrimidin-2-yl)piperazin-1-yl)-7H-pyrrolo[2,3-d]pyrimidin-6-yl)-1H-pyrazol-1-yl)propan-2-ol (4)

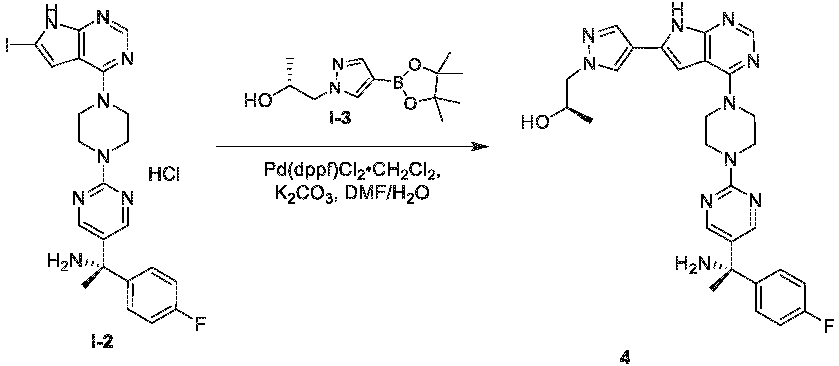

The title compound was prepared as described for comparative example 3, except that I-3 was used in place of 2-(4-(4,4,5,5-tetramethyl-1,3,2-dioxaborolan-2-yl)-1H-pyrazol-1-yl)ethan-1-ol. The crude product was purified by prep-HPLC (column: 3_Phenomenex Luna C18 75 x 30 mm x 3 mm; mobile phase: [water (0.05% HCl) - CH3CN]; B%: 11% - 31%, 6.5 min). The fractions containing product were adjusted pH to 8 and extracted with EtOAc (20.0 mL x 3). The combined organic extracts were washed with brine (20.0 mL), dried with Na2SO4 and concentrated to give the title compound (51.3 mg, 17.0% yield) as an off-white solid: LCMS: RT = 0.858 min, m/z = 526.4 (M-16)+. 1H NMR (400 MHz, d6-DMSO) δ 12.0 (s, 1H), 8.39 (s, 2H), 8.05 - 8.20 (m, 2H), 7.94 (s, 1H), 7.41 - 7.53 (m, 2H), 7.01 - 7.18 (m, 2H), 6.86 (d, J = 2.0 Hz, 1H), 4.97 (d, J = 4.8 Hz, 1H), 4.01 - 4.05 (m, 2H), 3.92 - 3.98 (m, 4H), 3.81 - 3.91 (m, 4H), 1.73 (s, 3H), 1.06 (d, J = 6.0 Hz, 3H). Compounds 7, 8, 9, 10, and 11 are prepared using the same procedure and substituting I-2 with the appropriate intermediate.

### Example 5: Synthesis of (S)-1-(4-(4-(4-(5-((S)-1-amino-1-(4-fluorophenyl)ethyl)pyrimidin-2-yl)piperazin-1-yl)-7H-pyrrolo[2,3-d]pyrimidin-6-yl)-1H-pyrazol-1-yl)propan-2-ol hydrochloride (5)

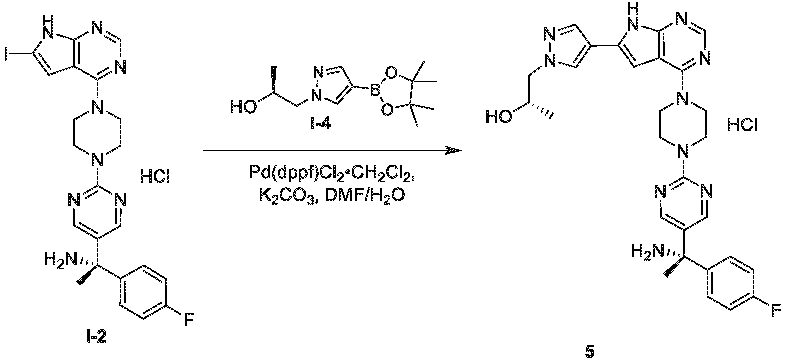

The title compound was prepared as described for example 2, except that I-4 was used in place of 2-(4-(4,4,5,5-tetramethyl-1,3,2-dioxaborolan-2-yl)-1H-pyrazol-1-yl)ethan-1-ol. The crude product was purified by prep-HPLC (column: 3_Phenomenex Luna C18 75 x 30 mm x 3 mm; mobile phase: [water (0.05% HCl) - CH3CN]; B%: 10% - 30%, 7 min). Fractions containing desired product were combined and lyophilized to give the title compound (128.5 mg, 57.8% yield) as a yellow solid: LCMS: RT = 0.850 min, m/z = 526.5 (M-16)+. 1H NMR: (400 MHz, d6-DMSO) δ 13.3 (br s, 1H), 9.55 (br s, 3H), 8.47 (s, 2H), 8.35 (s, 1H), 8.28 (s, 1H), 8.04 (s, 1H), 7.51 - 7.55 (m, 2H), 7.26 - 7.34 (m, 2H), 7.26 (s, 1H), 4.15 - 4.17 (m, 4H), 4.10 - 4.11 (m, 2H), 4.02 - 4.04 (m, 4H), 3.96 - 4.00 (m, 2H), 2.03 (s, 3H), 1.07 (d, J = 6.0 Hz, 3H). Compounds 38, 39, 40, 41, and 42 are prepared using the same procedure and substituting I-2 with the appropriate intermediate.

### Comparative Example 6: Synthesis of (S)-1-(4-fluorophenyl)-1-(2-(4-(6-(1-(oxetan-3-ylmethyl)-1H-pyrazol-4-yl)-7H-pyrrolo[2,3-d]pyrimidin-4-yl)piperazin-1-yl)pyrimidin-5-yl)ethan-1-amine hydrochloride

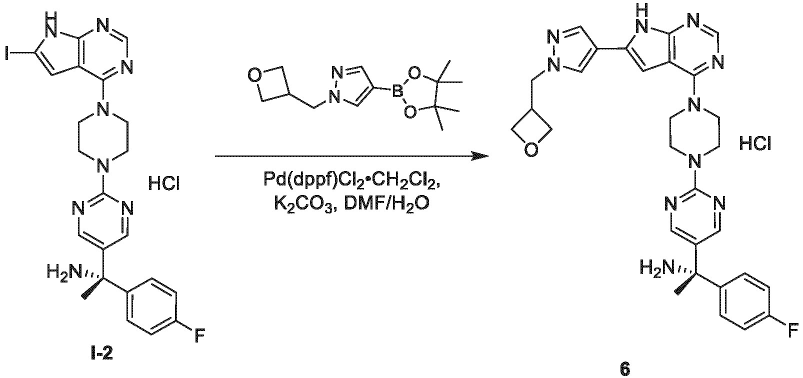

The title compound was prepared as described for comparative example 3, except that 1-(oxetan-3-ylmethyl)-4-(4,4,5,5-tetramethyl-1,3,2-dioxaborolan-2-yl)-1H-pyrazole was used in place of 2-(4-(4,4,5,5-tetramethyl-1,3,2-dioxaborolan-2-yl)-1H-pyrazol-1-yl)ethan-1-ol. The crude product was purified by prep-HPLC (column: 3_Phenomenex Luna C18 75 x 30 mm x 3 um; mobile phase: [water (0.05% HCl) - CH3CN]; B%: 10% - 30%, 7 min). Fractions containing the desired product were combined and lyophilized to give the title compound (66.4 mg, 29.4% yield) as a light yellow solid: LCMS: RT = 0.776 min, m/z = 555.5 (M+H)+. 1H NMR: (400 MHz, d6-DMSO) δ 13.3 (s, 1H), 9.44 (br s, 3H), 8.95 (s, 2H), 8.44 (s, 2H), 8.35 (s, 1H), 7.43 - 7.55 (m, 3H), 7.27 - 7.32 (m, 2H), 4.72 (dd, J = 8.0, 12.0 Hz, 2H), 4.49 (dd, J = 4.8, 11.6 Hz, 2H), 4.09 - 4.19 (m, 4H), 3.95 - 4.07 (m, 4H), 2.02 (s, 3H). Compounds 33, 34, 35, 36, and 37 are prepared using the same procedure and substituting I-2 with the appropriate intermediate.

### Example 7:

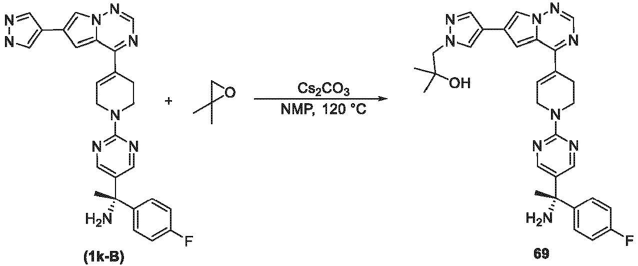

A mixture of 1k-B (prepared according to Preparation 3)(0.4 mmol), Cs₂CO₃ (0.8 mmol) and 2,2-dimethyloxirane (1.2 mmol) in NMP (5 mL) is stirred at 120 °C for 10 h. The reaction mixture is diluted with EA, washed with H₂O and brine, and dried over Na₂SO₄. The organic layer is concentrated in vacuum, and the residue is purified by Prep-HPLC followed by lyophilization to give compound 69. Compounds 70, 71, 72, 73, and 74 are prepared using the same procedure and substituting I-kB with the appropriate intermediate.

### Example 8:

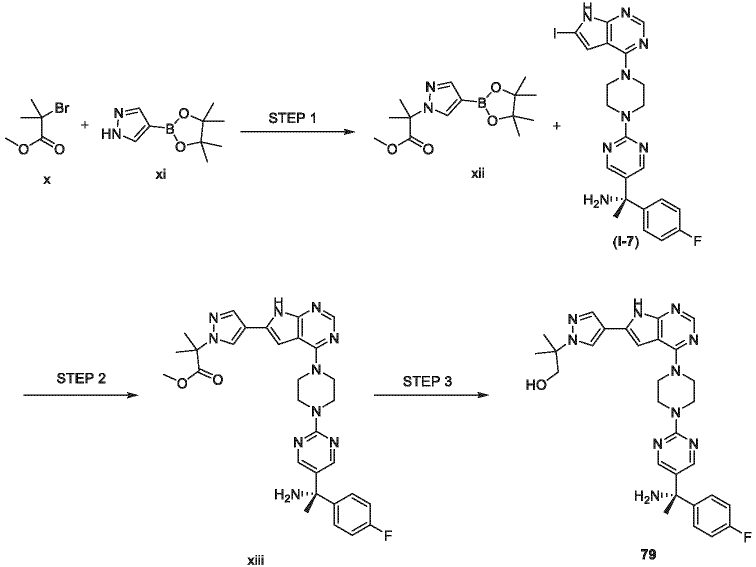

Step 1: To a solution of methyl 2-bromo-2-methylpropanoate (x) (16 mmol) and 4-(4,4,5,5-tetramethyl-1,3,2-dioxaborolan-2-yl)-1H-pyrazole (xi) (16 mmol) in NMP (20 mL) is added cesium carbonate (50 mmol) and sodium iodide (16 mmol) at rt. The resulting mixture is stirred at 120 °C for 8 h. The reaction mixture is diluted with DCM and washed in sequence with H₂O and brine. The organic layer is concentrated *in vacuo,* and the residue is purified by flash column chromatography on silica gel (petroleum ether: ethyl acetate) to afford the compound (xii).

Step 2: A mixture of methyl 2-methyl-2-(4-(4,4,5,5-tetramethyl-1,3,2-dioxaborolan-2-yl)-1H-pyrazol-1-yl)propanoate (xii) (0.6 mmol), I-7 (0.6 mmol), Pd(dppf)Cl₂ (0.12 mmol) and K₂CO₃ (1.8 mmol) in DMF/H₂O (8 mL/2 mL) is stirred at 70 °C for 4 h under N₂ (g). After that, the solution is diluted with DCM, washed with H₂O and brine, and concentrated. The residue is purified by flash column chromatography on silica gel (DCM/MeOH) to afford the compound (xiii).

Step 3: To a solution of (S)-methyl 2-(4-(4-(4-(5-(1-amino-1-(4-fluorophenyl)ethyl)pyrimidin-2-yl)piperazin-1-yl)pyrrolo[2,1-f][1,2,4]triazin-6-yl)-1H-pyrazol-1-yl)-2-methylpropanoate (xiii) (0.34 mmol) in THF (20 mL) is added LiAlH₄ (13.4 mmol) at 0 °C, and the resulting mixture is stirred at rt for 6 h. The reaction mixture is quenched with H₂O (100 mL) and 10% NaOH H₂O (300 mL) then extracted with EA. The organic layer is concentrated *in vacuo,* and the residue is purified by Prep-HPLC followed by lyophilization to afford the compound **(79).** Compounds 75, 76, 77, 78 and 80 are prepared using the same procedure and substituting **I-7** with the appropriate intermediate.

### Example 9:

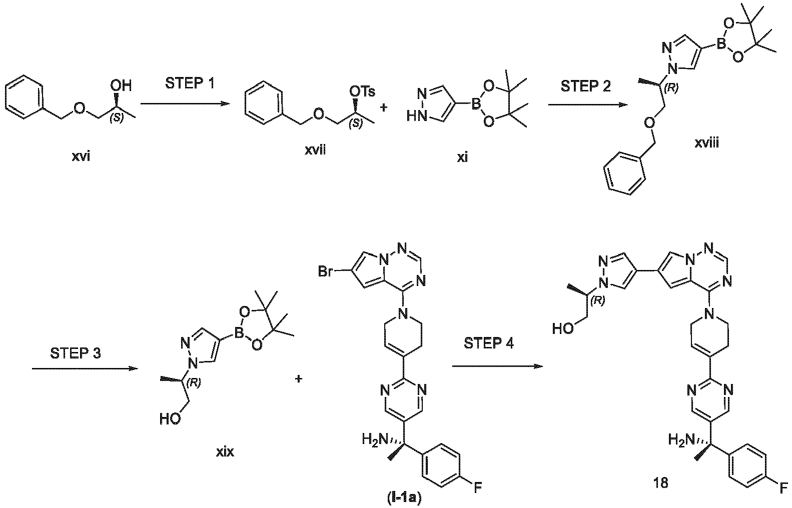

Step 1: To a solution of (S)-1-(benzyloxy)propan-2-ol (xvi)(30 mmol) and TEA (90 mmol) in DCM (80 mL) is added TsCl (33 mmol). The mixture is stirred at rt for 24 h. The solution is diluted with DCM, washed with H₂O, and washed with brine. The organic layer is concentrated, and the residue is purified by flash column chromatography on silica gel (petroleum ether / ethyl acetate) to afford the compound (xvii).

Step 2: A mixture of (S)-1-(benzyloxy)propan-2-yl 4-methylbenzenesulfonate (xvii) (6.2 mmol), 4-(4,4,5,5-tetramethyl-1,3,2-dioxaborolan-2-yl)-1H-pyrazole (xi) (6.2 mmol) and Cs₂CO₃ (13 mmol) in NMP (12 mL) is irradiated at 110 °C by microwave for 0.5 h. After that, the solution is diluted with EA, washed with H₂O, and washed with brine. The organic layer is concentrated, and the residue is purified by flash column chromatography on silica gel (PE/EA) to afford the compound (xviii).

Step 3: To a solution of (R)-1-(1-(benzyloxy)propan-2-yl)-4-(4,4,5,5-tetramethyl-1,3,2-dioxaborolan-2-yl)-1H-pyrazole (xviii) (2.3 mmol) in MeOH (20 mL) is added Pd/C (800 mg) and HOAc (0.2 mL), the solution is purged with H₂ (g) for 5 minutes then stirred at rt under H₂ (g) for 16 h. After that, the mixture is filtered and the filtrate is concentrated to give the compound (xix).

Step 4: A mixture of ((R)-2-(4-(4,4,5,5-tetramethyl-1,3,2-dioxaborolan-2-yl)-1H-pyrazol-1-yl)propan-1-ol (xix) (595 µmol), I-1a (595 µmol), Pd(dppf)Cl₂ (60 µmol) and K₂CO₃ (1.8 mmol) in DMF/H₂O (4 mL /1 mL) is purged with N₂ (g) for 10 mins and stirred at 70 °C for 16 h under N₂ (g). The mixture is extracted with EA, and the combined organic extracts are concentrated. The residue is purified by flash column chromatography on silica gel (DCM/MeOH). Compounds 16, 17, 19, 20, and 21 are prepared using the same procedure and substituting I-1a with the appropriate intermediate.

### Example 10:

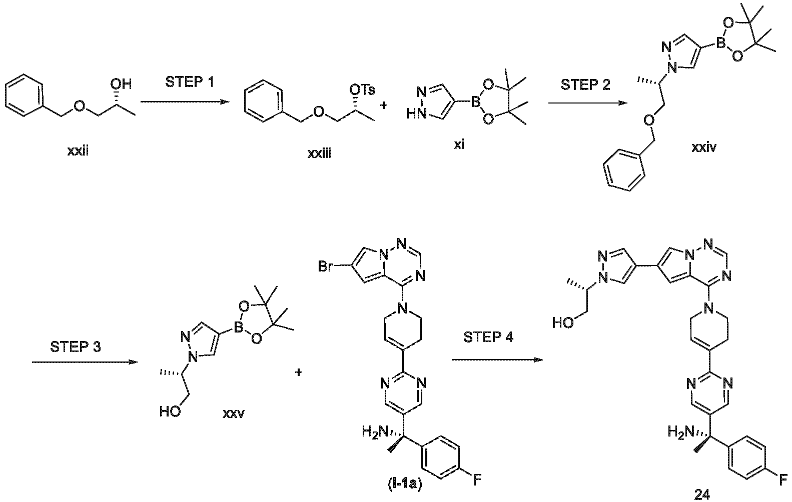

Step 1: To a solution of (R)-1-(benzyloxy)propan-2-ol (xxii) (18 mmol) and TEA (54 mmol) in DCM (30 mL) is added TsCl (22 mmol). The resulting mixture is stirred at 25 °C for 16 h. The mixture is then concentrated *in vacuo,* and the residue is purified by flash column chromatography on silica gel (petroleum ether / ethyl acetate) to afford the compound (xxiii).

Step 2: A mixture of (R)-1-(benzyloxy)propan-2-yl 4-methylbenzenesulfonate (xxiii) (6.9 mmol), 4-(4,4,5,5-tetramethyl-1,3,2-dioxaborolan-2-yl)-1H-pyrazole (xi) (10 mmol) and Cs₂CO₃ (6.9 mmol) in NMP (50 mL) is stirred at 110 °C by in the microwave for 16 h. After that, the solution is diluted with EA, washed with H₂O and brine, and concentrated. The residue is purified by flash column chromatography on silica gel (PE /EA) to afford the compound (xxiv).

Step 3: A mixture of (S)-1-(1-(benzyloxy)propan-2-yl)-4-(4,4,5,5-tetramethyl-1,3,2-dioxaborolan-2-yl)-1H-pyrazole (xxiv) (2.6 mmol) in MeOH (20 mL) is added Pd/C (800 mg) and HOAc (0.2 mL). The resulting mixture is purged with H₂ (g) for 5 min then stirred at rt under H₂ (g) for 16 h. After that, the mixture is filtered and concentrated to afford the compound (xxv).

Step 4: A mixture of (S)-2-(4-(4,4,5,5-tetramethyl-1,3,2-dioxaborolan-2-yl)-1H-pyrazol-1-yl)propan-1-ol (xxv) (392 µmol), I-1 (261 µmol), K₂CO₃ (227 µmol) and Pd(dppf)Cl₂ (7 µmol) in DMF/H₂O (5 mL/1 ml) is stirred at 70 °C under N₂ (g) for 4 h. After that, the solution is diluted with EA, washed with H₂O and brine, and concentrated. The residue is purified by Prep-HPLC followed by lyophilization to afford the compound **(24).** Compounds 22, 23, 25, 26, and 27 are prepared using the same procedure and substituting I-1a in Step 4 with the appropriate intermediate.

### Example 11:

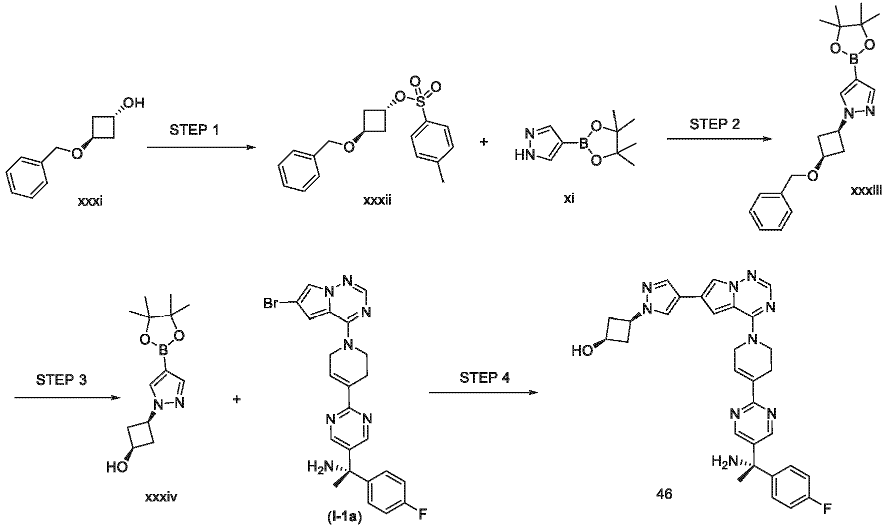

To a solution of trans-3-(benzyloxy)cyclobutanol (xxxi) (1.7 mmol) in DCM (20 mL) is added TsCl (2.0 mmol) and TEA (3.4 mmol). The mixture is stirred at rt for 16 h. The solution is diluted with DCM, washed with H₂O and brine, then concentrated. The residue is purified by flash column chromatography on silica gel (PE/EA) to afford the compound (xxxii).

Step 2: A mixture of trans-3-(benzyloxy)cyclobutyl 4-methylbenzenesulfonate (xxxii) (0.95 mmol), 4-(4,4,5,5-tetramethyl-1,3,2-dioxaborolan-2-yl)-1H-pyrazole (xi) (0.95 mmol), and Cs₂CO₃ (1.9 mmol) in NMP (5 mL) is irradiated at 110 °C by microwave for 0.5 h. After that, the solution is diluted with EA and washed with H₂O and brine. The organic layer is concentrated *in vacuo,* and the residue is purified by flash column chromatography on silica gel (PE/EA) to afford the title compound (xxxiii).

Step 3: To a solution of cis-3-(benzyloxy)cyclobutyl)-4-(4,4,5,5-tetramethyl-1,3,2-dioxaborolan-2-yl)-1H-pyrazole (xxxiii) (0.54 mmol) in MeOH (5 mL) is added Pd/C (200 mg) and HOAc (5 drops), the solution is purged with H₂ (g) for 5 min and stirred at rt under H₂ (g) for 16 h. The mixture is filtered, and the filtrate is evaporated to dryness *in vacuo* to afford the compound (xxxiv).

Step 4: A mixture of cis-3-(4-(4,4,5,5-tetramethyl-1,3,2-dioxaborolan-2-yl)-1H-pyrazol-1-yl)cyclobutanol (xxxiv) (0.21 mmol), I-1a (0.21 mmol), Pd(dppf)Cl₂ (0.021 µmol) and K₂CO₃ (0.63 mmol) in DMF/H₂O (4 mL/1 mL) is purged with N₂ for 10 min and stirred at 70 °C for 16 h under N₂ (g). After that, the solution is diluted with EA, washed with H₂O and brine, and concentrated. The residue is purified directly by flash column chromatography (DCM/MeOH). The resulting material is purified further by Prep-HPLC followed by lyophilization to afford the compound **(46).** Compounds 44, 45, 47, 48, and 49 are prepared using the same procedure and substituting I-1a in Step 4 with the appropriate intermediate.

### Example 12:

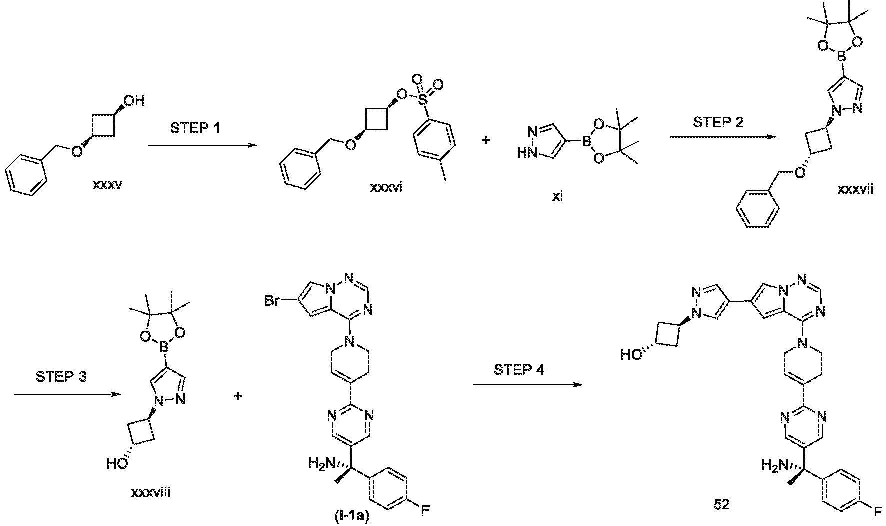

Step 1: To a solution of cis-3-benzyloxy-cyclobutanol (xxxv) (2.8 mmol) and TEA (8.4 mmol) in DCM (10 mL) is added 4-methyl-benzenesulfonyl chloride (3.4 mmol), and the resulting mixture is stirred at rt for 16 h. The mixture is diluted with brine and extracted with DCM. The organic extract is concentrated. The residue is purified directly by flash column chromatography on silica gel (PE/EA) to afford the compound (xxxvi).

Step 2: A mixture of cis-toluene-4-sulfonic acid 3-benzyloxy-cyclobutyl ester (xxxvi) (1.5 mmol), 4-(4,4,5,5-tetramethyl-[1,3,2]dioxaborolan-2-yl)-1H-pyrazole (xi) (2.2 mmol), and Cs₂CO₃ (4.5 mmol) in NMP (15 mL) is irradiated by microwave at 120 °C for 2 h. After that, the solution is diluted with EA, washed with H₂O and brine, and concentrated. The residue is purified by flash column chromatography on silica gel (PE/EA) to afford the compound (xxxvii).

Step 3: To a solution of trans-1-(3-benzyloxy-cyclobutyl)-4-(4,4,5,5-tetramethyl-[1,3,2]dioxaborolan-2-yl)-1H-pyrazole (xxxvii) (1.2 mmol) in MeOH (10 mL) is added Pd/C (200 mg) and concentrated HCl (0.5 mL). The reaction mixture is stirred under H₂ (g) at rt for 16 h. The mixture is filtered, and the filtrate is concentrated to afford the compound (xxxviii).

Step 4: A mixture of trans-3-[4-(4,4,5,5-tetramethyl-[1,3,2]dioxaborolan-2-yl)-pyrazol-1-yl]-cyclobutanol (xxxviii) (0.8 mmol), I-1a (0.8 mmol), Pd(dppf)Cl₂ (0.08 mmol) and K₂CO₃ (2.3 mmol) in dioxane/H₂O (4 mL/1 mL) is purged with N₂ (g) for 10 mins and stirred at 70 °C for 4 h under N₂ (g). After that, the solution is diluted with EA, washed with H₂O and brine, and concentrated. The residue is purified by flash chromatography on silica gel. The resulting material is purified further by Prep-HPLC followed by lyophilization to afford the compound **(52).** Compounds 50, 51, 53, 54, and 55 are prepared using the same procedure and substituting I-1a in Step 4 with the appropriate intermediate.

### Example 13:

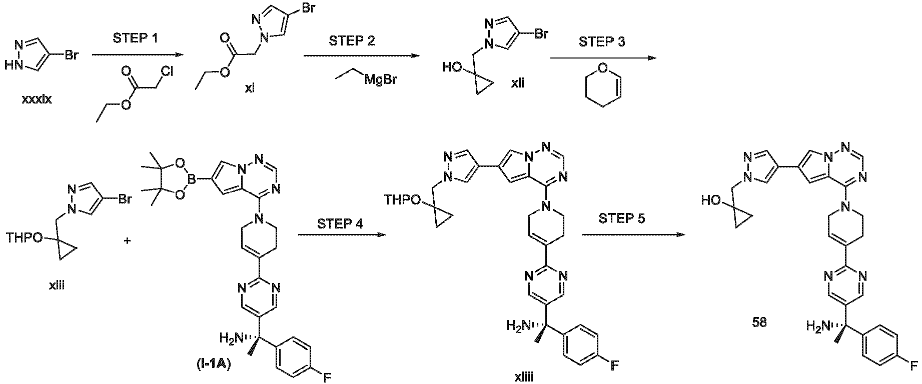

Step 1: A mixture of 4-bromo-1H-pyrazole (xxxix) (55 mmol) and K₂CO₃ (110 mmol) in ethyl 2-chloroacetate (25 mL) is stirred at 80 °C for 15 h. The reaction mixture is cooled, diluted with EA, and washed with H₂O. The organic layer is evaporated, and the residue is purified by chromatography on silica gel (petroleum ether/ethyl acetate) to give the compound (xl).

Step 2: To a solution of ethyl 2-(4-bromo-1H-pyrazol-1-yl)acetate (xl) (30 mmol) and titanium tetraisopropanolate (15 mmol) in anhydrous THF (60 mL) is added a solution of ethyl magnesium bromide (3 M in hexane, 30 mL, 90 mmol) dropwise at 60 °C over 2 h. After stirring at same temperature for 2 h, the reaction mixture is diluted with EA and washed sequentially with 1N aq. HCl and H₂O. The organic layer is evaporated, and the residue is purified by chromatography on silica gel (petroleum ether/ethyl acetate) to give the compound (xli).

Step 3: To a solution of 1-[(4-bromo-1H-pyrazol-1-yl)methyl]cyclopropan-1-ol (xli) (1.4 mmol) and 3,4-dihydro-2H-pyran (4.1 mmol) in DCM (8 mL) is added pyridinium para-toluene sulfonate (1.4 mmol) at rt. The mixture is stirred for 4 h, then is diluted with brine and washed with DCM. The organic layer is concentrated, and the residue is purified by chromatography on silica gel (PE/EA) to obtain the compound (xlii).

Step 4: A mixture of 4-bromo-1-{[1-(oxan-2-yloxy)cyclopropyl]methyl}-1H-pyrazole (xlii) (0.5 mmol), I-1A (1.1 mmol), Pd(dppf)Cl₂ (106 µmol) and Na₂CO₃ (1.6 mmol) in a mixture of 1,4-dioxane (3 ml), H₂O (1 mL) and DMF (0.5 mL) is stirred at 80 °C for 3 h under N₂ (g). After that, the solution is diluted with EA, washed with H₂O and brine, and concentrated. The residue is purified by chromatography on silica gel (ethyl acetate / methanol) to give the compound (xliii).

Step 5: To a solution of 1-(4-fluoro-phenyl)-1-{2-[4-(6-{1-[1-(tetrahydro-pyran-2-yloxy)-cyclopropylmethyl]-1H-pyrazol-4-yl}-pyπolo[2,1-f][1,2,4]triazin-4-yl)-piperazin-1-yl]-pyrimidin-5-yl}-ethylamine (xliii) (0.32 mmol) in MeOH (4 mL) is added p-toluenesulfonic acid (1.0 mmol) at rt, and the resulting mixture is stirred for 2 h. The reaction mixture is concentrated, and the residue is purified by Prep-HPLC followed by lyophilization to give the compound **(58).** Compounds 56, 57, 60, 61, and 62 are prepared using the same procedure and substituting I-1a in Step 4 with the appropriate intermediate.

### Example 14:

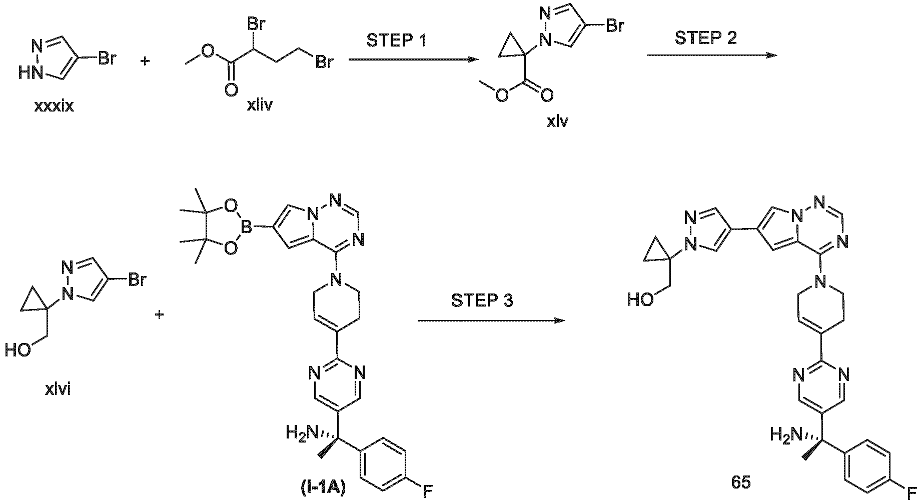

Step 1: To a solution of 4-bromo-1H-pyrazole (xxxix) (14 mmol) in THF (50 mL) is added NaH (30 mmol) at 0 °C. The solution is stirred at rt for 1 h, then methyl 2,4-dibromobutanoate (xliv) (14 mmol) is added to the solution. The mixture is stirred for 16 h, then diluted with EA. The organic layer is washed with H₂O, washed with brine, and concentrated *in vacuo.* The residue is purified by flash column chromatography on silica gel (petroleum ether/ethyl acetate) to afford the compound (xiv).

Step 2: To a solution of methyl 1-(4-bromo-1H-pyrazol-1-yl)cyclopropanecarboxylate (xiv) (2.3 mmol) in MeOH (15 mL) is added NaBH₄ (6.8 mmol), and the resulting mixture is stirred at 50 °C until completion. The reaction mixture is diluted with DCM, washed in sequence with H₂O and brine, and concentrated *in vacuo.* The residue is purified by flash column chromatography on silica gel (PE/EA) to afford the compound (xlvii).

Step 3: A mixture of (1-(4-bromo-1H-pyrazol-1-yl)cyclopropyl)methanol (xlvii) (463 µmol), I-1A (prepared as described in preparation 1) (695 µmol), Pd(t-Bu₃P)₂ (93 µmol) and Cs₂CO₃ (1.4 mmol) in THF/H₂O (8 mL/2 mL) is purged with N₂ (g) for 10 min and stirred at 80 °C for 12 h under N₂ (g). After that, the solution is diluted with EA, washed with H₂O and brine, and concentrated. The residue is purified by flash column chromatography (DCM/MeOH). The resulting material is purified further by Prep-HPLC followed by lyophilization to afford the compound **(65).** Compounds 63, 64, 66, 67, and 68 are prepared using the same procedure and substituting I-1A in Step 3 with the appropriate intermediate.

### Example 15:

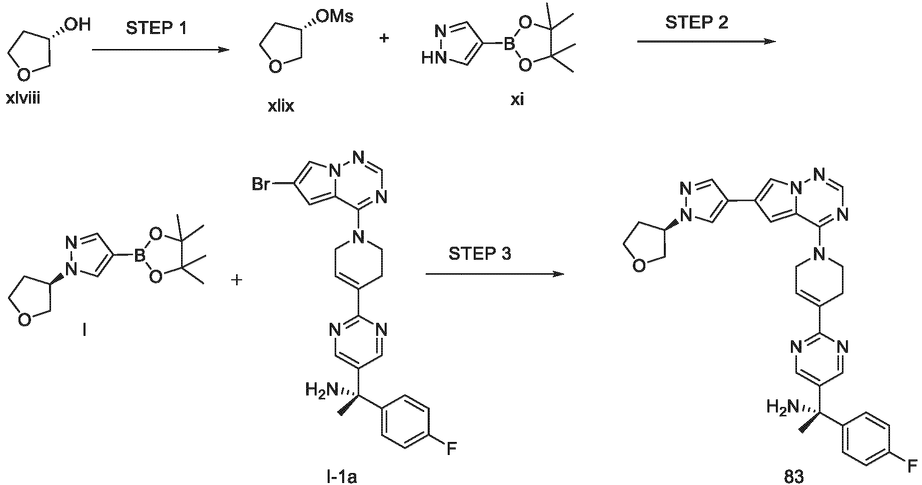

Step 1: To a solution of tetrahydro-furan-3-ol (xlviii)(23 mmol) and TEA (45 mmol) in DCM (20 mL) is added MsCl (25 mmol) at rt. The mixture is stirred at rt for 16 h. The reaction mixture is then diluted with DCM, washed in sequence with H₂O and brine, dried over anhydrous Na₂SO₄, and concentrated to dryness to afford the compound (xlix).

Step 2: To a solution of (S)-tetrahydrofuran-3-yl methanesulfonate (xlviii)(11 mmol) in NMP (50 mL) is added 4-(4,4,5,5-tetramethyl-[1,3,2]dioxaborolan-2-yl)-1H-pyrazole (xi) (17 mmol) and Cs₂CO₃ (34 mmol) at rt. The mixture is stirred at 120 °C for 2 h. The solution is diluted with EA, washed in sequence with H₂O and brine, and concentrated *in vacuo.* The residue is purified by flash column chromatography on silica gel (PE/EA) to afford the compound (l).

Step 3: A mixture of (R)-1-(tetrahydro-furan-3-yl)-4-(4,4,5,5-tetramethyl-[1,3,2]dioxaborolan-2-yl)-1H-pyrazole (l) (0.3 mmol), I-1a (0.3 mmol), Pd(dppf)Cl₂ (0.06 mmol), and K₂CO₃ (0.9 mmol) in DMF (2 mL) and H₂O (0.5 mL) is stirred at 80 °C under reaction completion under N₂ (g). After that, the solution is diluted with EA, washed with H₂O and brine, and concentrated. The residue is purified by flash column chromatography on silica gel (DCM/MeOH). The resulting material is subsequently purified by Prep-HPLC followed by lyophilization to afford the compound **(83).** Compounds 81, 82, 84, 85, and 86 are prepared using the same procedure and substituting I-1a in Step 3 with the appropriate intermediate.

### Example 16:

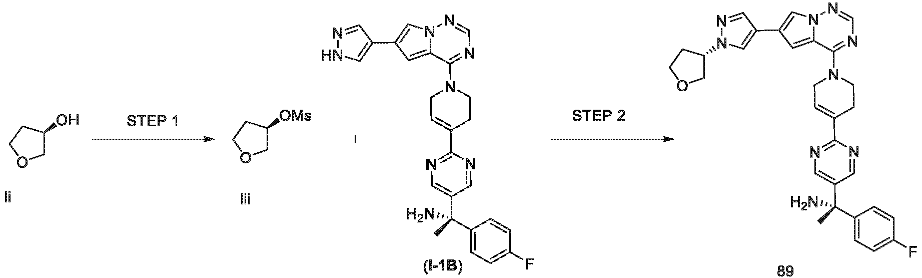

Step 1: To a solution of (R)-tetrahydrofuran-3-ol (li)(11 mmol) and TEA (23 mmol) in DCM (20 mL) is added MsCl (12.5 mmol) at rt and the resulting mixture is stirred at rt for 6 h. The reaction mixture is diluted with DCM, washed in sequence with H₂O and brine, and concentrated to afford the compound (lii).

Step 2: A mixture of I-1B (0.6 mmol), (R)-tetrahydrofuran-3-yl methanesulfonate (lii) (0.9 mmol) and Cs₂CO₃ (1.9 mmol) in NMP (10 mL) is stirred at 120 °C under reaction completion by tlc. After that, the solution is diluted with EA, washed with H₂O and brine, and concentrated. The residue is purified directly by Prep-HPLC followed by lyophilization to afford the compound **(89).** Compounds 87, 88, 90, 91, and 92 are prepared using the same procedure and substituting I-1B in Step 2 with the appropriate intermediate.

### Example 17:

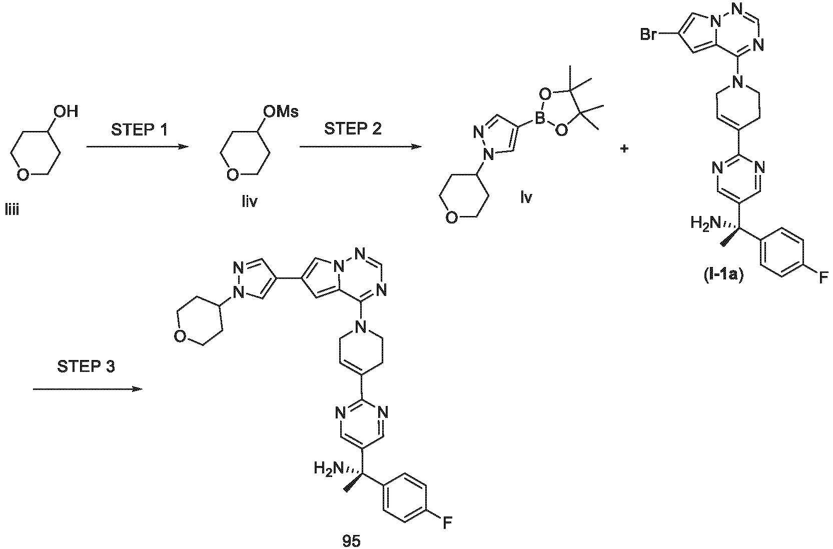

Step 1: To a solution of tetrahydro-2H-pyran-4-ol (liii)(31 mmol) and TEA (94 mmol) in DCM (100 mL) is added MsCl (47 mmol) at 0 °C. The reaction is stirred at rt for 3 h, then diluted with DCM, washed with saturated aq. Na₂CO₃ solution, and dried with anhydrous Na₂SO₄. The solvent is removed to afford the compound (liv).

Step 2: A mixture of tetrahydro-2H-pyran-4-yl methanesulfonate (liv) (18 mmol), 4-(4,4,5,5-tetramethyl-1,3,2-dioxaborolan-2-yl)-1H-pyrazole (21 mmol) and Cs₂CO₃ (27 mmol) in NMP (50 mL) is stirred at 80 °C for 4 h. The reaction mixture is diluted with DCM and washed with brine. The organic layer is evaporated *in vacuo.* The residue is purified by flash column chromatography on silica gel (PE/EA) to afford the compound (lv).

Step 3: A mixture of I-1a (603 µmol), 1-(tetrahydro-2H-pyran-4-yl)-4-(4,4,5,5-tetramethyl-1,3,2-dioxaborolan-2-yl)-1H-pyrazole (lv) (754 µmol), K₂CO₃ (754 µmol), and Pd(dppf)Cl₂ (41 µmol) in DMF/H₂O (10 mL/2 ml) is stirred at 70 °C under N₂ (g) for 4 hrs. After that, the solution is diluted with EA, washed with H₂O and brine, and concentrated. The residue is purified by Prep-HPLC followed by lyophilization to afford the compound (95). Compounds 93, 94, 96, 97, and 98 are prepared using the same procedure and substituting I-la in Step 3 with the appropriate intermediate.

### Example 18:

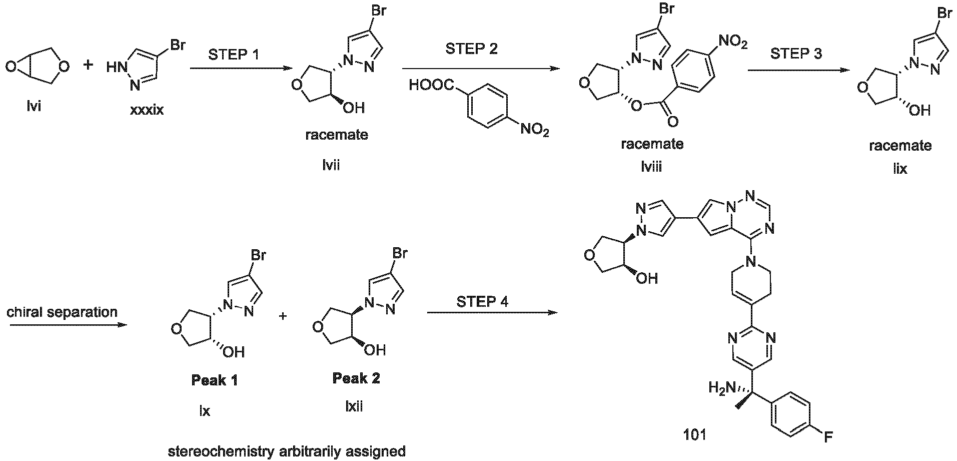

Step 1: To a solution of 3,6-dioxabicyclo[3.1.0]hexane (lvi) (61 mmol), 4-bromo-1H-pyrazole (xxxix) (61 mmol) and Cs₂CO₃ (121 mmol) in NMP (100 mL) is stirred at 120 °C for 16 h. The solution is cooled and diluted with DCM, then washed with H₂O and brine. The organic layer is concentrated and purified by flash column chromatography on silica gel (PE/EA) to afford the compound (lvii).

Step 2: A mixture of rac-trans-4-(4-bromo-1H-pyrazol-1-yl)tetrahydrofuran-3-ol (lvii) (12 mmol), 4-nitrobenzoic acid (12 mmol), diisopropyl azodicarboxylate (17 mmol), and triphenylphosphine (17 mmol) in THF (50 mL) is stirred at rt for 16 h. The solution is diluted with EA and washed with H₂O and brine. The organic layer is concentrated and purified by flash column chromatography on silica gel (PE/EA) to afford the compound (lviii).

Step 3: A mixture of rac-cis-4-(4-bromo-1H-pyrazol-1-yl)tetrahydrofuran-3-yl 4-nitrobenzoate (lvii) (11 mmol) and lithium hydroxide (53 mmol) in MeOH/THF/H₂O (30 mL/30 mL/30 mL) is stirred at rt for 4 h. The resulting mixture is diluted with EA, washed with H₂O and brine, and concentrated *in vacuo.* The residue is purified by flash column chromatography on silica gel (PE/EA) to afford rac-cis-4-(4-bromo-1H-pyrazol-1-yl)tetrahydrofuran-3-ol. This material is subjected to chiral separation via SFC (Column: AD 20*250mm, 10µm (Daicel); Mobile Phase: CO₂/MeOH (0.2% ammonia in methanol) = 60/40; Flow Rate: 80 g/min) to afford Peak 1 (lx) and Peak 2 (lxi). Peak 1 is arbitrarily assigned as (3S,4S)-4-(4-bromo-1H-pyrazol-1-yl)tetrahydrofuran-3-ol and Peak 2 is arbitrarily assigned as (3R,4R)-4-(4-bromo-1H-pyrazol-1-yl)tetrahydrofuran-3-ol.

Step 4: A mixture of (3R,4R)-4-(4-bromo-1H-pyrazol-1-yl)tetrahydrofuran-3-ol (lxi) (70 mg, 0.3 mmol) (Peak 2 from Step 3), I-1A (0.6 mmol), Pd[(t-Bu)₃P]₂ (0.06 mmol) and Na₂CO₃ (0.9 mmol) in dioxane/H₂O (8 mL/2 mL) is stirred at 90 °C for 4 h. After cooling, the solution is diluted with EA, washed with H₂O and brine, and concentrated. The residue is purified by flash column chromatography on silica gel (DCM/MeOH = 10/1) to afford the compound **(101).** Compounds 99, 100, 102, 103, and 104 are prepared using the same procedure and substituting I-1A in Step 4 with the appropriate intermediate.

### Example 19:

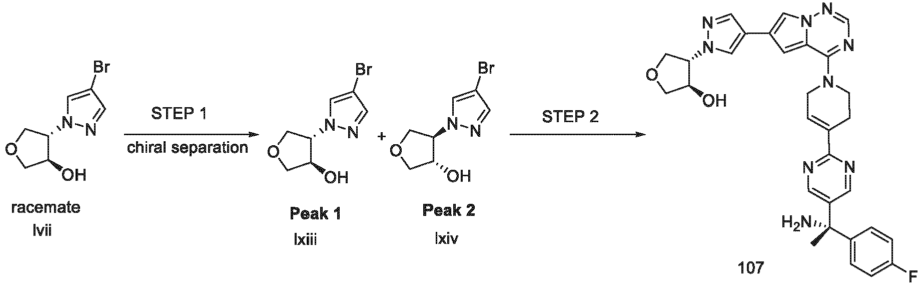

Step 1: rac-trans-4-(4-bromo-1H-pyrazol-1-yl)tetrahydrofuran-3-ol (1.1 g) (from Step 1 of Example 18) is subjected to chiral separation via SFC (Column: AD 20*250mm, 10µm (Daicel); Mobile Phase: CO₂/MeOH (0.2% ammonia in MeOH) = 80/20; Flow Rate: 80 g/min) to afford Peak 1 (lxiii) and Peak 2 (lxiv). Peak 1 is arbitrarily assigned as (3R,4S)-4-(4-bromo-1H-pyrazol-1-yl)tetrahydrofuran-3-ol and Peak 2 is arbitrarily assigned as (3S,4R)-4-(4-bromo-1H-pyrazol-1-yl)tetrahydrofuran-3-ol.

Step 2: A mixture of (3R,4S)-4-(4-bromo-1H-pyrazol-1-yl)tetrahydrofuran-3-ol (0.3 mmol) (lxiii)(Peak 1 from Step 1), I-1A (0.6 mmol), Pd[(t-Bu)₃P]₂ (0.06 mmol) and Na₂CO₃ (0.9 mmol) in dioxane/H₂O (8 mL/2 mL) is degassed with N₂ and stirred at 90 °C for 4 h. After that, the solution is diluted with DCM, washed with H₂O and brine, and concentrated. The residue is purified by flash column chromatography on silica gel (DCM/MeOH) to afford the title compound **(107).** Compounds 105, 106, 108, 109, 110 are prepared using the same procedure and substituting I-1a in Step 2 with the appropriate intermediate.

### Example 20:

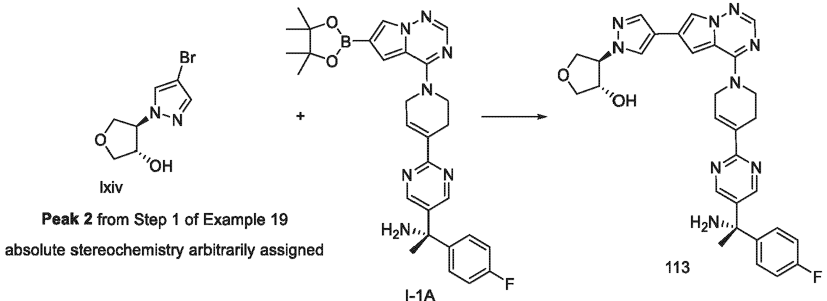

A mixture of (3S,4R)-4-(4-bromo-1H-pyrazol-1-yl)tetrahydrofuran-3-ol (70 mg, 0.3 mmol) (lxiv) (Peak 2 from Step 1 of Example 19), I-1A (0.6 mmol), Pd[(t-Bu)₃P]₂ (0.06 mmol) and Na₂CO₃ (0.9 mmol) in dioxane/H₂O (8 mL/2 mL) is degassed with N₂ and stirred at 90 °C for 4 h. After that, the solution is diluted with EA, washed with H₂O and brine, and concentrated. The residue is purified by flash column chromatography on silica gel (DCM/MeOH) to afford the compound (113). Compounds 111, 112, 114, 115, and 116 are prepared using the same procedure and substituting I-1A with the appropriate intermediate.

### Example 21:

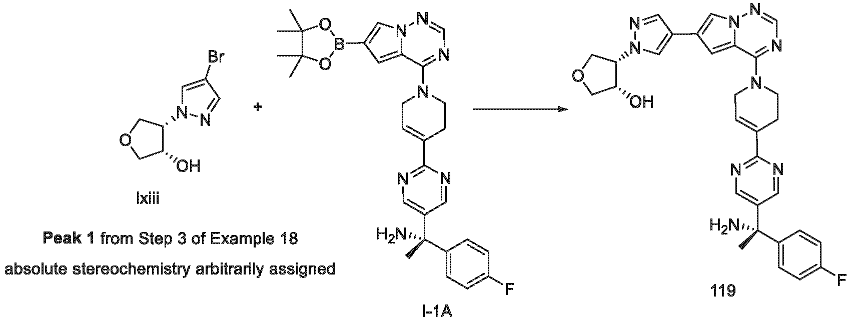

A mixture of (3S,4S)-4-(4-bromo-1H-pyrazol-1-yl)tetrahydrofuran-3-ol (lxiii) (0.22 mmol) (Peak 1 from Step 3 of Example 18), I-1A (0.44 mmol), Pd[(t-Bu)₃P]₂ (0.044 mmol) and Na₂CO₃ (0.66 mmol) in dioxane/H₂O (8 mL/2 mL) is stirred at 90 °C for 4 h. After cooling, the solution is diluted with EA, washed with H₂O and brine, and concentrated. The residue is purified by flash column chromatography on silica gel (DCM/MeOH) to afford the title compound (119). Compounds 117, 118, 120, 121, and 122 are prepared using the same procedure and substituting I-1A with the appropriate intermediate.

### Example 22:

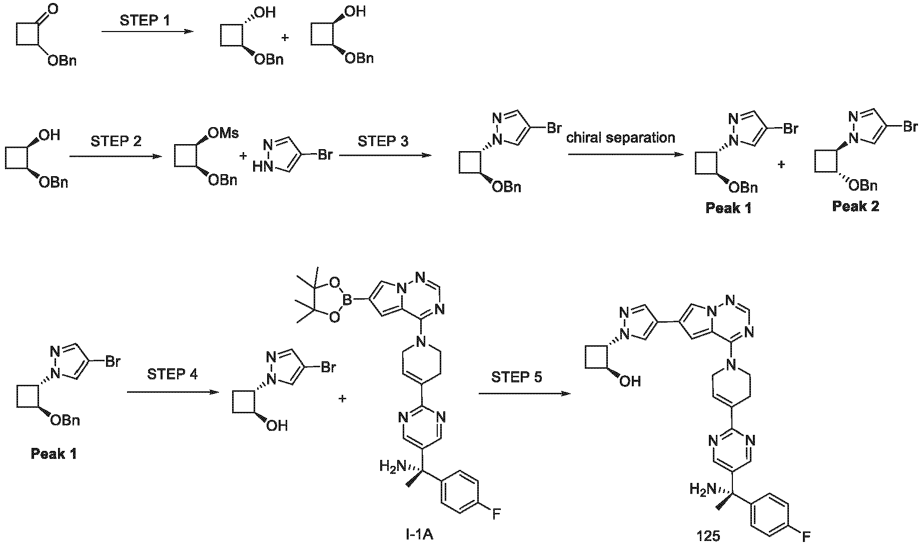

Step 1: To a solution of 2-(benzyloxy)cyclobutanone (5.7 mmol) in MeOH (20 mL) is added NaBH₄ (11.4 mmol) at 0 °C. Then the solution is stirred at rt for 3 h. The mixture is diluted with EA, washed with water and brine, then the organic layer is concentrated and purified by flash column chromatography on silica gel (PE/EA) to afford Peak 1 (arbitrarily assigned as cis-2-(benzyloxy)cyclobutanol) as a colorless oil and Peak 2 (arbitrarily assigned as trans-2-(benzyloxy)cyclobutanol).

Step 2: To a solution of cis-2-(benzyloxy)cyclobutanol (1.5 mmol) in DCM (10 mL) is added mesyl chloride (2.3 mmol) and triethylamine (4.6 mmol) at 0 °C. The mixture is stirred at rt for 3 h. After that, the solution is diluted with DCM, washed with water and brine, dried over anhydrous Na₂SO₄, and concentrated to afford the desired compound.

Step 3: A mixture of cis-2-(benzyloxy)cyclobutyl methanesulfonate (1.2 mmol), 4-bromo-1H-pyrazole (1.2 mmol), and Cs₂CO₃ (3.5 mmol) in DMF (8 mL) is stirred at 100 °C for 16 h. After that, the solution is diluted with EA, washed with water and brine, dried over anhydrous Na₂SO₄, concentrated and purified by flash column chromatography (PE/EA) to afford the desired compound. Chiral separation of trans-2-(benzyloxy)cyclobutyl)-4-bromo-1H-pyrazole: trans-2-(benzyloxy)cyclobutyl)-4-bromo-1H-pyrazole is subjected to chiral separation via SFC (Column: IG 20*250mm, 10µm (Daicel); Mobile Phase: CO₂/MeOH (0.2% ammonia in methanol) = 75/25; Flow Rate: 4 g/min) to afford Peak 1 (250 mg) and Peak 2 (250 mg). Peak 1 is arbitrarily assigned as 1-((1S,2S)-2-(benzyloxy)cyclobutyl)-4-bromo-1H-pyrazole and peak 2 is arbitrarily assigned as 1-((1R,2R)-2-(benzyloxy)cyclobutyl)-4-bromo-1H-pyrazole.

Step 4: To a solution of 1-((1S,2S)-2-(benzyloxy)cyclobutyl)-4-bromo-1H-pyrazole (820 µmol) in TFA (2 mL) is stirred at 80 °C for 16 h. After that, the solution is concentrated and purified by flash column chromatography on silica gel (petroleum ether/ethyl acetate) to afford the desired compound.

Step 5: A mixture of (1S,2S)-2-(4-bromo-1H-pyrazol-1-yl)cyclobutanol (556 µmol), I-1A (667 µmol), Pd(t-Bu₃P)₂ (99 µmol) and Cs₂CO₃ (1.1 mmol) in dioxane/H₂O (8 mL/2 mL) is purged with N₂ for 10 mins and stirred at 90 °C for 4 hrs under N₂. After that, the solution is diluted with DCM, washed with H₂O and brine, and concentrated. The residue is purified by flash column chromatography on silica gel (DCM/MeOH). The resulting material is purified further by Prep-HPLC followed by lyophilization to afford the compound 125. Compounds 123, 124, 126, 127, and 128 are prepared using the same procedure and substituting I-1A in Step 5 with the appropriate intermediate.

### Example 23:

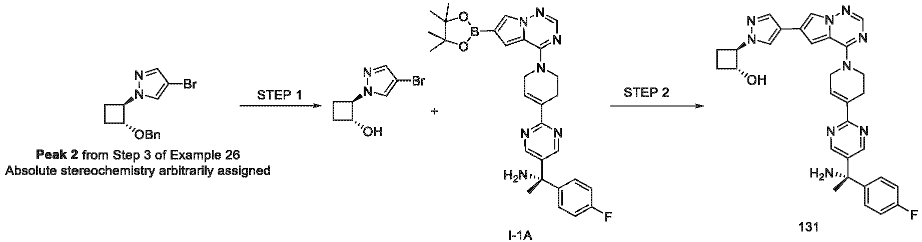

Step 1: To a solution of 1-((1R,2R)-2-(benzyloxy)cyclobutyl)-4-bromo-1H-pyrazole (820 µmol) (from Peak 2 in Step 3 of Example 22) in TFA (2 mL) is stirred at 80 °C for 16 h. After that, the solution is concentrated and purified by flash column chromatography on silica gel (petroleum ether/ethyl acetate) to afford the desired compound. Step 2: A mixture of (1R,2R)-2-(4-bromo-1H-pyrazol-1-yl)cyclobutanol (556 µmol), I-1A (667 µmol), Pd(t-Bu₃P)₂ (99 µmol) and Cs₂CO₃ (1.1 mmol) in dioxane/H₂O (8 mL/2 mL) is purged with N₂ (g) for 10 min and stirred at 90 °C for 4 h under N (g). After that, the solution is diluted with EA, washed with H₂O and brine, and concentrated. The residue is purified by flash column chromatography on silica gel (DCM/MeOH). The resulting material is purified further by Prep-HPLC followed by lyophilization to afford the compound 131. Compounds 129, 130, 132, 133, and 134 are prepared using the same procedure and substituting I-1A with the appropriate intermediate.

### BIOLOGICAL EXAMPLE 1: Biochemical Enzymatic Activity Inhibition Assays

PDGFRα and KIT enzymatic activity was monitored using the Perkin Elmer electrophoretic mobility shift technology platform, the EZReader 2. Fluorescent labeled substrate peptide was incubated in the presence of kinase and ATP, and in the presence of test compound, such that each dose of test compound resulted in a reflective proportion of the peptide to be phosphorylated.

Within the linear, steady-state phase of the kinase enzymatic reaction, the mixed pool of phosphorylated (product) and non-phosphorylated (substrate) peptides was passed through the microfluidic system of the PerkinElmer EZ Reader 2, under an applied electric potential difference. The presence of the phosphate group on the product peptide provided a difference in mass and charge between that of the substrate peptide, resulting in a separation of the substrate and product pools in the sample (Perrin et al., Expert Opin Drug Discovery 2010, Jan 5(1):51-63).

As the product and substrate peptide mixture passes the lasers within the instrument, these pools are detected (λₑₓ = 488 nm, λₑₘ = 568 nm) and resolved as separate peaks. The ratio between these peaks reflects the activity of the compound at that concentration, in that well, under those conditions.

### Inhibition of KIT (D816V) Mutant Biochemical Enzymatic Activity Inhibition

All test articles were dissolved in 100% DMSO at a stock concentration of 10 mM. A 100X, 10-point, 4-fold serial dilution of all test compounds was created in 100% DMSO, starting at a relevant concentration, usually 1 mM. A volume of 130 nL of each concentration was transferred to the relevant well of a 384-well assay plate (Greiner 781 201) using a TTPLabtech Mosquito nano-liter dispenser. Using the Multidrop, the remaining constituents of the reaction were then added to the 130 nL of compound as follows:

KIT D816V assay at the APPKM for ATP: In each well of a 384-well assay plate, 0.3 nM of untreated enzyme was incubated in a total of 13 µL of buffer (100 mM HEPES pH 7.5, 0.015% Brij 35, 10 mM MgCl₂, 1mM DTT) with 1 µM Src tide (5-FAM-GEEPLYWSFPAKKK-NH2) and 20 µM ATP at 25°C for 60 minutes in the presence or absence of a dosed concentration series of compound (1% DMSO final concentration). The reaction was stopped by the addition of 70 µl of Stop buffer (100 mM HEPES pH 7.5, 0.015% Brij 35, 35 mM EDTA and 0.2% of Coating Reagent 3, Caliper Lifesciences). The plate was read on a Caliper EZReader 2. The results obtained in these experiments for compounds prepared according to the examples are summarized in Table 2 below. For biochemical D816V and D842V activity, the following designations are used: ≤ 0.30 nM = A; ≥ 0.31 and < 1.4 nM = B; ≥ 1.4 nM = C; and ND = not determined.

**Table 2.**

| Example | Kit D816V @ ATP Km IC₅₀ (nM) |
|---|---|
| 1 | B |
| 2 | A |
| 3 | A |
| 4 | B |
| 5 | B |
| 6 | B |
| Comparator A | A |

For reference, the chemical structure of Comparator A is:

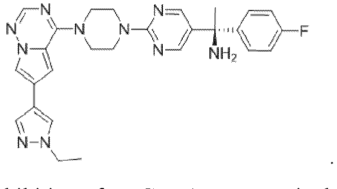

### Inhibition of PDGFRA Mutant Biochemical Enzymatic Activity Inhibition

All test articles are dissolved in 100% DMSO at a stock concentration of 10 mM. A 100X, 10-point, 4-fold serial dilution of all test compounds are created in 100% DMSO, starting at a relevant concentration, usually 1 mM. A volume of 130 nL of each concentration is transferred to the relevant well of a 384-well assay plate (Greiner 781 201) using a TTPLabtech Mosquito nano-liter dispenser. Using the Multidrop, the remaining constituents of the reaction are then added to the 130 nL of compound as follows:

PDGFRα D842V assay at the apparent Michaelis-Menten constant (APPKM) for ATP: In each well of a 384-well assay plate, 7 nM of untreated enzyme are incubated in a total of 13 µL of buffer (100 mM HEPES pH 7.5, 0.015% Brij 35, 10 mM MgCl₂, 1 mM DTT) with 1 µM CSKtide (5-FAM-AHA-KKKKDDIYFFFG-NH2) and 25 µM ATP at 25°C for 90 minutes in the presence or absence of a dosed concentration series of compound (1% DMSO final concentration). The reaction is stopped by the addition of 70 µl of Stop buffer (100 mM HEPES pH 7.5, 0.015% Brij 35, 35 mM EDTA and 0.2% of Coating Reagent 3, Caliper Lifesciences). The plate is read on a Caliper EZReader 2.

### BIOLOGICAL EXAMPLE 2: UT-7 cell proliferation with SCF stimulation assay as a measure of wild-type KIT activity

UT-7 cells are human megakaryoblastic leukemia cell lines that can be grown in culture with dependence on granulocyte macrophage colony stimulating factor (GM-CSF) or stem cell factor (SCF). UT-7 cells respond to SCF stimulation by activation of the KIT receptor tyrosine kinase and subsequent downstream signaling that can support cell growth and proliferation (Kuriu *et al,* 1999; Komatsu *et al,* 1991; Sasaki *et al,* 1995). Test compounds are assayed for their ability to inhibit SCF-stimulated proliferation of UT-7 cells.

Inhibition of SCF-stimulated UT-7 cell proliferation is assessed using the CellTiter-Glo assay that quantifies the amount of adenosine triphosphate (ATP) present, which is a readout of metabolically active cells and is directly proportional to the number of viable cells in culture. The ability of test compounds to inhibit SCF-stimulated UT-7 cell proliferation is determined using a 10-point dose curve ranging from 25 µM to 95.4 pM of test compound.

UT-7 cells are maintained in IMDM supplemented with 10% FBS, 5 ng/mL GM-CSF and 100 units/mL Penicillin-Streptomycin and grown in a 37°C humidified tissue culture incubator. UT-7 cells are washed once with serum free, GM-CSF free IMDM. Cells are then resuspended in IMDM containing 4% FBS and 50 ng/mL SCF and seeded at 2500 cells per well in a volume of 22 µL in a 384-well microplate. A 10-point dose concentration series of test compounds (25.0 µM to 95.4 pM) are then added to the cells in a volume of 3.1 µL to each well (0.25% DMSO final concentration) and placed in a tissue culture incubator (5% CO2, 37°C) for 72 hours. After 3-days with test compound, CellTiter-Glo reagent is prepared fresh and 25 µL of reagent is added to each well. The plate is mixed by shaking for 10 minutes at rt at 300 rpm on a plate shaker. The plate is read on an EnVision plate reader using the Ultra Sensitive Luminescence protocol for a 384-well plate. Data is normalized to 0% and 100% inhibition controls and the IC50 is calculated using Four Parameter Logistic IC50 curve fitting.

### BIOLOGICAL EXAMPLE 3: Evaluation of Brain Penetration in Rats Brain to Plasma Ratios (Kp,brain)

To understand the brain penetration, brain to plasma ratios of the compounds were obtained in Sprague-Dawley (SD) rats. *In vivo* equilibrium distribution between blood and brain in preclinical species such as rats is a commonly used parameter to evaluate brain penetration. Kₚ,brain is the ratio of concentrations in brain and blood (C_{brain}/Cₚₗₐₛₘₐ). The compound's passive diffusion characteristics, its affinity for membrane transporters at the blood-brain barrier (BBB), and the relative drug binding affinity differences between the plasma proteins and brain tissue influence the Kₚ,brain. Compounds with Kₚ,brain smaller than 0.1 have restricted access to the CNS, whereas compounds with Kₚ,brain greater than 0.3-0.5 are considered to have good brain penetration and compounds with Kₚ,brain greater than 1 freely cross the BBB (Expert Opin. Drug Delivery (2016) 13 (01): 85-92).

The brain penetration of the compounds disclosed herein was measured in Sprague-Dawley rats (3/compound). The animals received IV infusion of 1mg/kg/hr of the compound over 8 hours via jugular vein cannulation. At 24 hours, blood was collected via tail vein bleeding or cardiac puncture (under anesthesia) and centrifuged to obtain plasma samples. Brain tissues were collected and homogenized with phosphate-buffered saline (PBS). The concentrations of the compounds were obtained in the plasma and brain homogenates by LC-MS/MS analysis. Table 3 below shows the results of the Kₚ,brain for compounds **1** and **2** prepared according to the examples described herein and Comparator A.

Compounds **1** and **2** presents a very low Kₚ,brain (Mean=0.133 and 0.045) as compared to Comparator A (Mean=1.8).

Rat plasma protein binding of the tested compounds were evaluated *in vitro* using an equilibrium dialysis method. The tested compounds were assessed in 100% plasma in a dialysis block for 5 hours at 37°C. Samples from the donor and receiver sides were analyzed by LC-MS/MS. Plasma protein bound and unbound fractions were calculated using the following equations - $Fraction bound \left(fb\right) * \left(%\right) = 100 \times \left({\left[Donor\right]}_{5 h}-{\left[Receiver\right]}_{5 h}\right) / {\left[Donor\right]}_{5 h}$ (Equation 1) $Fraction unbound \left(fu\right) , p * \left(%\right) = 100 - % Bound *$ (Equation 2) where: [Donor]₅ₕ is measured donor concentration at 5-hour; [Received]₅ₕ is measured receiver concentration at 5-hour; fb* is bound fraction determined from plasma; fu,p* is calculated unbound fraction for plasma. Warfarin and quinidine were used as positive controls.

Similarly, rat brain protein binding of the tested compounds was also evaluated *in vitro* using equilibrium dialysis method. 1 µM of the compound was assessed in brain homogenate in a dialysis block for 5 hours at 37°C. Samples from the donor and receiver sides were analyzed by LC-MS/MS. Brain protein bound and unbound fractions were calculated using the equations mentions above (Equations 1 and 2).

### Unbound brain to plasma Ratios (Kpuu,brain)

Based on the brain and plasma concentrations obtained above and fu,brain values obtained above, unbound brain to plasma ratios (Kₚᵤᵤ, brain) were calculated to be as shown in Table 3.

Compounds **1** and **2** present a highly superior low K_{p,uu},brain (Mean=0.017 and 0.01) as compared to Comparator A (Mean=0.84). Unbound drug concentration in a tissue is the free drug available to exert its pharmacological effect in the tissue compartment. Since 1 and **2** have very low K_{p,uu},brain as compared to Comparator A, it means that the amount of 1 or **2** available in the brain to exert its pharmacological effect is very low as compared to Comparator A.

**Table 3.**

| Example | Kp, brain | Kpᵤᵤ |
|---|---|---|
| 1 | 0.133 | 0.017 |
| 2 | 0.045 | 0.01 |
| Comparator A | 1.75 | 0.84 |

## Claims

1. A compound of Formula (I): 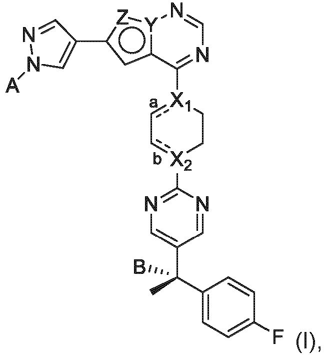
a pharmaceutically acceptable salt thereof, and/or a solvate of any of the foregoing,
wherein:
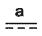 is chosen from a single bond and double bond;
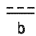 is chosen from a single bond and double bond;
Z is chosen from CH and NH;
Y is chosen from C and N;
X₁ is chosen from CH, C, and N;
X₂ is chosen from CH, C, and N;
provided that when X₁ and X₂ are both N, then Y is not N and Z is not CH;
A is 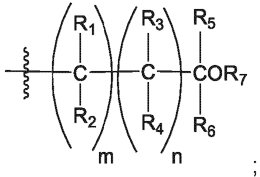
R₁ is chosen from hydrogen and methyl;
R₂ is chosen from hydrogen and methyl, or
R₁ and R₂ taken together form a cyclopropyl;
R₃ is chosen from hydrogen and methyl;
R₄ is chosen from hydrogen and methyl, or
R₃ and R₄ taken together form a cyclopropyl;
R₅ is chosen from hydrogen and methyl;
R₆ is chosen from hydrogen and methyl, or
R₅ and R₆ taken together form a cyclopropyl, or
one of R₂ or R₄ taken together with R₆ forms a cyclobutyl;
R₇ is hydrogen, or one of R₂, R₄, or R₆ taken together with R₇ forms a ring chosen from tetrahydrofuran and tetrahydropyran, wherein said tetrahydrofuran or tetrahydropyran is optionally substituted with hydroxyl;
m is 0 or 1;
n is 0 or 1; and
B is selected from OH and NH₂.

2. The compound of claim 1, a pharmaceutically acceptable salt thereof, and/or a solvate of any of the foregoing, wherein the compound is of Formula (II):

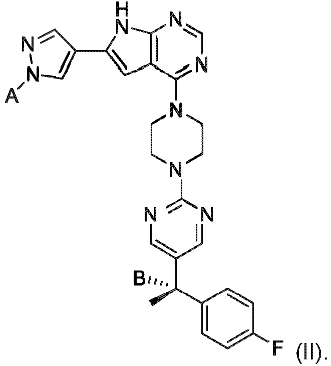

3. The compound of claim 1, a pharmaceutically acceptable salt thereof, and/or a solvate of any of the foregoing, wherein the compound is of Formula (III):

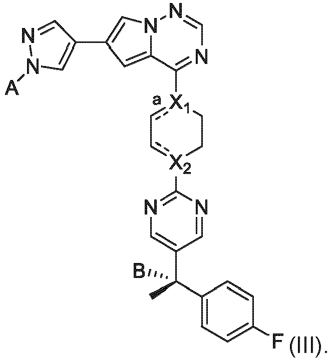

4. The compound of claim 3, a pharmaceutically acceptable salt thereof, and/or a solvate of any of the foregoing, wherein X₁ is N and 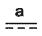 is a single bond and X₂ is C and 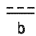 is a double-bond.

5. The compound of claim 3, a pharmaceutically acceptable salt thereof, and/or a solvate of any of the foregoing, wherein X₁ is C and 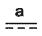 is a double bond and X₂ is N and 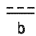 is a single bond.

6. The compound of any one of claims 1-5, a pharmaceutically acceptable salt thereof, and/or a solvate of any of the foregoing, wherein:
A is: 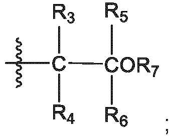
R₃ is chosen from hydrogen and methyl;
R₄ is chosen from hydrogen and methyl, or R₃ and R₄ taken together form a cyclopropyl;
R₅ is chosen from hydrogen and methyl;
R₆ is chosen from hydrogen and methyl; or
R₅ and R₆ taken together form a cyclopropyl, and
R₇ is hydrogen.

7. The compound of any one of claims 1-6, a pharmaceutically acceptable salt thereof, and/or a solvate of any of the foregoing, wherein B is NH₂.

8. The compound of any one of claims 1-6, a pharmaceutically acceptable salt thereof, and/or a solvate of any of the foregoing, wherein B is OH.

9. The compound of any one of claims 1-5, 7, and 8, a pharmaceutically acceptable salt thereof, and/or a solvate of any of the foregoing,
(i) wherein A is chosen from 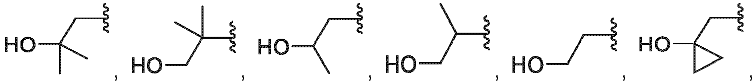 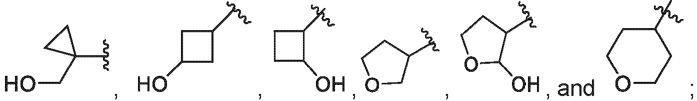 or
(ii) wherein A is chosen from 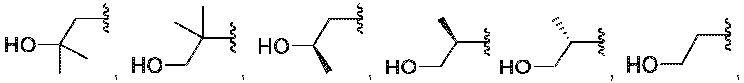 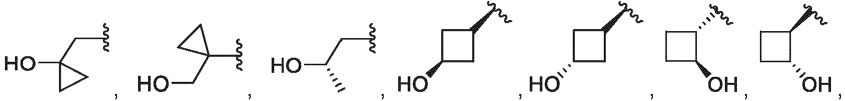 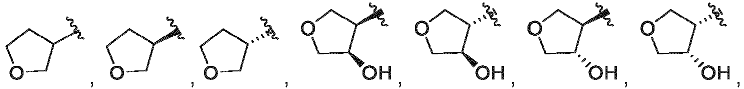 and 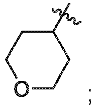 or
(iii) wherein A is chosen from 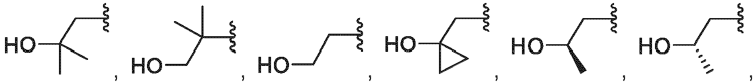 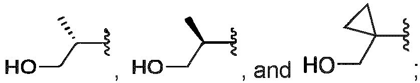 or
(iv) wherein A is chosen from 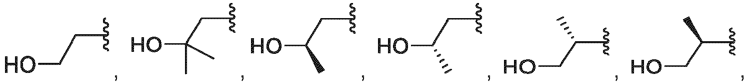 and

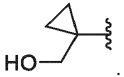

10. The compound of any one of claims 1-5, 7, and 8, a pharmaceutically acceptable salt thereof, and/or a solvate of any of the foregoing, wherein A is chosen from

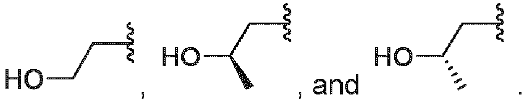

11. The compound of any one of claims 1-5, 7, and 8, a pharmaceutically acceptable salt thereof, and/or a solvate of any of the foregoing, wherein A is

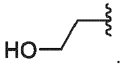

12. The compound of claim 1, a pharmaceutically acceptable salt thereof, and/or a solvate of any of the foregoing, wherein the compound is selected from:
| **No.** | **Chemical Structure** | **No.** | **Chemical Structure** |
|---|---|---|---|
| 1 | 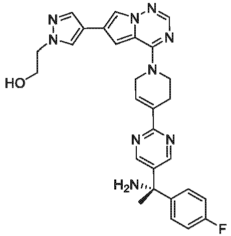 | 2 | 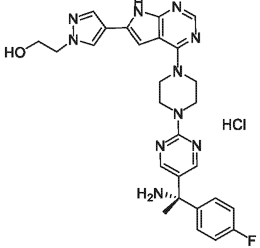 |
| | | 4 | 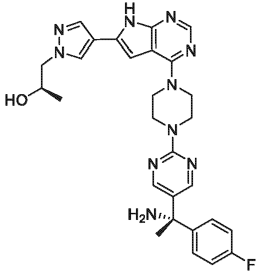 |
| 5 | 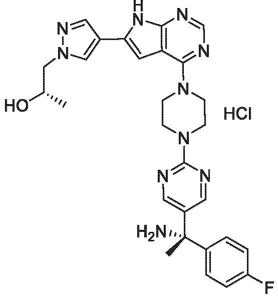 | | |
| 7 | 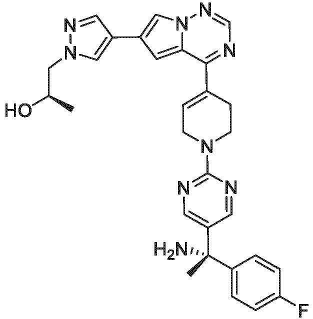 | 8 | 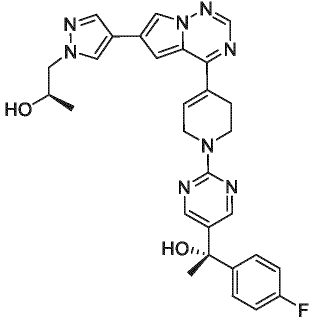 |
| 9 | 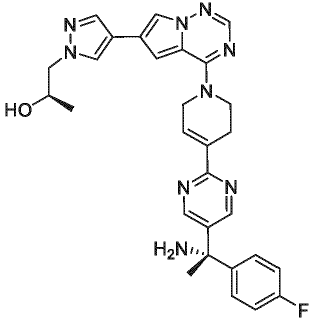 | 10 | 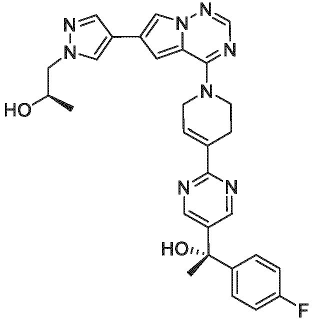 |
| 11 | 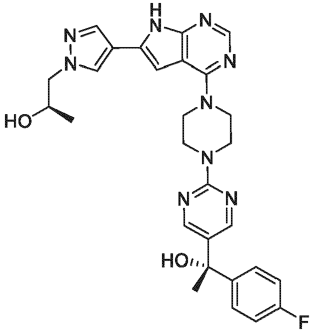 | 12 | 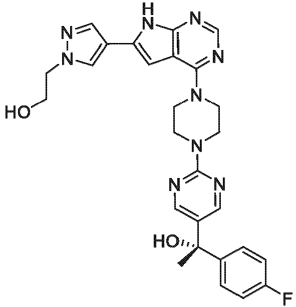 |
| 13 | 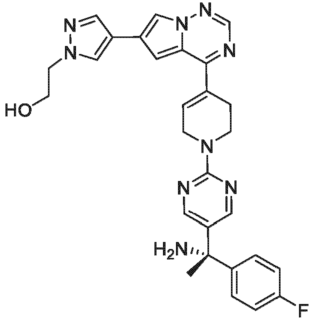 | 14 | 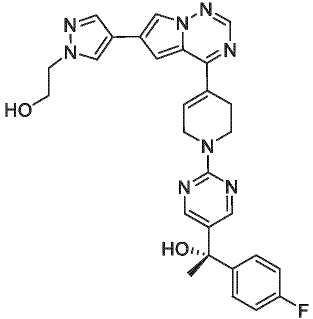 |
| 14-A | 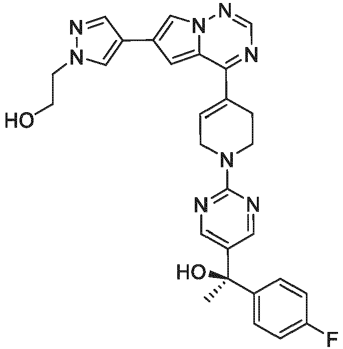 | 15 | 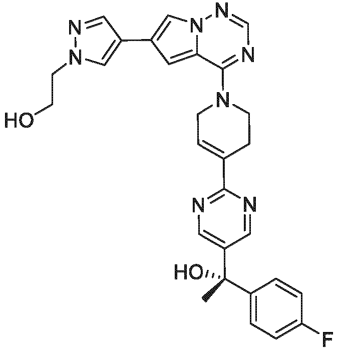 |
| 16 | 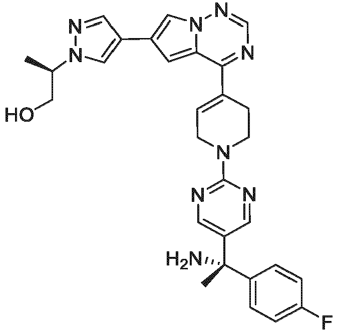 | 17 | 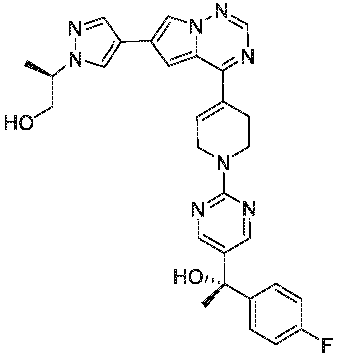 |
| 18 | 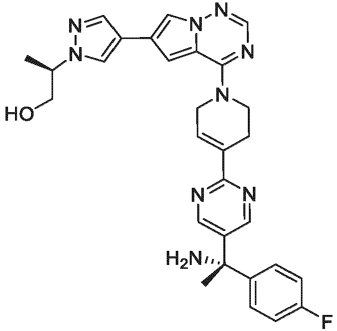 | 19 | 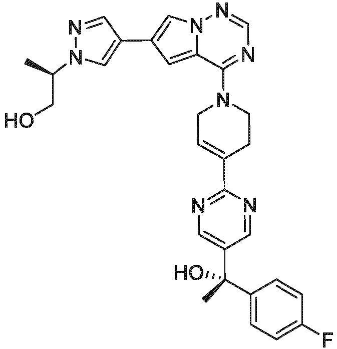 |
| 20 | 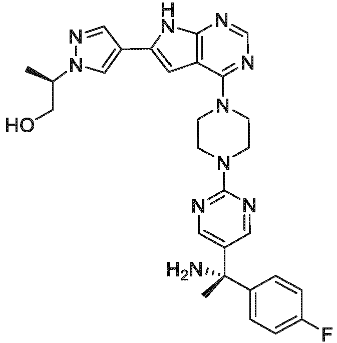 | 21 | 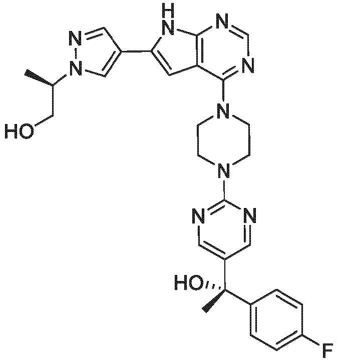 |
| 22 | 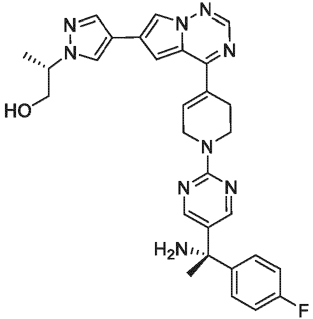 | 23 | 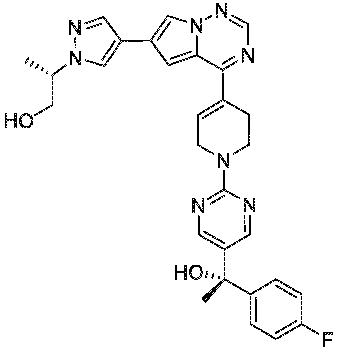 |
| 24 | 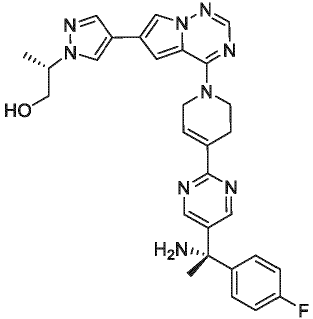 | 25 | 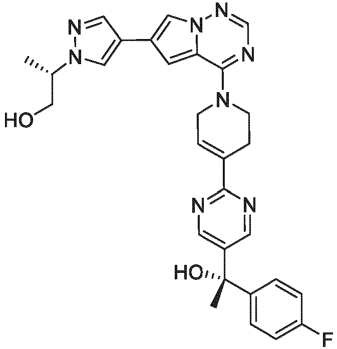 |
| 26 | 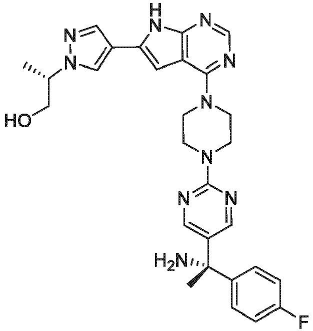 | 27 | 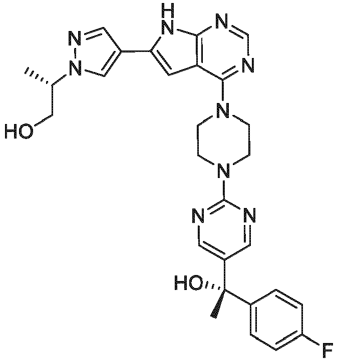 |
| 38 | 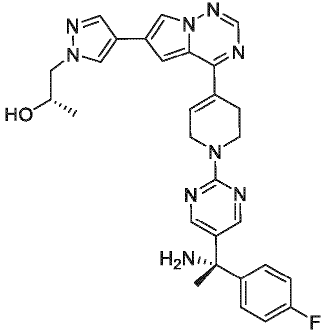 | 39 | 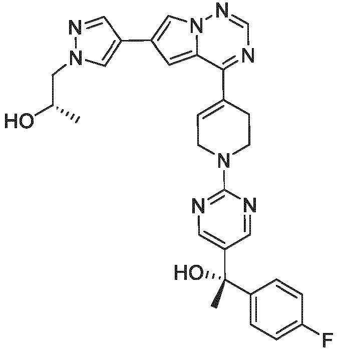 |
| 40 | 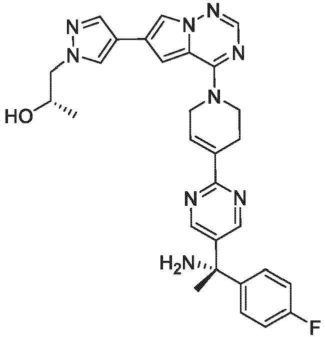 | 41 | 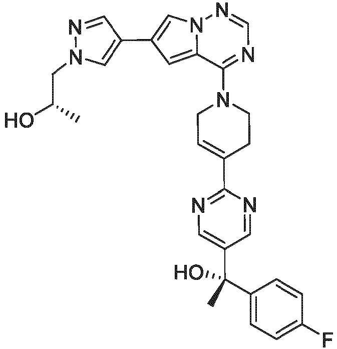 |
| 42 | 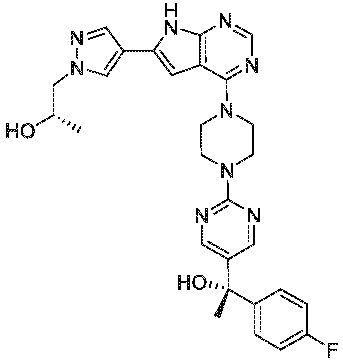 | 44 | 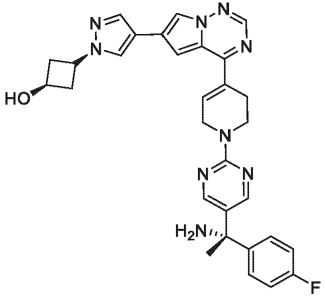 |
| 45 | 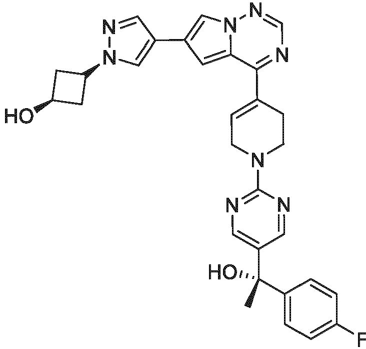 | 46 | 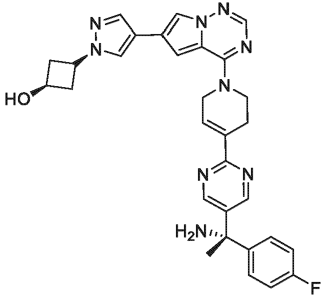 |
| 47 | 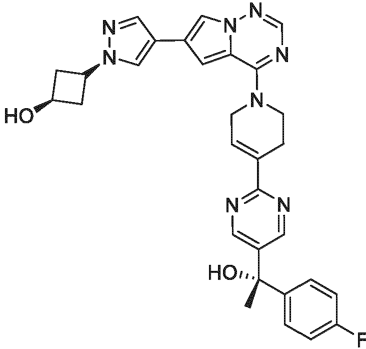 | 48 | 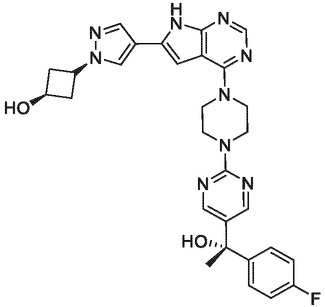 |
| 49 | 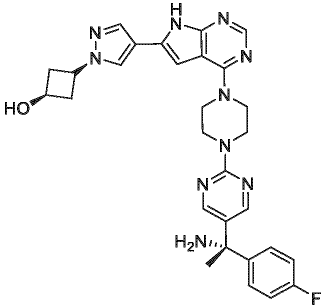 | 50 | 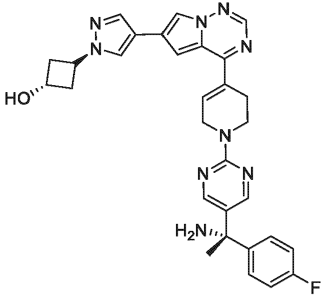 |
| 51 | 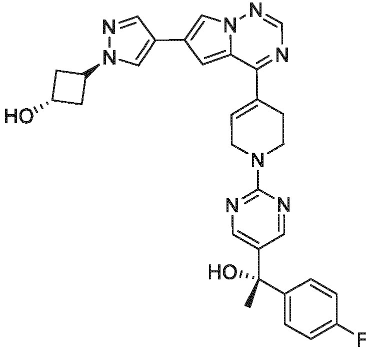 | 52 | 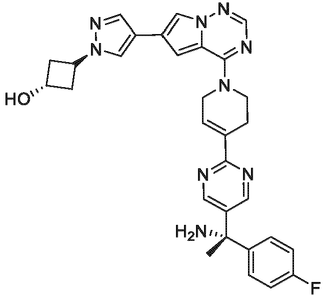 |
| 53 | 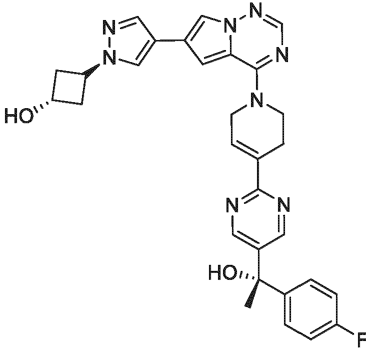 | 54 | 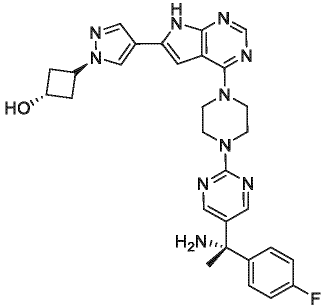 |
| 55 | 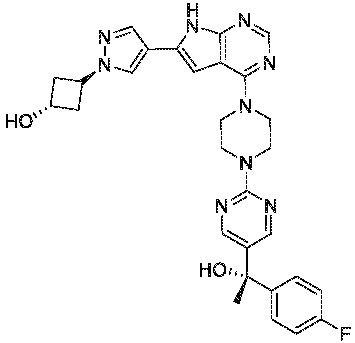 | 56 | 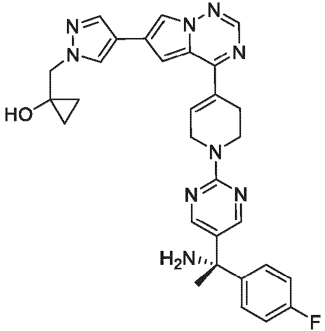 |
| 57 | 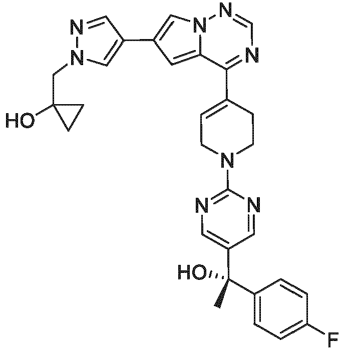 | 58 | 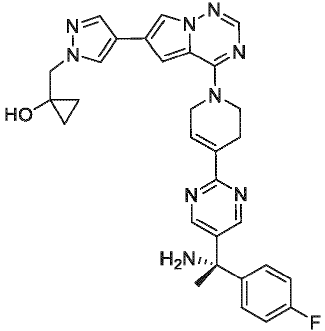 |
| 60 | 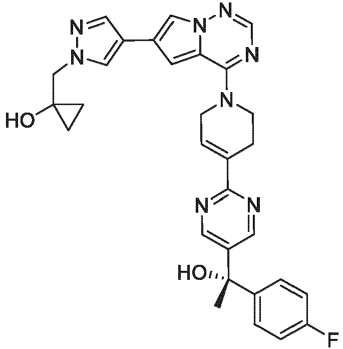 | 61 | 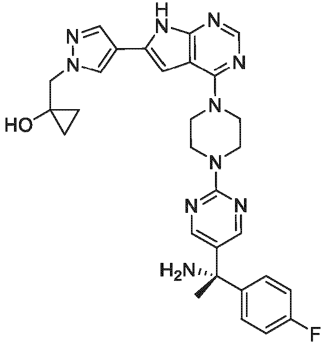 |
| 62 | 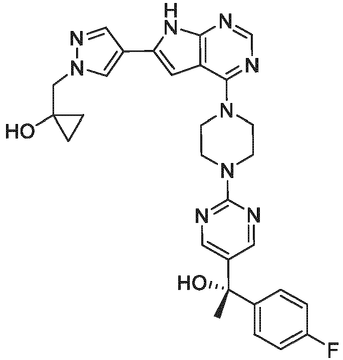 | 63 | 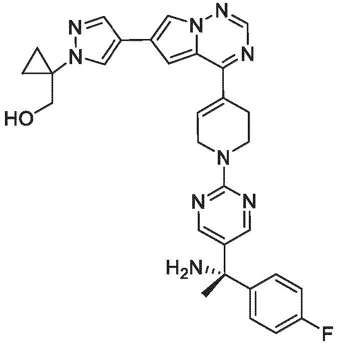 |
| 64 | 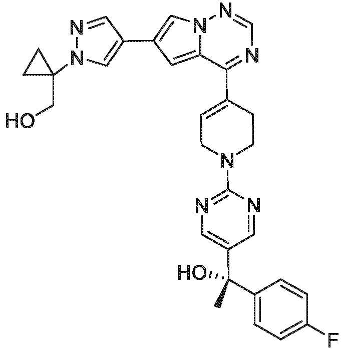 | 65 | 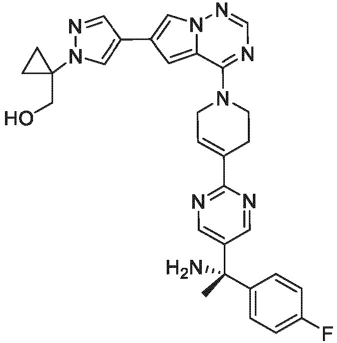 |
| 66 | 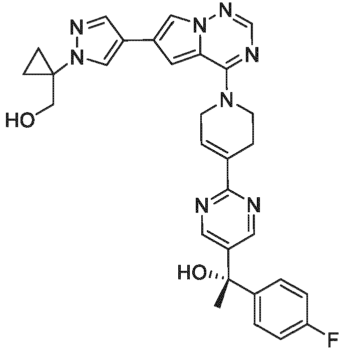 | 67 | 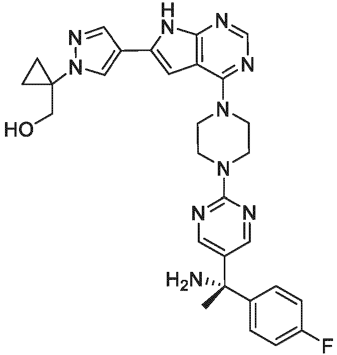 |
| 68 | 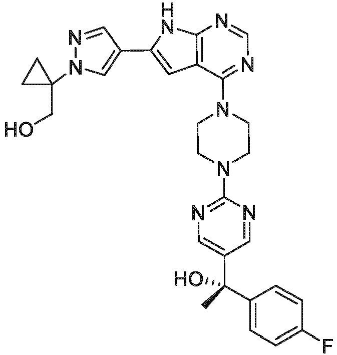 | 69 | 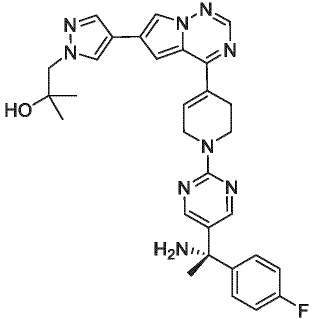 |
| 70 | 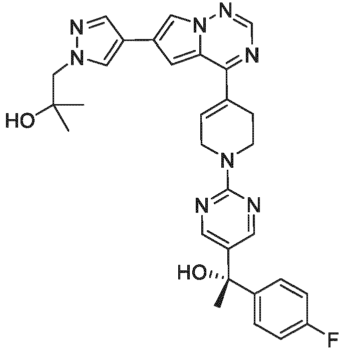 | 71 | 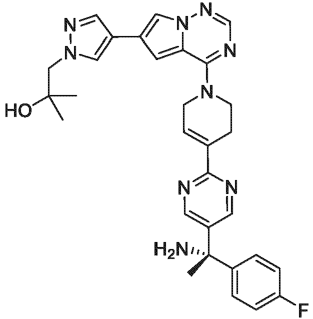 |
| 72 | 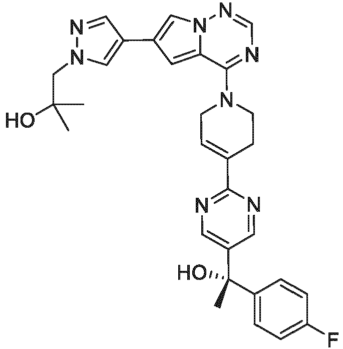 | 73 | 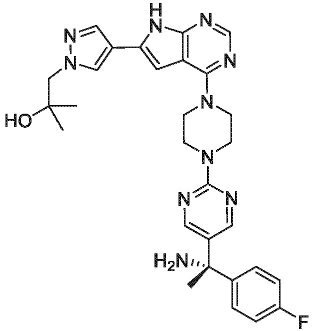 |
| 74 | 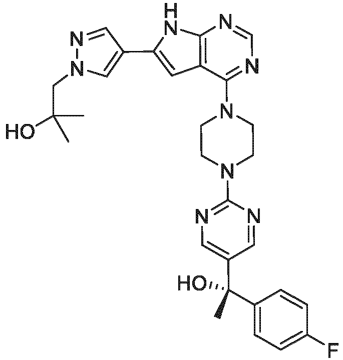 | 75 | 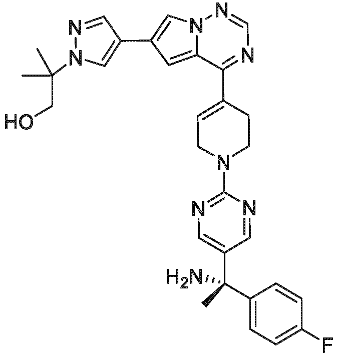 |
| 76 | 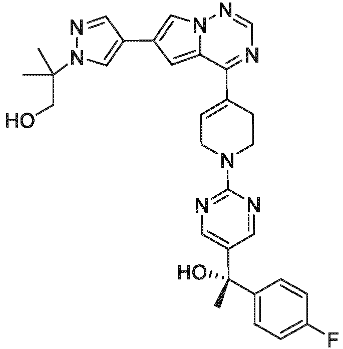 | 77 | 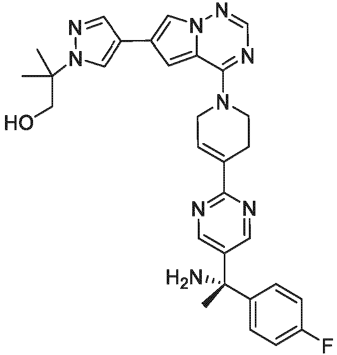 |
| 78 | 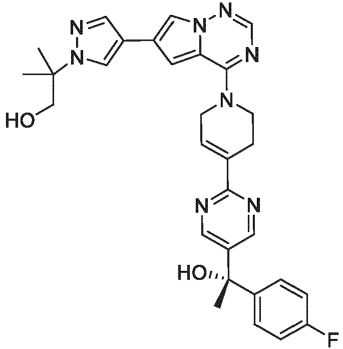 | 79 | 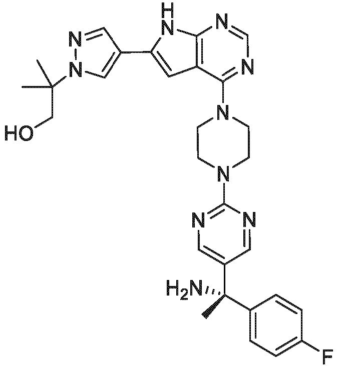 |
| 80 | 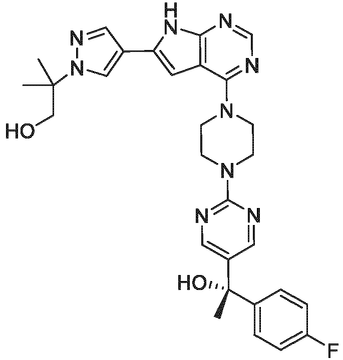 | 81 | 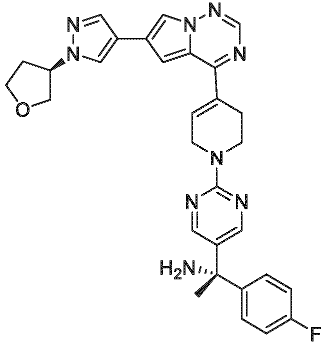 |
| 82 | 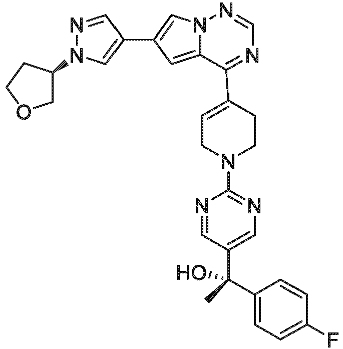 | 83 | 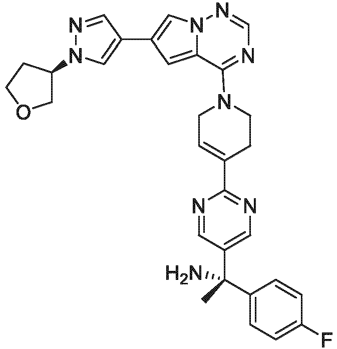 |
| 84 | 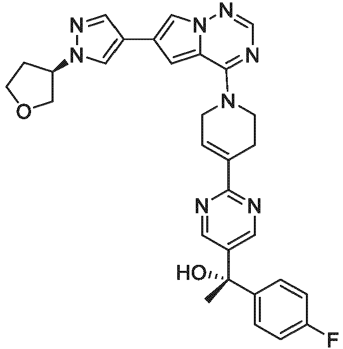 | 85 | 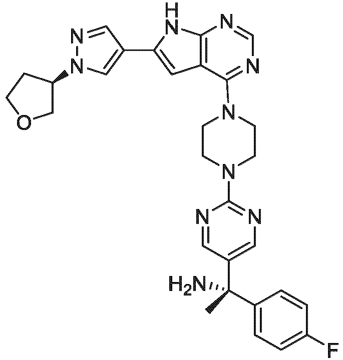 |
| 86 | 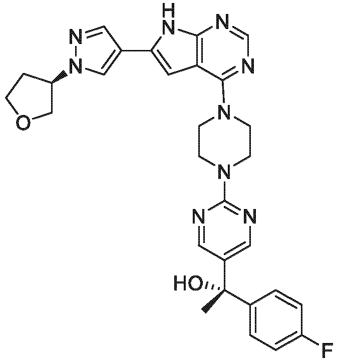 | 87 | 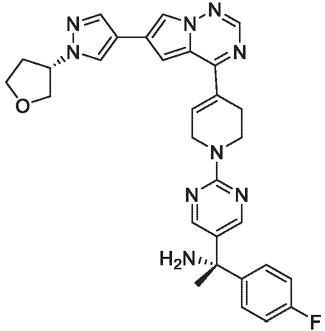 |
| 88 | 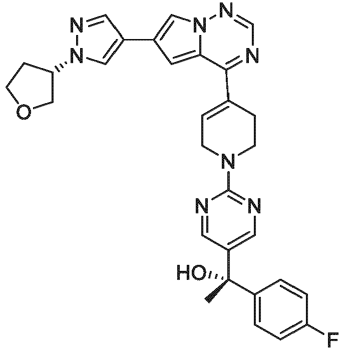 | 89 | 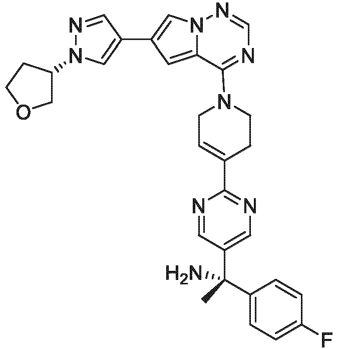 |
| 90 | 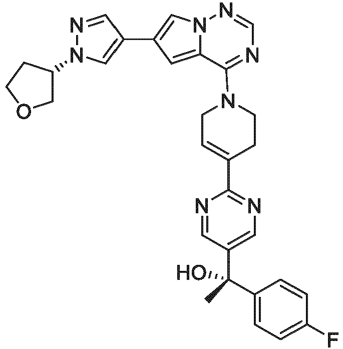 | 91 | 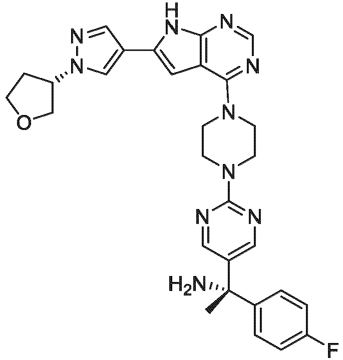 |
| 92 | 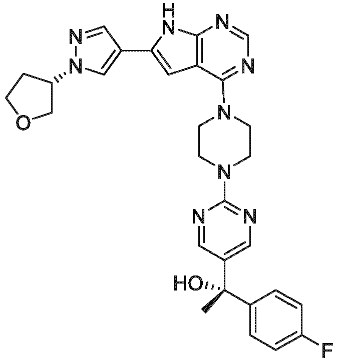 | 93 | 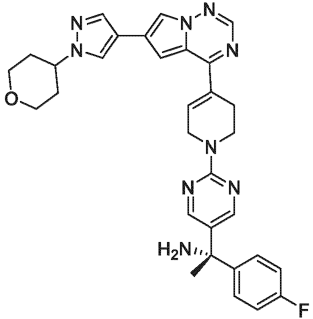 |
| 94 | 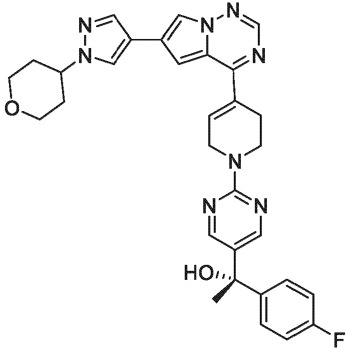 | 95 | 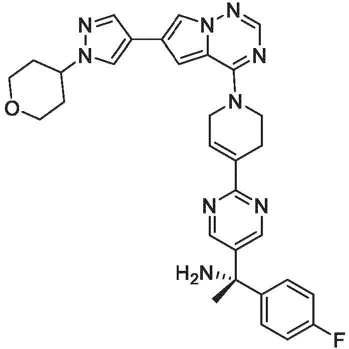 |
| 96 | 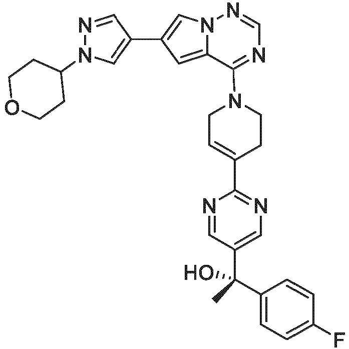 | 97 | 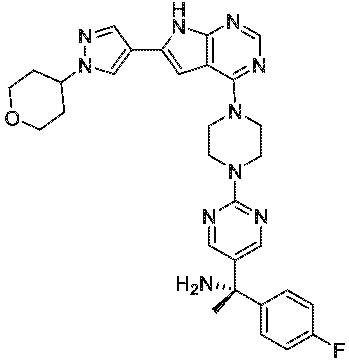 |
| 98 | 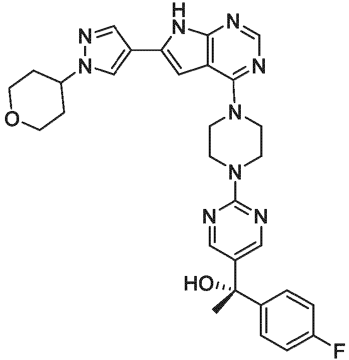 | 99 | 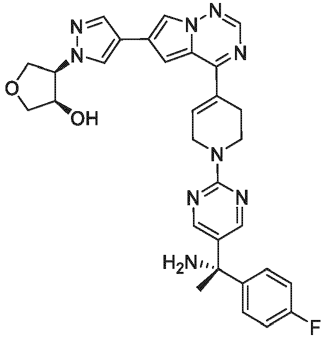 |
| 100 | 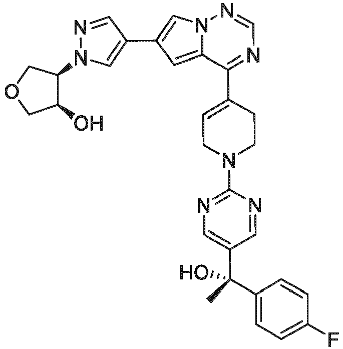 | 101 | 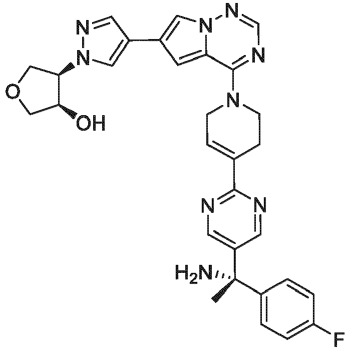 |
| 102 | 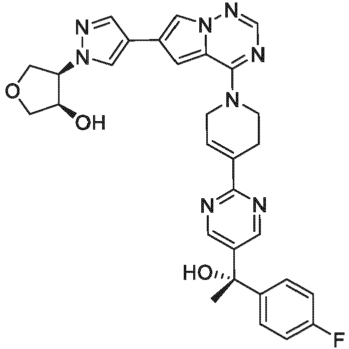 | 103 | 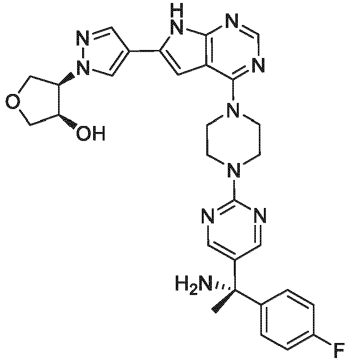 |
| 104 | 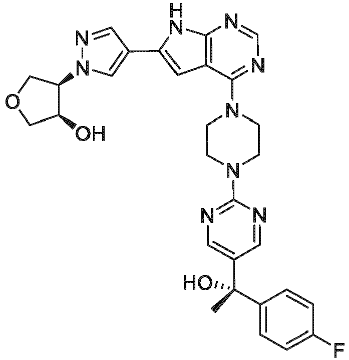 | 105 | 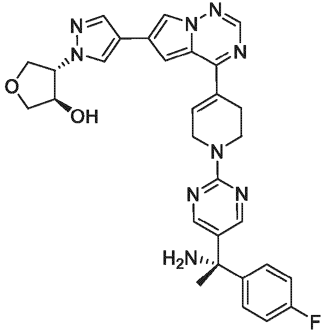 |
| 106 | 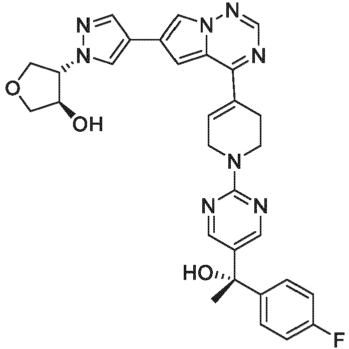 | 107 | 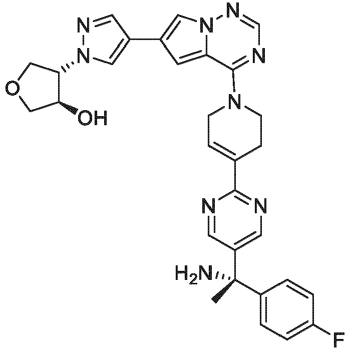 |
| 108 | 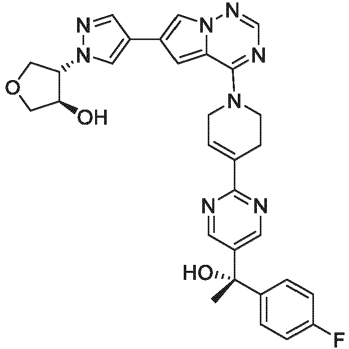 | 109 | 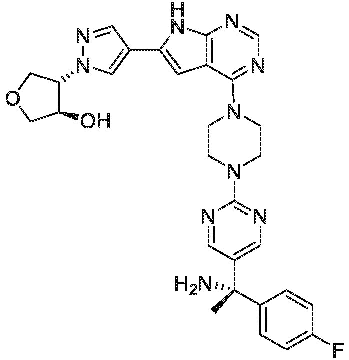 |
| 110 | 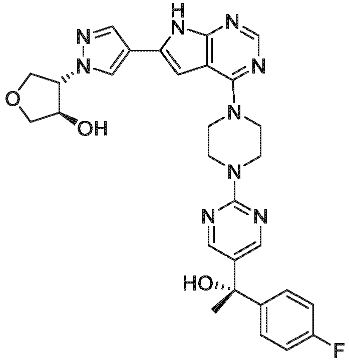 | 111 | 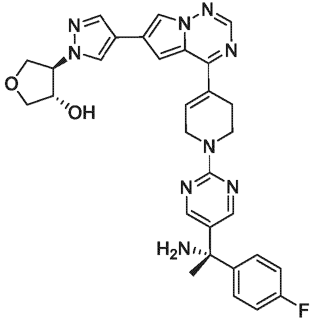 |
| 112 | 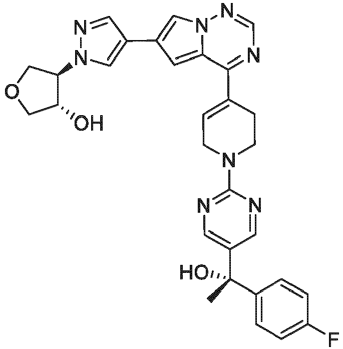 | 113 | 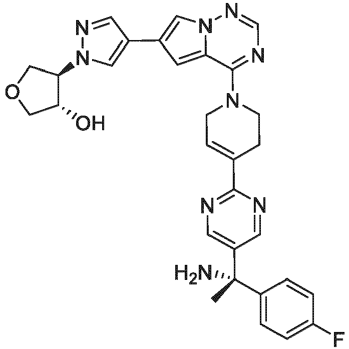 |
| 114 | 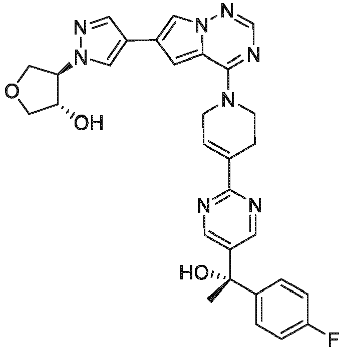 | 115 | 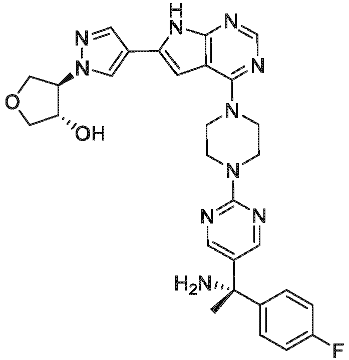 |
| 116 | 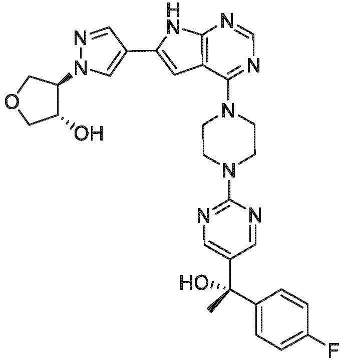 | 117 | 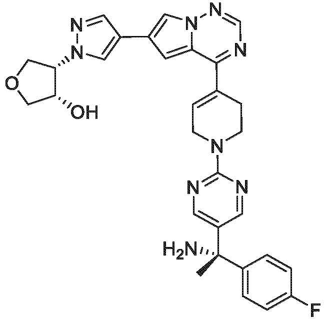 |
| 118 | 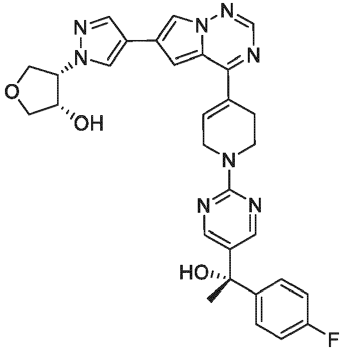 | 119 | 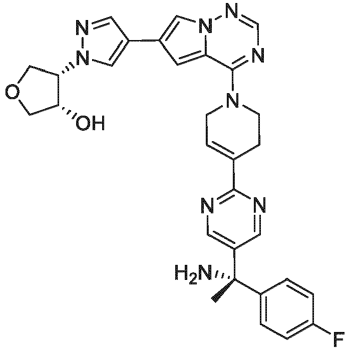 |
| 120 | 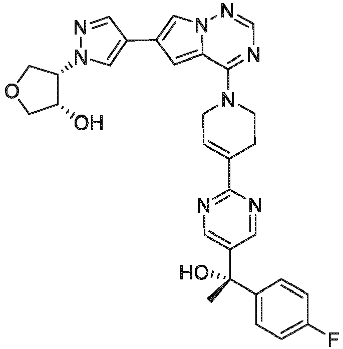 | 121 | 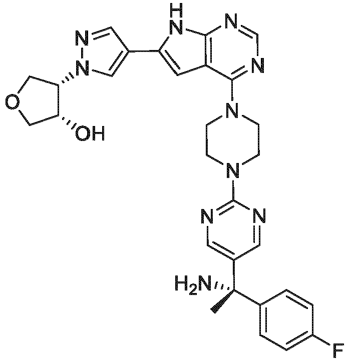 |
| 122 | 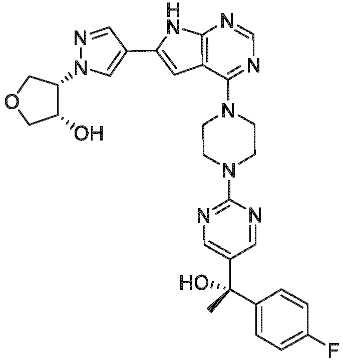 | 123 | 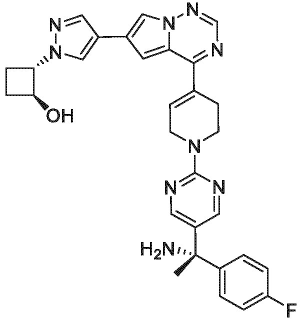 |
| 124 | 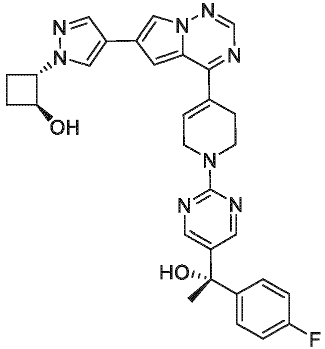 | 125 | 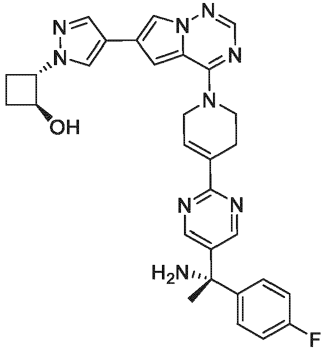 |
| 126 | 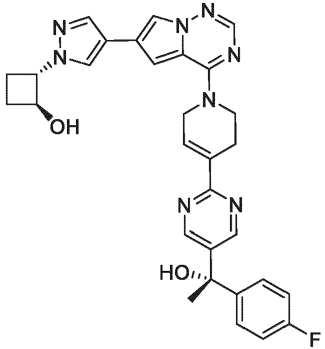 | 127 | 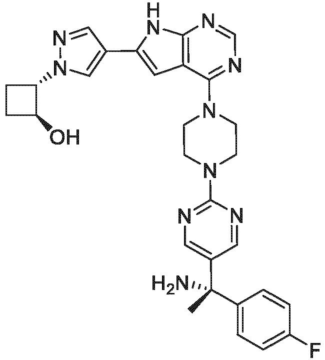 |
| 128 | 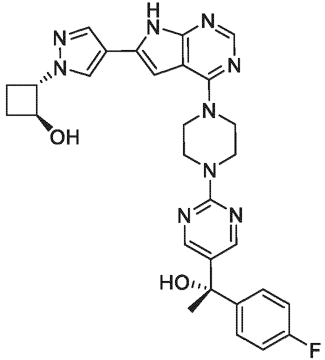 | 129 | 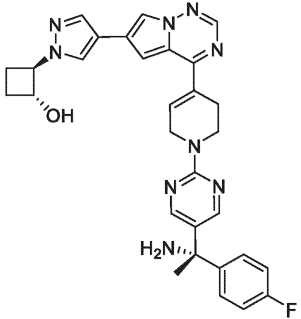 |
| 130 | 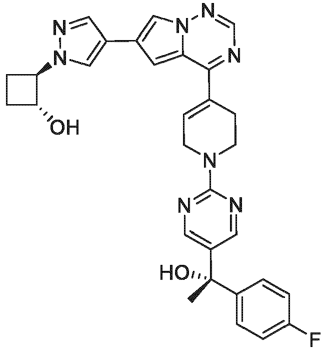 | 131 | 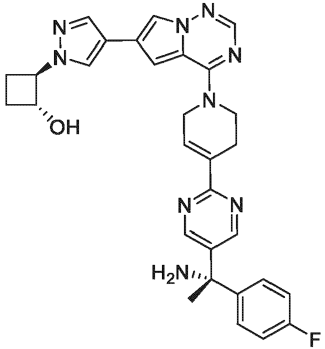 |
| 132 | 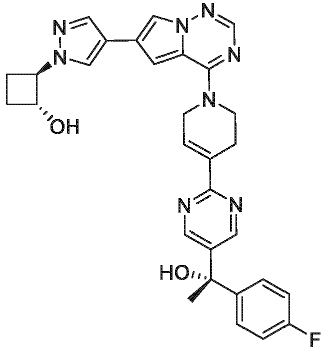 | 133 | 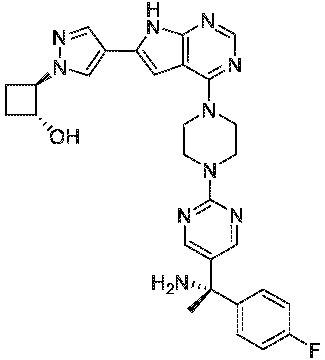 |
| 134 | 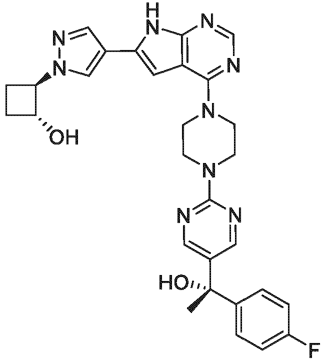 | | |

13. A pharmaceutical composition comprising:
a compound of any one of claims 1-12, a pharmaceutically acceptable salt thereof, and/or a solvate of any of the foregoing; and
a pharmaceutically acceptable excipient.

14. A compound according to any one of claims 1-12 a pharmaceutically acceptable salt thereof, and/or a solvate of any of the foregoing for use as a medicament in the treatment of a disease or condition in a patient in need thereof, wherein the disease or condition is chosen from systemic mastocytosis, gastrointestinal stromal tumors, acute myeloid leukemia, melanoma, seminoma, intracranial germ cell tumors, mediastinal B-cell lymphoma, Ewing's sarcoma, diffuse large B cell lymphoma, dysgerminoma, myelodysplastic syndrome, nasal NK/T-cell lymphoma, chronic myelomonocytic leukemia, and brain cancer; preferably wherein the disease or condition is systemic mastocytosis; preferably wherein the systemic mastocytosis is chosen from indolent systemic mastocytosis and smoldering systemic mastocytosis.

## Patentansprüche

1. Eine Verbindung der Formel (I): 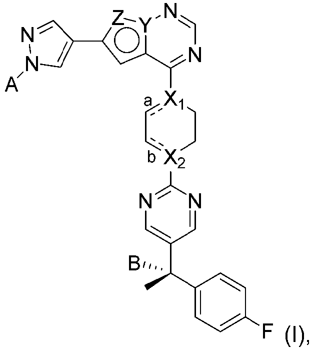
ein pharmazeutisch akzeptables Salz davon und/oder ein Solvat eines der Vorgenannten, wobei:
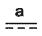 aus einer Einfachbindung und einer Doppelbindung ausgewählt ist;
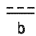 aus einer Einfachbindung und einer Doppelbindung ausgewählt ist;
Z aus CH und NH ausgewählt ist;
Y aus C und N ausgewählt ist;
X₁ aus CH, C und N ausgewählt ist;
X₂ aus CH, C und N ausgewählt ist;
mit der Maßgabe, dass, wenn X₁ und X₂ beide N sind, dann Y nicht N ist und Z nicht CH ist;
A 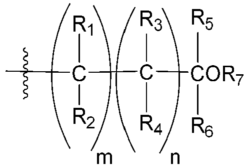 ist;
R₁ aus Wasserstoff und Methyl ausgewählt ist;
R₂ aus Wasserstoff und Methyl ausgewählt ist oder
R₁ und R₂ zusammengenommen ein Cyclopropyl bilden;
R₃ aus Wasserstoff und Methyl ausgewählt ist;
R₄ aus Wasserstoff und Methyl ausgewählt ist oder
R₃ und R₄ zusammengenommen ein Cyclopropyl bilden;
R₅ aus Wasserstoff und Methyl ausgewählt ist;
R₆ aus Wasserstoff und Methyl ausgewählt ist oder
R₅ und R₆ zusammengenommen ein Cyclopropyl bilden oder
eines von R₂ oder R₄ zusammengenommen mit R₆ ein Cyclobutyl bildet;
R₇ Wasserstoff ist oder eines von R₂, R₄ oder R₆ zusammengenommen mit R₇ einen Ring, ausgewählt aus Tetrahydrofuran und Tetrahydropyran, bildet, wobei das genannte Tetrahydrofuran oder Tetrahydropyran optional mit Hydroxyl substituiert ist;
m 0 oder 1 ist;
n 0 oder 1 ist; und
B aus OH und NH₂ ausgewählt ist.

2. Verbindung nach Anspruch 1, ein pharmazeutisch akzeptables Salz davon und/oder ein Solvat eines der Vorgenannten, wobei die Verbindung eine der Formel (II) ist:

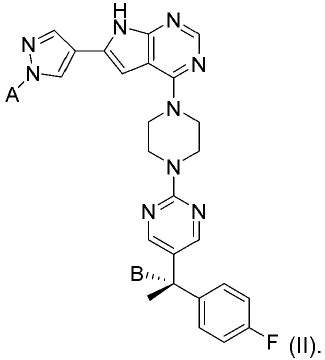

3. Verbindung nach Anspruch 1, ein pharmazeutisch akzeptables Salz davon und/oder ein Solvat eines der Vorgenannten, wobei die Verbindung eine der Formel (III) ist:

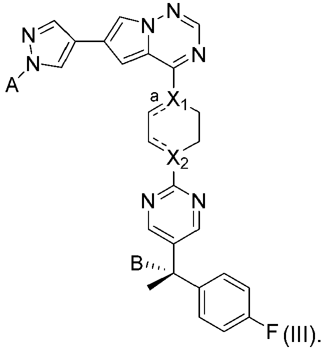

4. Verbindung nach Anspruch 3, ein pharmazeutisch akzeptables Salz davon und/oder ein Solvat eines der Vorgenannten, wobei X₁ N ist und 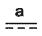 eine Einfachbindung ist und X₂ C ist und 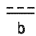 eine Doppelbindung ist.

5. Verbindung nach Anspruch 3, ein pharmazeutisch akzeptables Salz davon und/oder ein Solvat eines der Vorgenannten, wobei X₁ C ist und 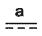 eine Doppelbindung ist und X₂ N ist und 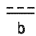 eine Einfachbindung ist.

6. Verbindung nach einem der Ansprüche 1-5, ein pharmazeutisch akzeptables Salz davon und/oder ein Solvat eines der Vorgenannten, wobei:
A: 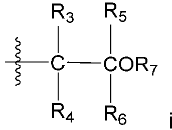 ist;
R₃ aus Wasserstoff und Methyl ausgewählt ist;
R₄ aus Wasserstoff und Methyl ausgewählt ist oder R₃ und R₄ zusammengenommen ein Cyclopropyl bilden;
R₅ aus Wasserstoff und Methyl ausgewählt ist;
R₆ aus Wasserstoff und Methyl ausgewählt ist; oder
R₅ und R₆ zusammengenommen ein Cyclopropyl bilden und
R₇ Wasserstoff ist.

7. Verbindung nach einem der Ansprüche 1-6, ein pharmazeutisch akzeptables Salz davon und/oder ein Solvat eines der Vorgenannten, wobei B NH₂ ist.

8. Verbindung nach einem der Ansprüche 1-6, ein pharmazeutisch akzeptables Salz davon und/oder ein Solvat eines der Vorgenannten, wobei B OH ist.

9. Verbindung nach einem der Ansprüche 1-5, 7 und 8, ein pharmazeutisch akzeptables Salz davon und/oder ein Solvat eines der Vorgenannten,
(i) wobei A aus 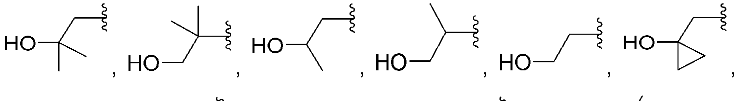 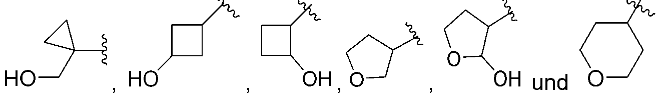 ausgewählt ist; oder
(ii) wobei A aus 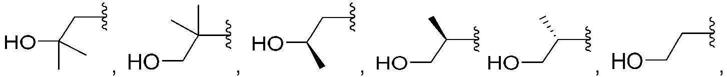 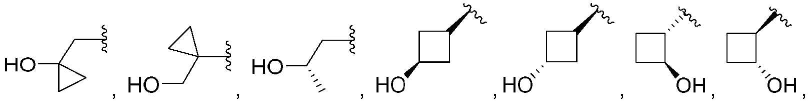 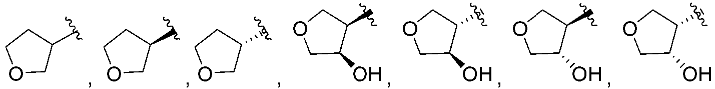 und 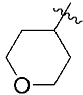 ausgewählt ist; oder
(iii) wobei A aus 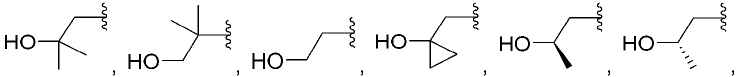 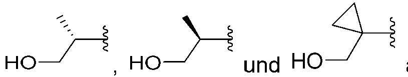 ausgewählt ist; oder
(iv) wobei A aus 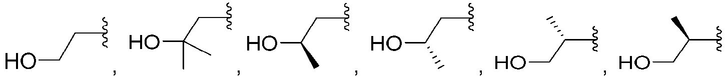 und 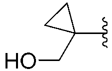 ausgewählt ist.

10. Verbindung nach einem der Ansprüche 1-5, 7 und 8, ein pharmazeutisch akzeptables Salz davon und/oder ein Solvat eines der Vorgenannten, wobei A aus 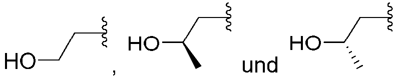 ausgewählt ist.

11. Verbindung nach einem der Ansprüche 1-5, 7 und 8, ein pharmazeutisch akzeptables Salz davon und/oder ein Solvat eines der Vorgenannten, wobei A 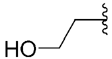 ist.

12. Verbindung nach Anspruch 1, ein pharmazeutisch akzeptables Salz davon und/oder ein Solvat eines der Vorgenannten, wobei die Verbindung aus den Folgenden ausgewählt ist:
| **Nr.** | **Chemische Struktur** | **Nr.** | **Chemische Struktur** |
|---|---|---|---|
| 1 | 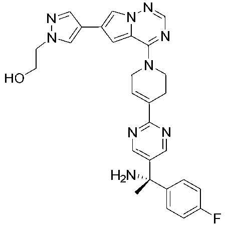 | 2 | 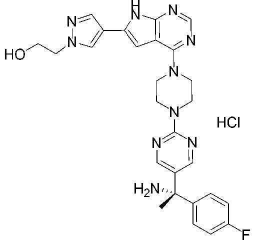 |
| | | 4 | 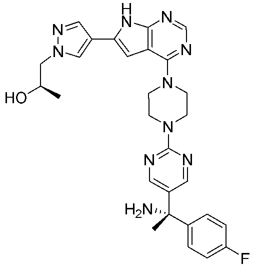 |
| 5 | 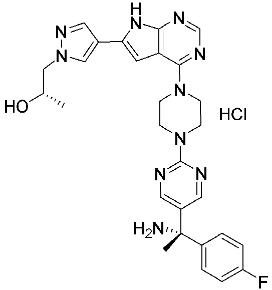 | | |
| 7 | 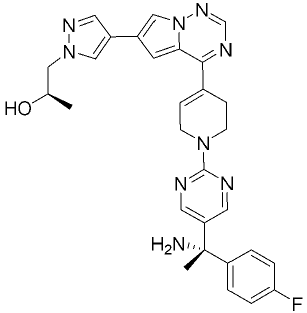 | 8 | 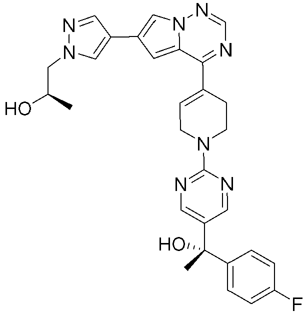 |
| 9 | 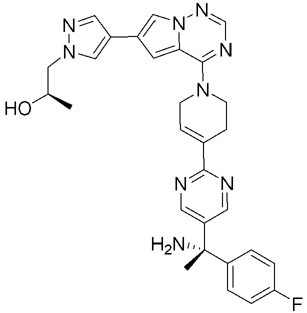 | 10 | 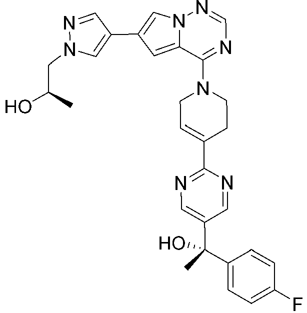 |
| 11 | 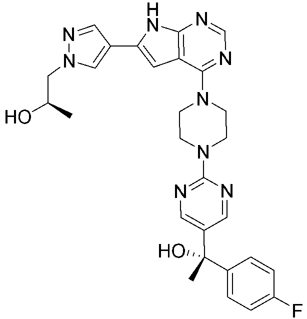 | 12 | 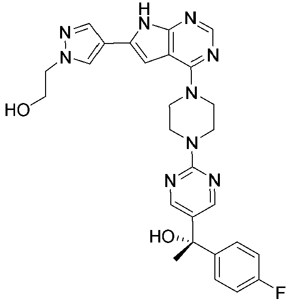 |
| 13 | 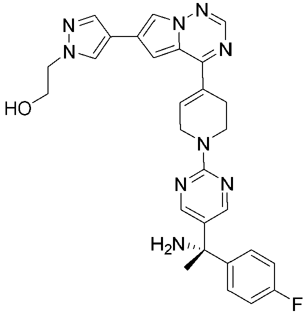 | 14 | 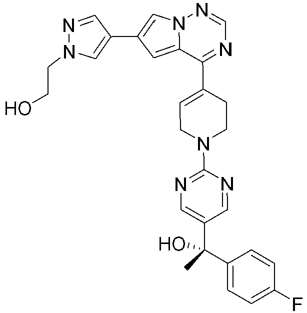 |
| 14-A | 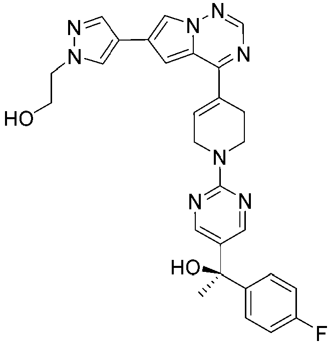 | 15 | 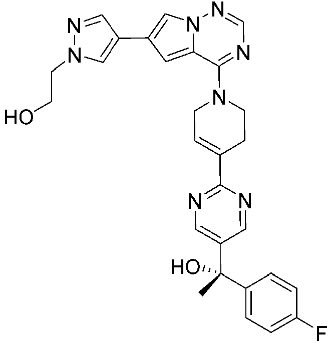 |
| 16 | 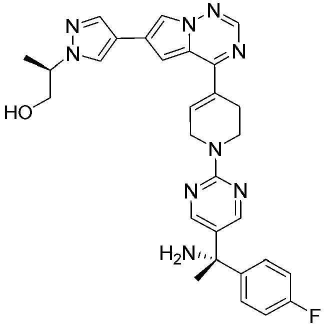 | 17 | 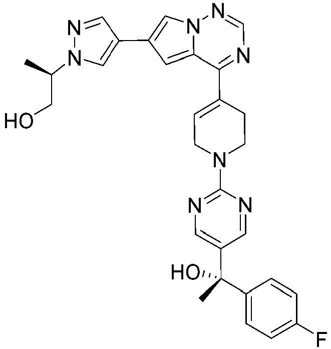 |
| 18 | 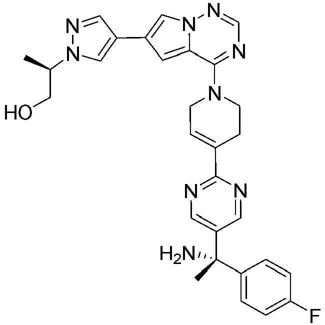 | 19 | 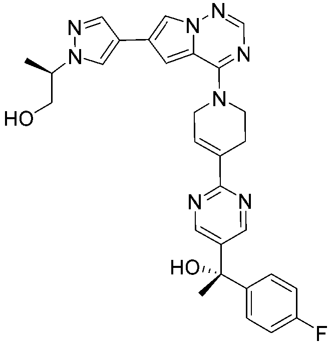 |
| 20 | 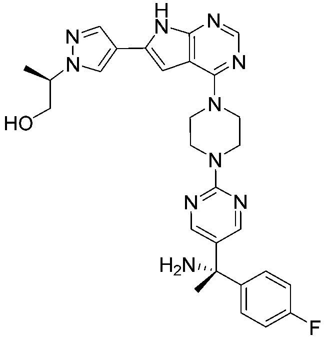 | 21 | 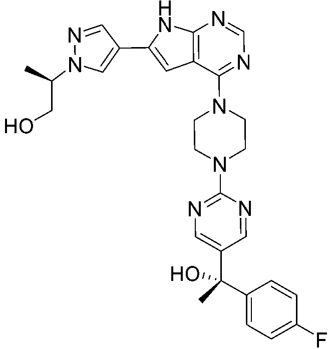 |
| 22 | 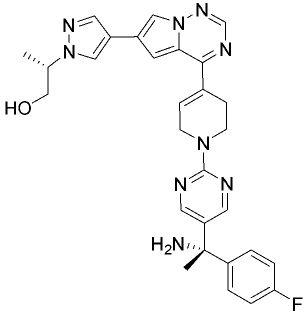 | 23 | 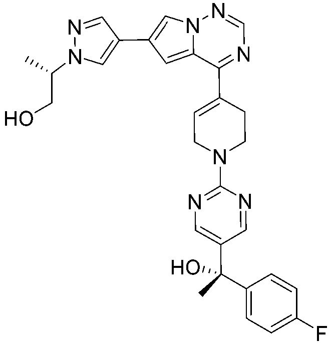 |
| 24 | 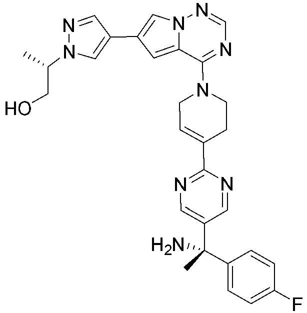 | 25 | 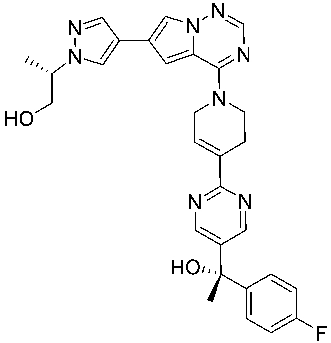 |
| 26 | 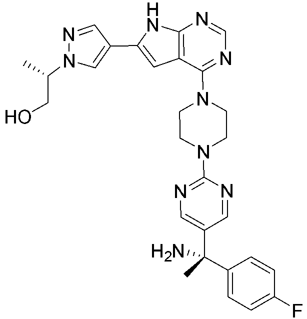 | 27 | 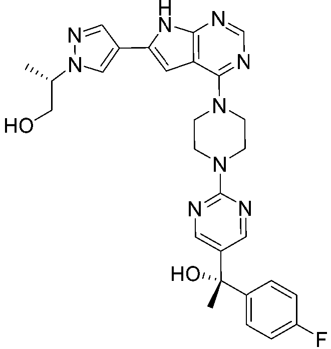 |
| 38 | 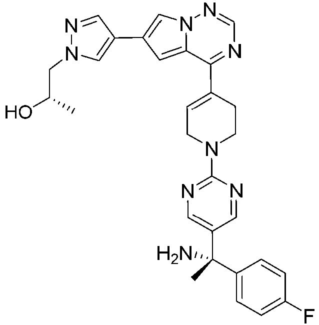 | 39 | 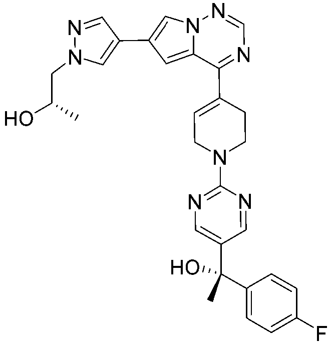 |
| 40 | 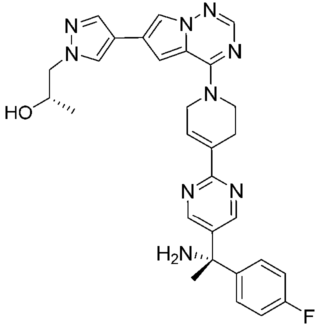 | 41 | 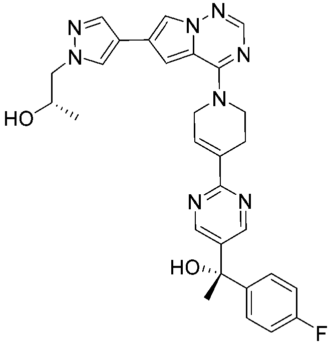 |
| 42 | 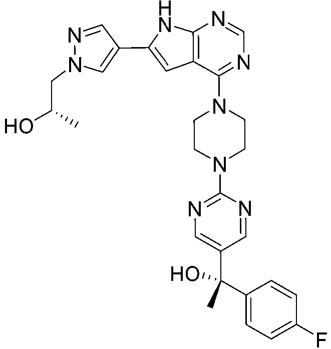 | 44 | 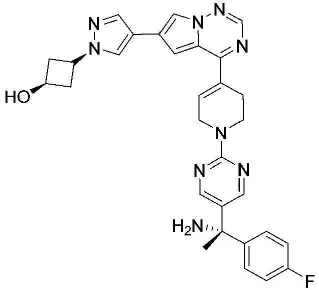 |
| 45 | 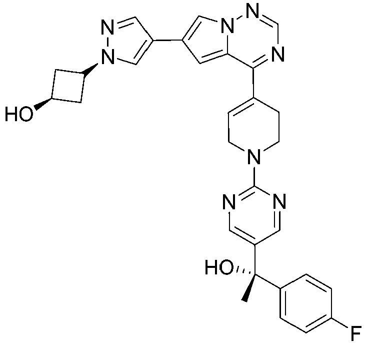 | 46 | 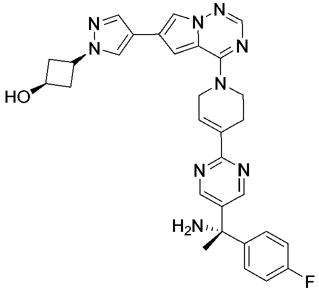 |
| 47 | 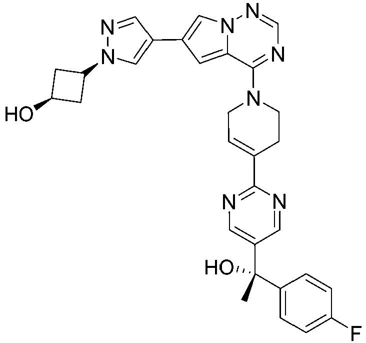 | 48 | 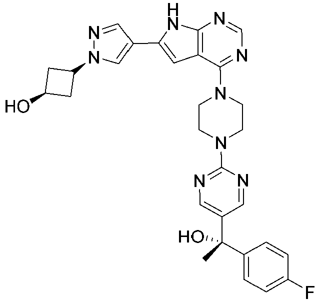 |
| 49 | 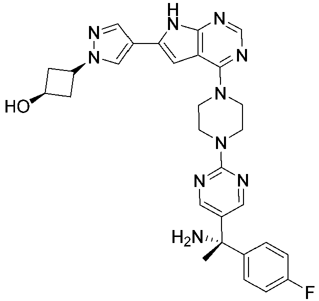 | 50 | 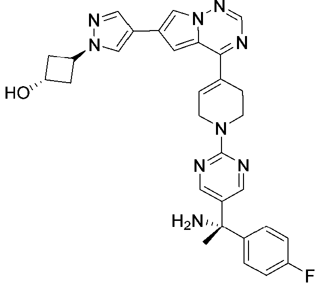 |
| 51 | 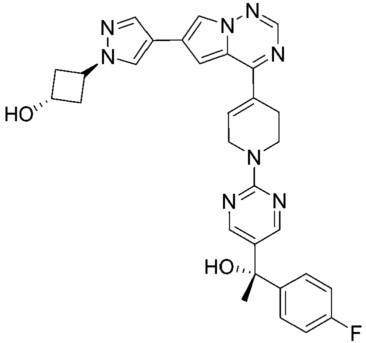 | 52 | 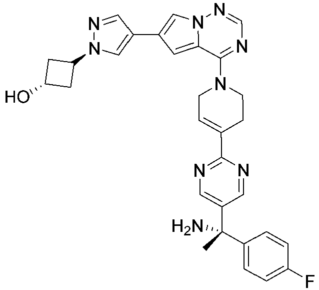 |
| 53 | 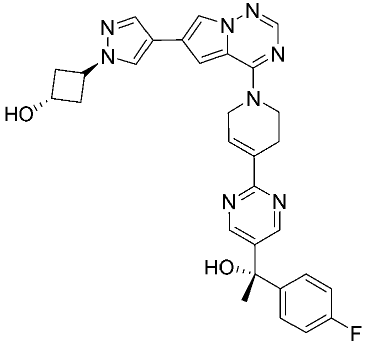 | 54 | 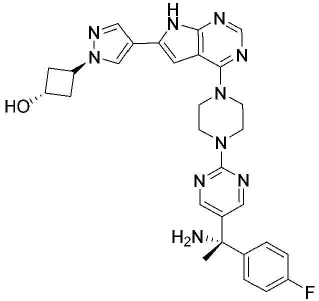 |
| 55 | 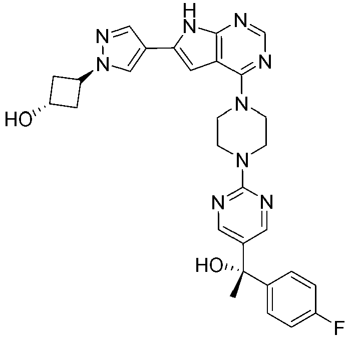 | 56 | 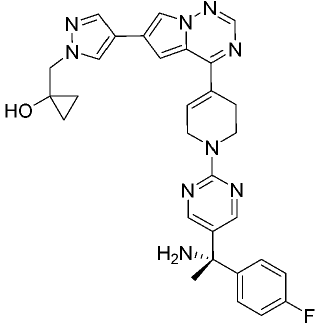 |
| 57 | 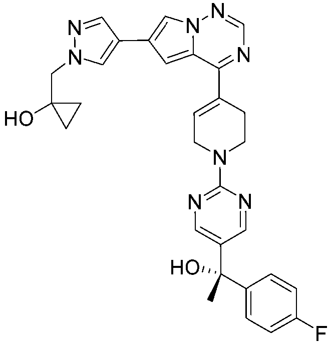 | 58 | 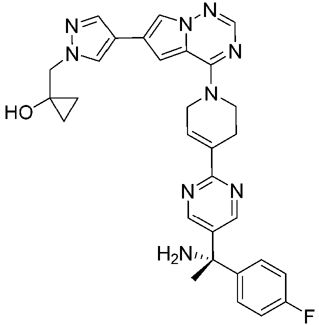 |
| 60 | 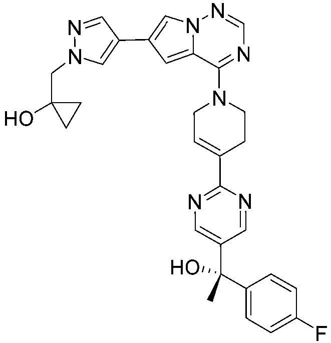 | 61 | 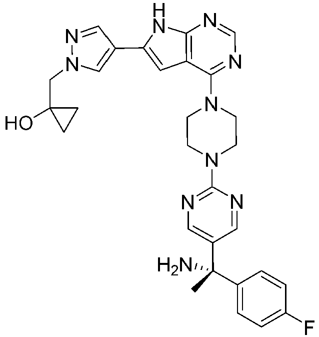 |
| 62 | 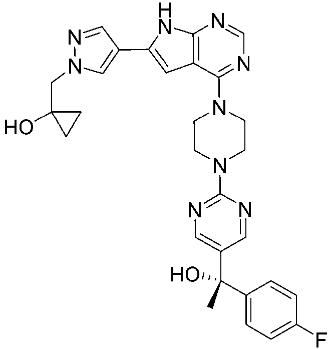 | 63 | 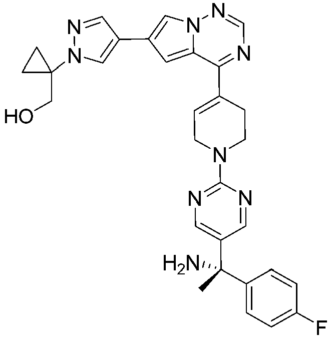 |
| 64 | 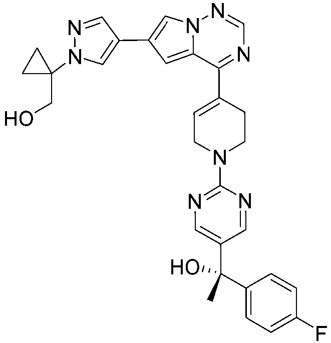 | 65 | 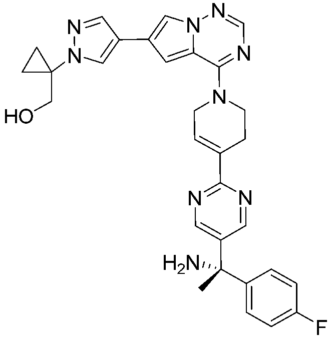 |
| 66 | 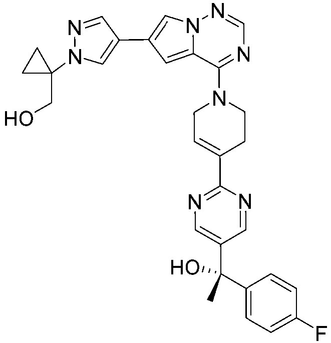 | 67 | 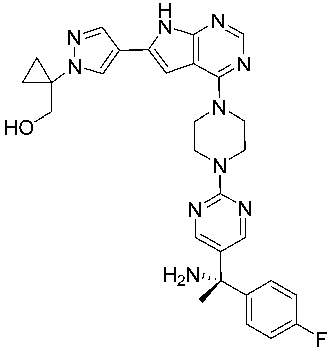 |
| 68 | 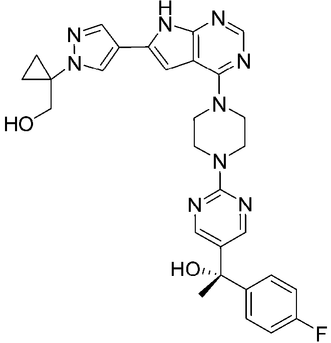 | 69 | 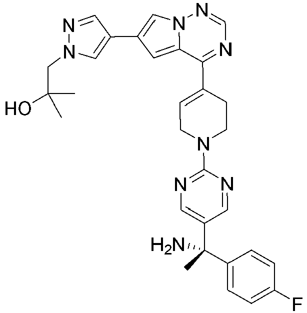 |
| 70 | 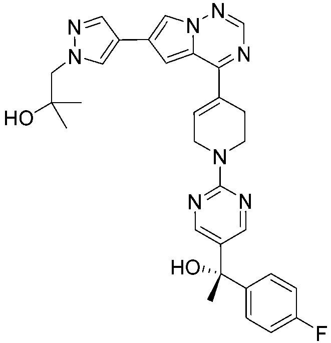 | 71 | 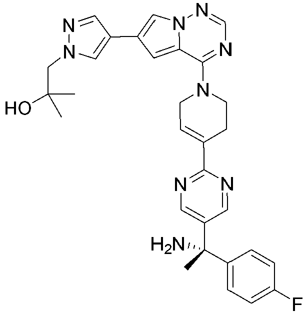 |
| 72 | 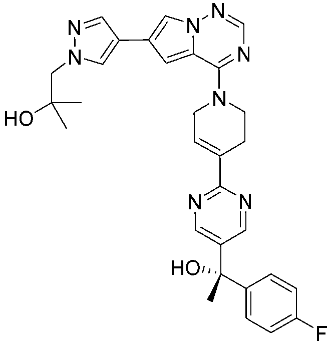 | 73 | 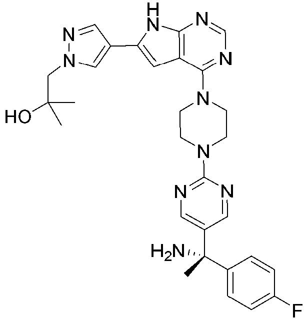 |
| 74 | 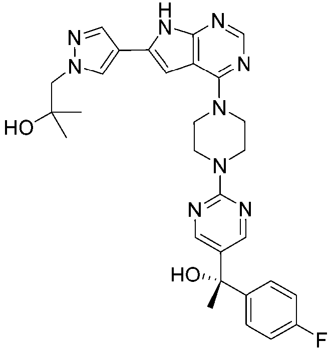 | 75 | 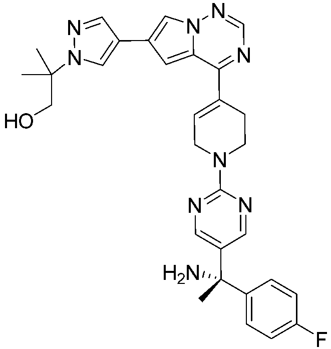 |
| 76 | 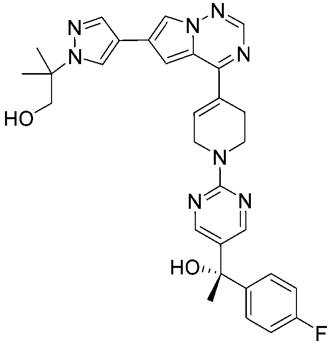 | 77 | 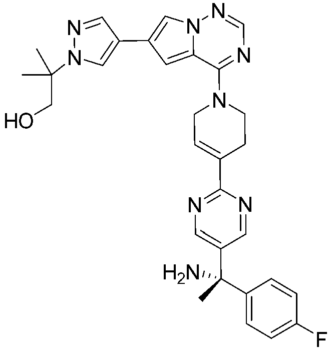 |
| 78 | 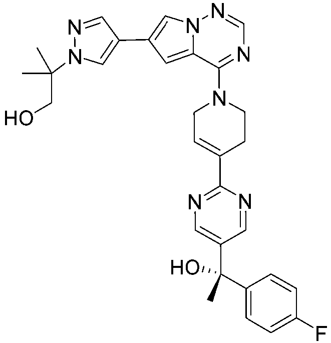 | 79 | 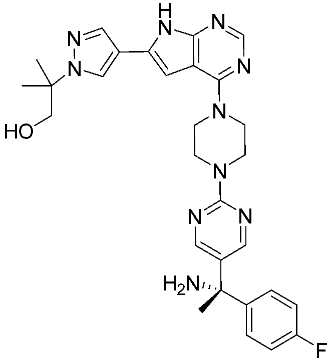 |
| 80 | 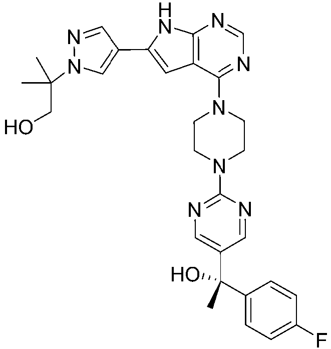 | 81 | 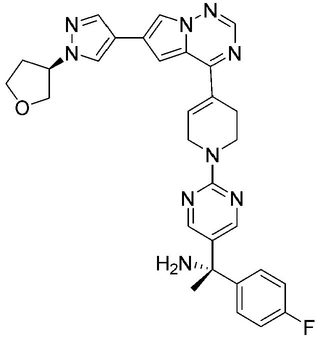 |
| 82 | 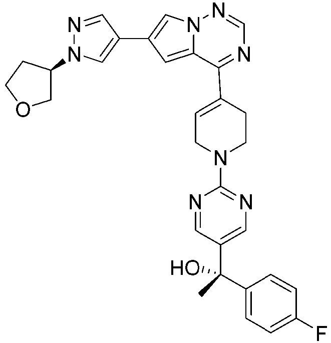 | 83 | 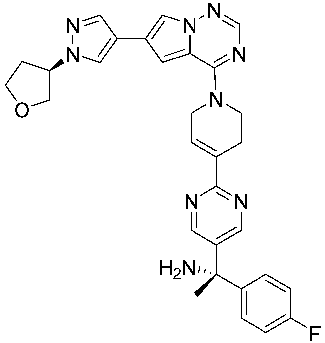 |
| 84 | 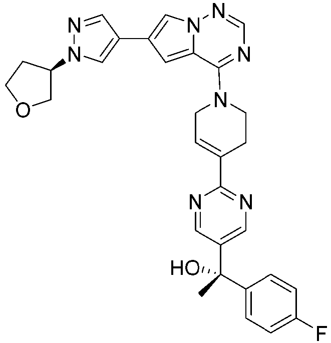 | 85 | 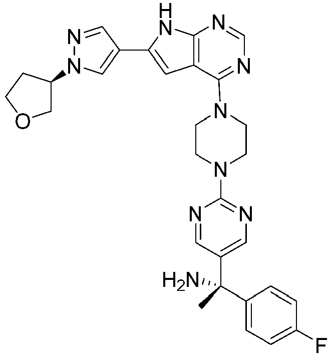 |
| 86 | 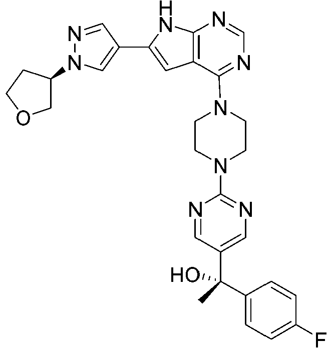 | 87 | 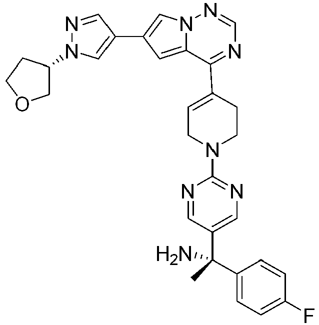 |
| 88 | 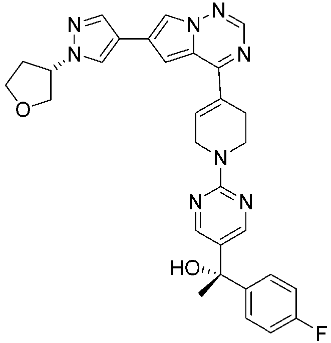 | 89 | 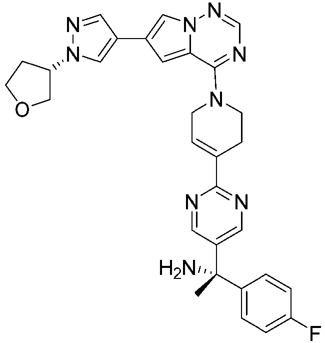 |
| 90 | 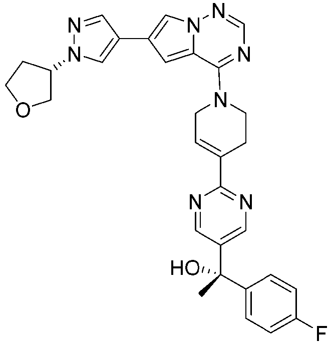 | 91 | 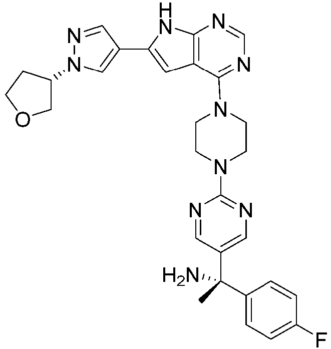 |
| 92 | 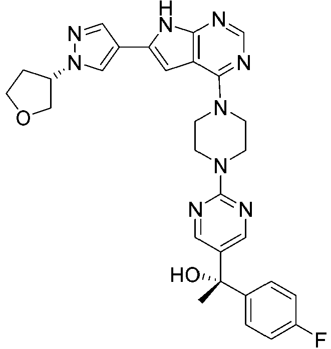 | 93 | 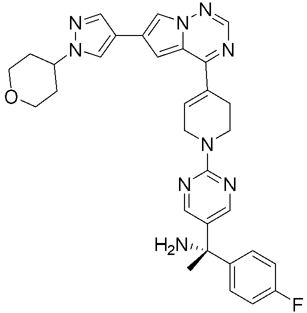 |
| 94 | 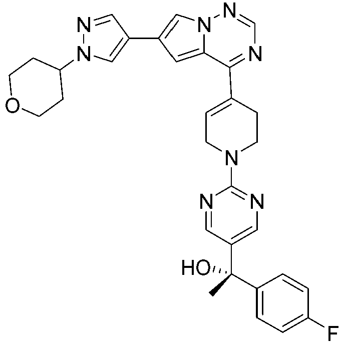 | 95 | 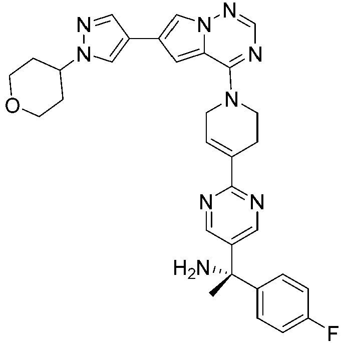 |
| 96 | 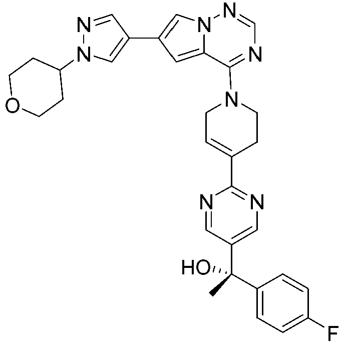 | 97 | 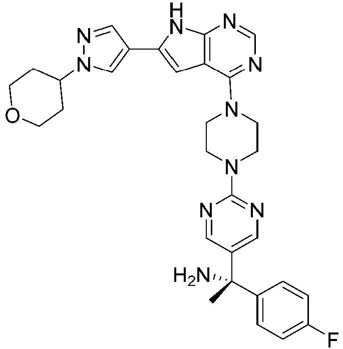 |
| 98 | 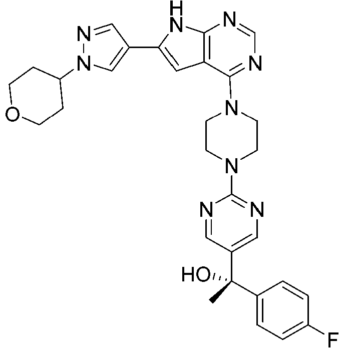 | 99 | 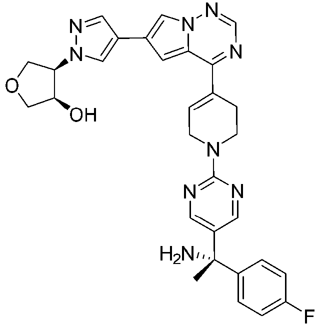 |
| 100 | 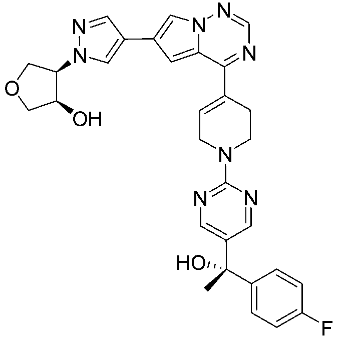 | 101 | 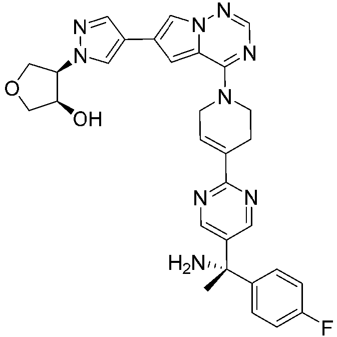 |
| 102 | 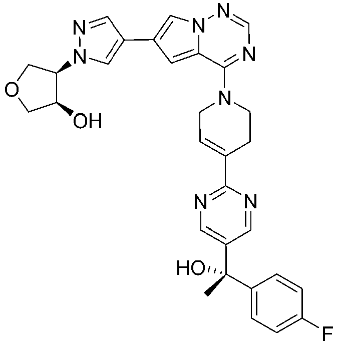 | 103 | 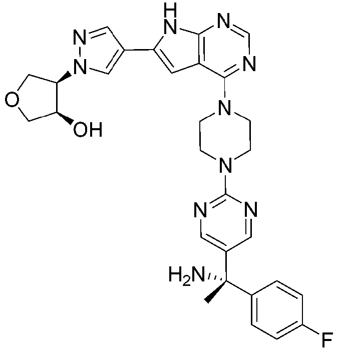 |
| 104 | 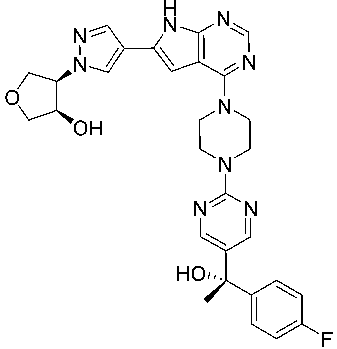 | 105 | 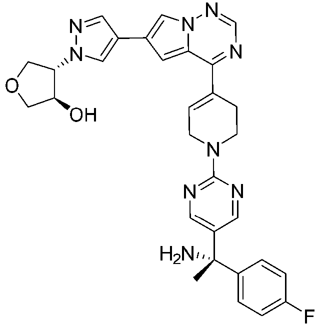 |
| 106 | 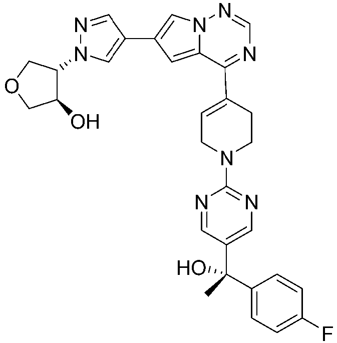 | 107 | 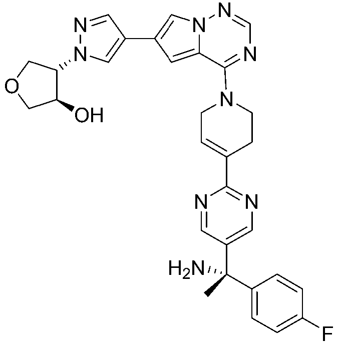 |
| 108 | 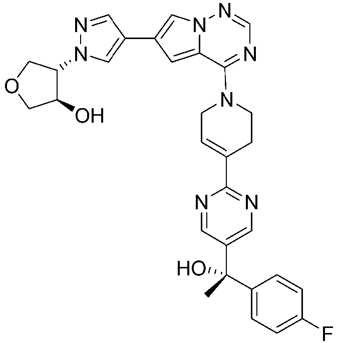 | 109 | 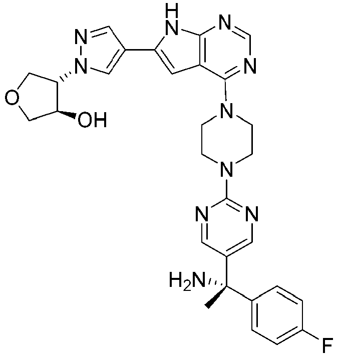 |
| 110 | 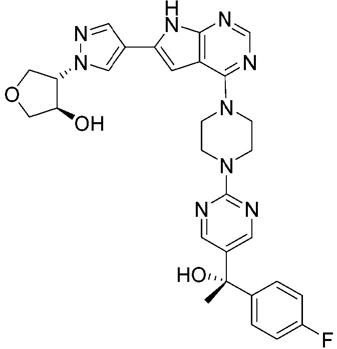 | 111 | 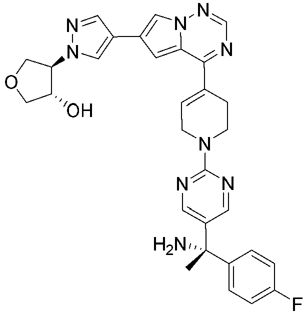 |
| 112 | 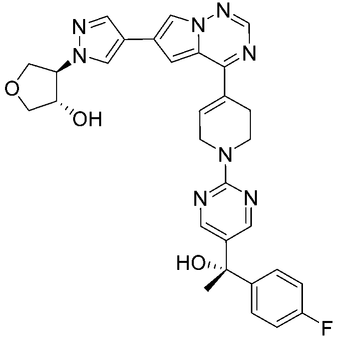 | 113 | 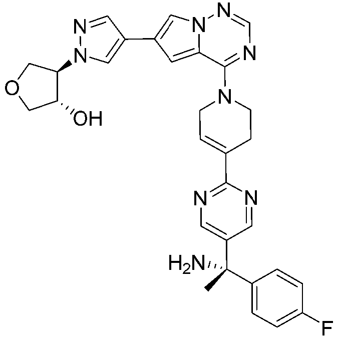 |
| 114 | 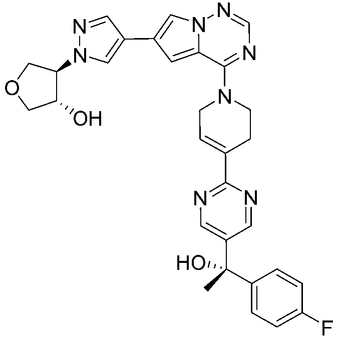 | 115 | 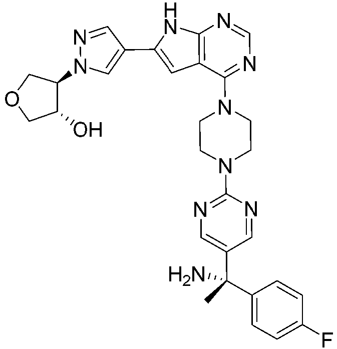 |
| 116 | 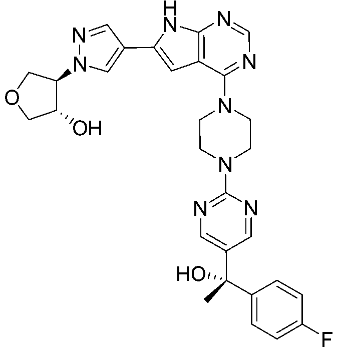 | 117 | 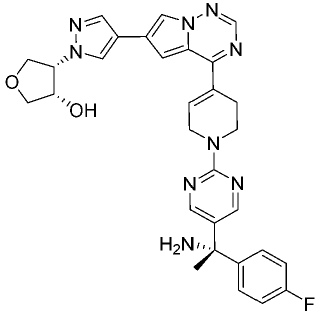 |
| 118 | 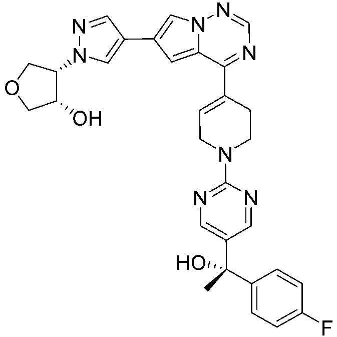 | 119 | 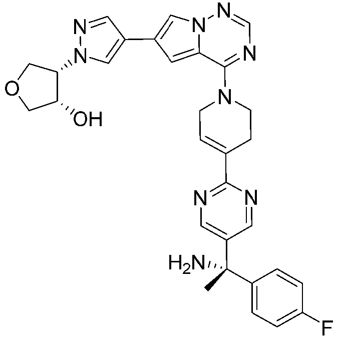 |
| 120 | 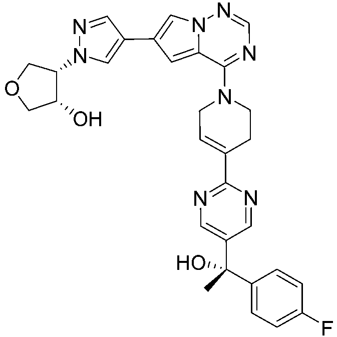 | 121 | 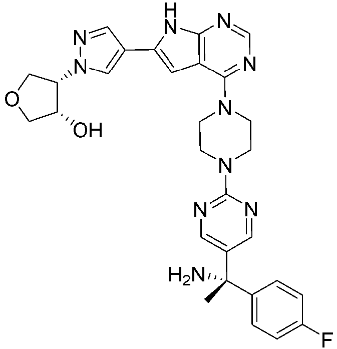 |
| 122 | 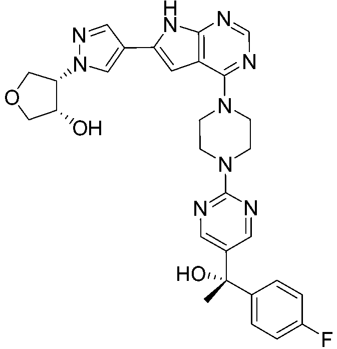 | 123 | 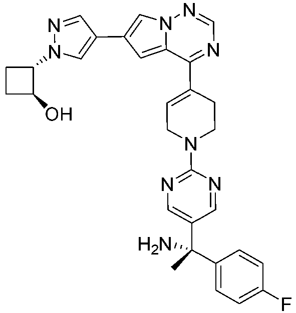 |
| 124 | 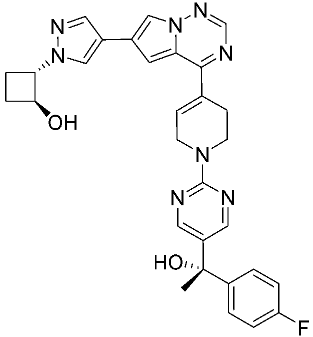 | 125 | 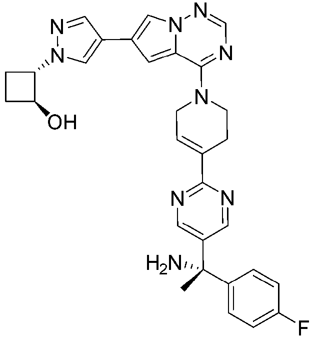 |
| 126 | 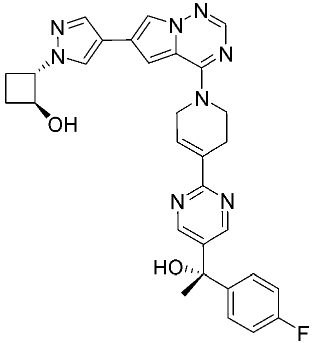 | 127 | 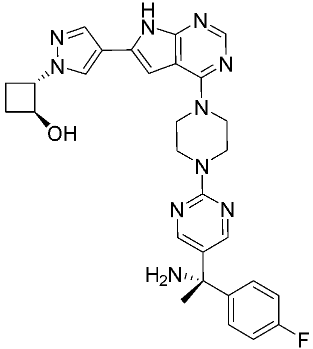 |
| 128 | 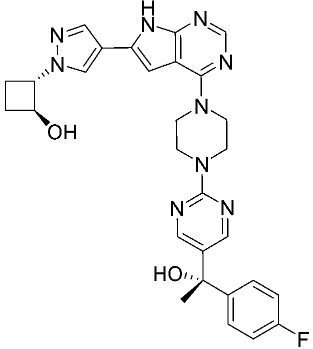 | 129 | 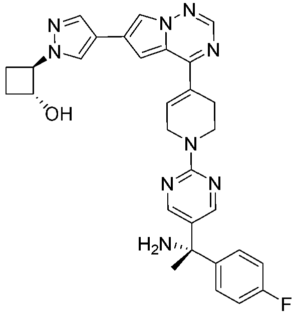 |
| 130 | 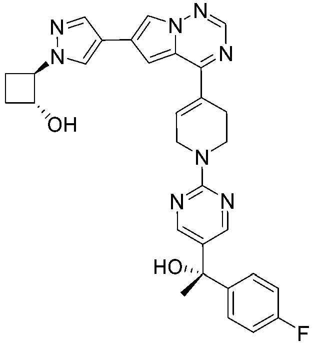 | 131 | 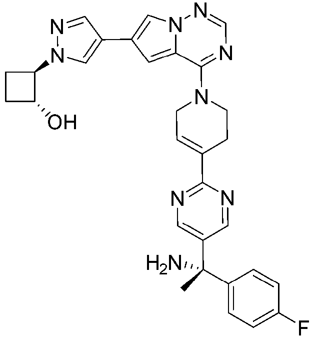 |
| 132 | 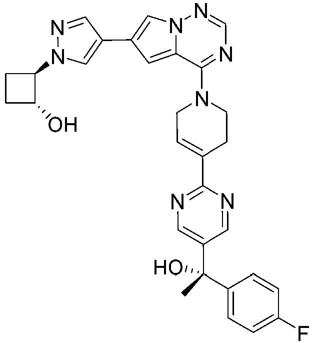 | 133 | 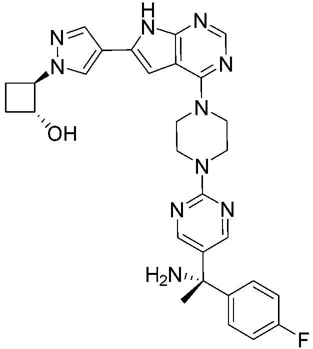 |
| 134 | 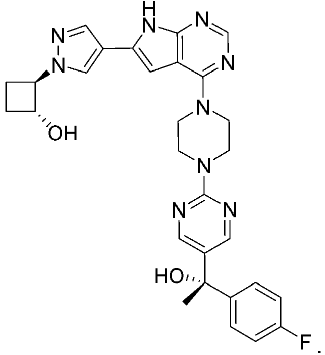 | | |

13. Eine pharmazeutische Zusammensetzung, die Folgendes umfasst:
eine Verbindung nach einem der Ansprüche 1-12, ein pharmazeutisch akzeptables Salz davon und/oder ein Solvat eines der Vorgenannten; und
einen pharmazeutisch akzeptablen Hilfsstoff.

14. Eine Verbindung nach einem der Ansprüche 1-12, ein pharmazeutisch akzeptables Salz davon und/oder ein Solvat eines der Vorgenannten zur Verwendung als ein Medikament bei der Behandlung einer Erkrankung oder eines Krankheitszustands bei einem Patienten, der diese benötigt, wobei die Erkrankung oder der Krankheitszustand aus systemischer Mastozytose, gastrointestinalen Stromatumoren, akuter myeloischer Leukämie, Melanom, Seminom, intrakraniellen Keimzelltumoren, mediastinalem B-Zell-Lymphom, Ewing-Sarkom, diffusem großzelligem B-Zell-Lymphom, Dysgerminom, myelodysplastischem Syndrom, nasalem NK/T-Zell-Lymphom, chronischer myelomonozytärer Leukämie und Hirnkrebs ausgewählt ist; wobei die Erkrankung oder der Krankheitszustand bevorzugt systemische Mastozytose ist; wobei die systemische Mastozytose bevorzugt aus indolenter systemischer Mastozytose und schwelender systemischer Mastozytose ausgewählt ist.

## Revendications

1. Composé selon la formule I : 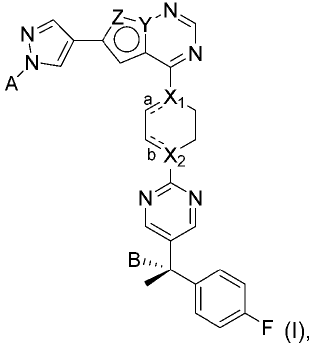 sel pharmaceutiquement acceptable de celui-ci, et/ou solvate de l'un quelconque de ce qui précède, dans lequel :
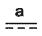 est choisi parmi une liaison simple et une double liaison ;
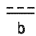 est choisi parmi une liaison simple et une double liaison ;
Z est choisi parmi CH et NH ;
Y est choisi parmi C et N ;
X₁ est choisi parmi CH, C et N ;
X₂ est choisi parmi CH, C et N ;
sous réserve que lorsque X₁ et X₂ sont tous deux N, Y ne soit pas N et Z ne soit pas CH ;
A est 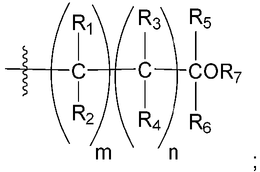
R₁ est choisi parmi l'hydrogène et le méthyle ;
R₂ est choisi parmi l'hydrogène et le méthyle, ou R₁ et R₂ forment ensemble un groupe cyclopropyle ;
R₃ est choisi parmi l'hydrogène et le méthyle ;
R₄ est choisi parmi l'hydrogène et le méthyle, ou R₃ et R₄ forment ensemble un groupe cyclopropyle ;
R₅ est choisi parmi l'hydrogène et le méthyle ;
R₆ est choisi parmi l'hydrogène et le méthyle, ou R₅ et R₆ forment ensemble un groupe cyclopropyle, ou l'un de R₂ ou R₄, conjointement avec R₆, forme un groupe cyclobutyle ; R₇ est l'hydrogène, ou l'un de R₂, R₄, or R₆, conjointement avec R₇, forme un cycle choisi parmi le tétrahydrofurane et le tétrahydropyrane, ledit tétrahydrofurane ou tétrahydropyrane étant optionnellement substitué par un groupe hydroxyle ;
m vaut 0 ou 1 ; n vaut 0 ou 1 ; et B est sélectionné parmi OH et NH₂.

2. Composé selon la revendication 1, sel pharmaceutiquement acceptable de celui-ci, et/ou solvate de l'un quelconque de ce qui précède, le composé ayant la formule (II) :

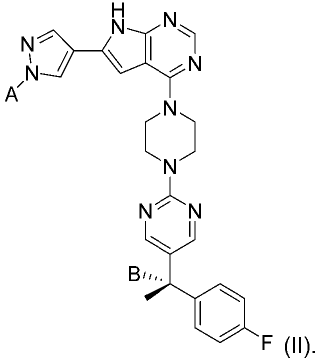

3. Composé selon la revendication 1, sel pharmaceutiquement acceptable de celui-ci, et/ou solvate de l'un quelconque de ce qui précède, le composé ayant la formule (III) :

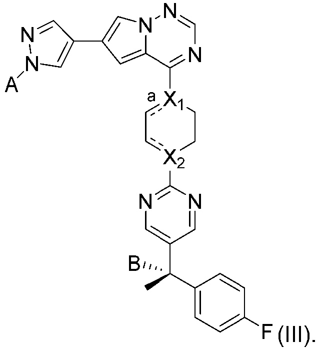

4. Composé selon la revendication 3, sel pharmaceutiquement acceptable de celui-ci, et/ou solvate de l'un quelconque de ce qui précède, dans lequel X₁ est N et 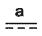 est une liaison simple et X₂ est C et 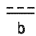 est une double liaison.

5. Composé selon la revendication 3, sel pharmaceutiquement acceptable de celui-ci, et/ou solvate de l'un quelconque de ce qui précède, dans lequel X₁ est C et 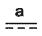 est une double liaison et X₂ est N et 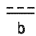 est une liaison simple.

6. Composé selon l'une quelconque des revendications 1 à 5, sel pharmaceutiquement acceptable de celui-ci, et/ou solvate de l'un quelconque de ce qui précède, dans lequel :
A est : 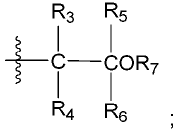
R₃ est choisi parmi l'hydrogène et le méthyle ;
R₄ est choisi parmi l'hydrogène et le méthyle, ou R₃ et R₄ forment ensemble un groupe cyclopropyle ;
R₅ est choisi parmi l'hydrogène et le méthyle ;
R₆ est choisi parmi l'hydrogène et le méthyle ; ou
R₅ et R₆ forment ensemble un groupe cyclopropyle, et
R⁷ est l'hydrogène.

7. Composé selon l'une quelconque des revendications 1 à 6, sel pharmaceutiquement acceptable de celui-ci, et/ou solvate de l'un quelconque de ce qui précède, dans lequel B est NH₂.

8. Composé selon l'une quelconque des revendications 1 à 6, sel pharmaceutiquement acceptable de celui-ci, et/ou solvate de l'un quelconque de ce qui précède, dans lequel B est OH.

9. Composé selon l'une quelconque des revendications 1 à 5, 7 et 8, sel pharmaceutiquement acceptable de celui-ci, et/ou solvate de l'un quelconque de ce qui précède, (i) dans lequel A est choisi parmi 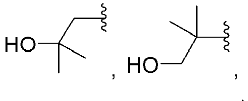 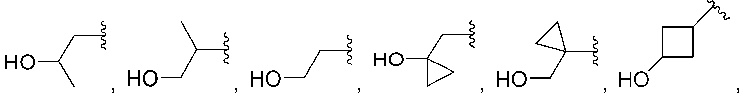 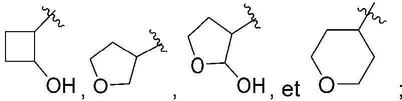 ou (ii) dans lequel A est choisi parmi 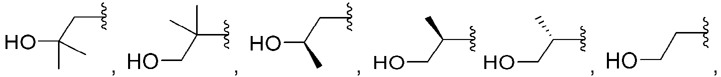 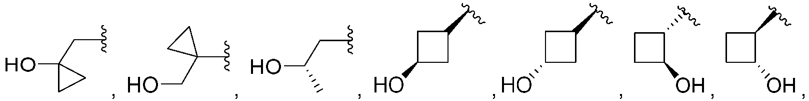 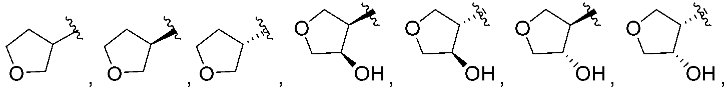 et 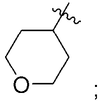 ou
(iii) dans lequel A est choisi parmi 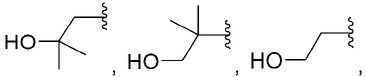 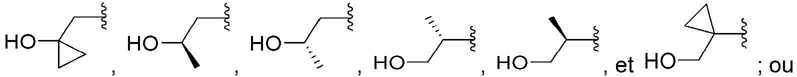
(iv) dans lequel A est choisi parmi

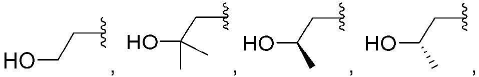

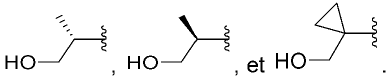

10. Composé selon l'une quelconque des revendications 1 à 5, 7 et 8, sel pharmaceutiquement acceptable de celui-ci, et/ou solvate de l'un quelconque de ce qui précède, dans lequel A est choisi parmi

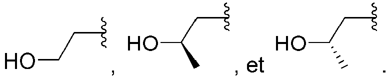

11. Composé selon l'une quelconque des revendications 1 à 5, 7 et 8, sel pharmaceutiquement acceptable de celui-ci, et/ou solvate de l'un quelconque de ce qui précède, dans lequel A est

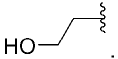

12. Composé selon la revendication 1, sel pharmaceutiquement acceptable de celui-ci, et/ou solvate de l'un quelconque de ce qui précède, le composé étant sélectionné parmi :
| **N°** | **Structure chimique** | **N°** | **Structure chimique** |
|---|---|---|---|
| 1 | 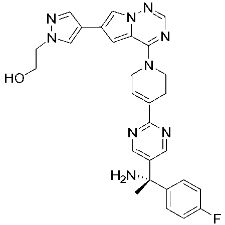 | 2 | 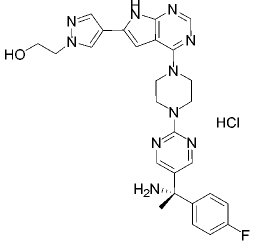 |
| | | 4 | 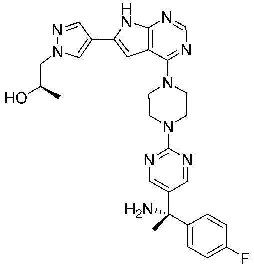 |
| 5 | 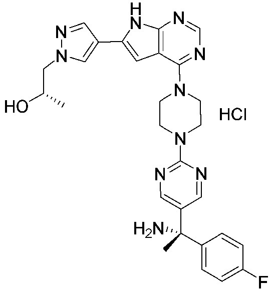 | | |
| 7 | 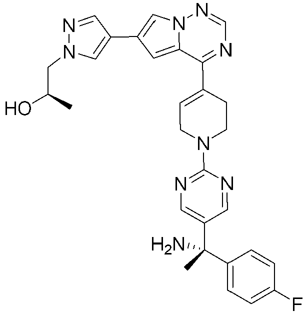 | 8 | 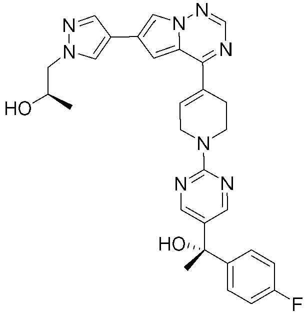 |
| 9 | 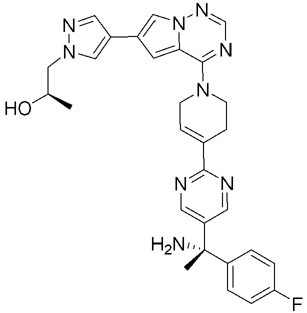 | 10 | 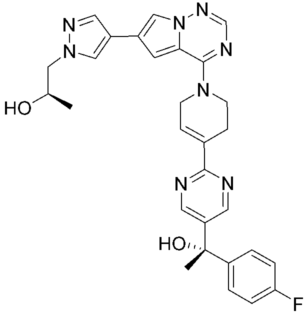 |
| 11 | 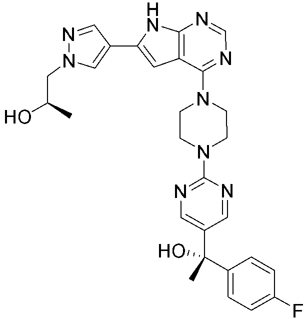 | 12 | 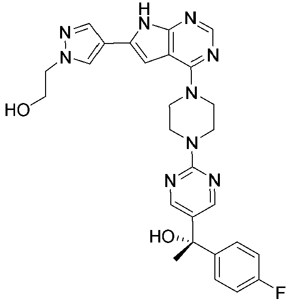 |
| 13 | 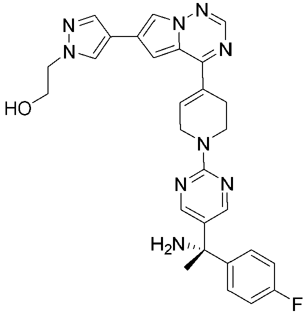 | 14 | 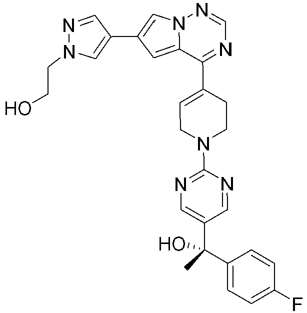 |
| 14-A | 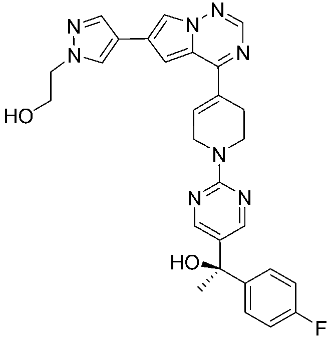 | 15 | 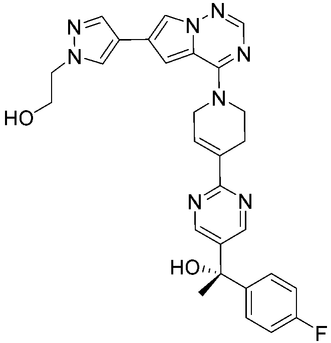 |
| 16 | 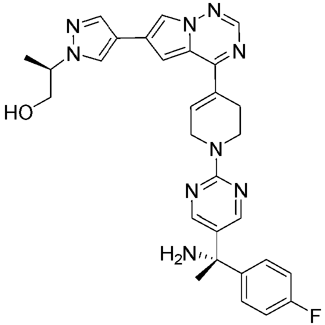 | 17 | 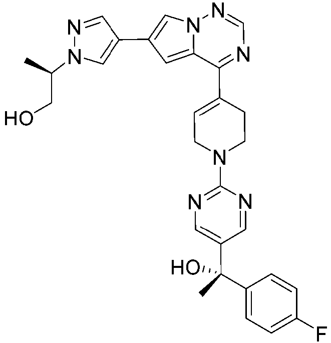 |
| 18 | 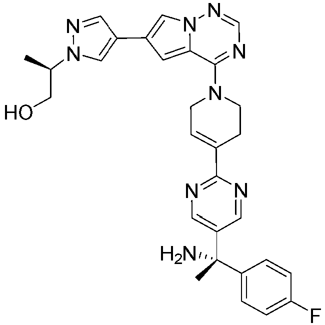 | 19 | 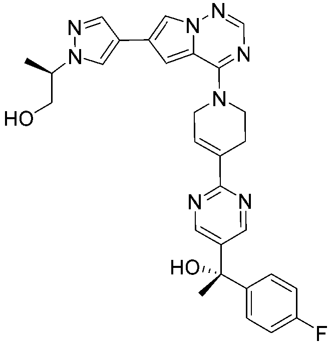 |
| 20 | 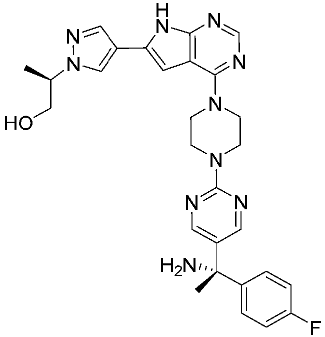 | 21 | 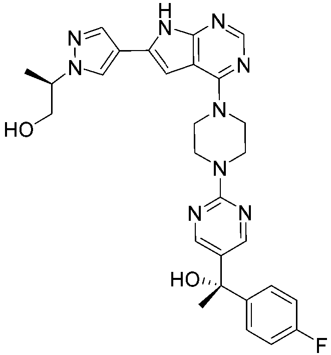 |
| 22 | 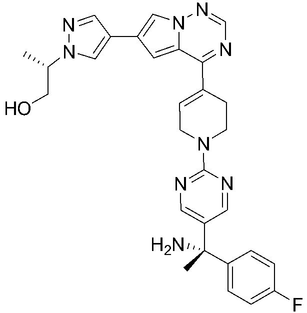 | 23 | 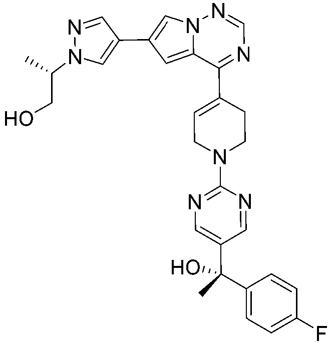 |
| 24 | 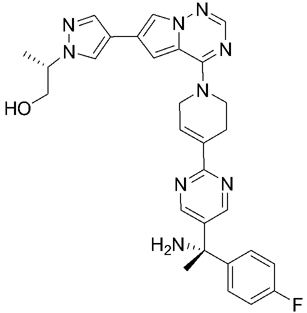 | 25 | 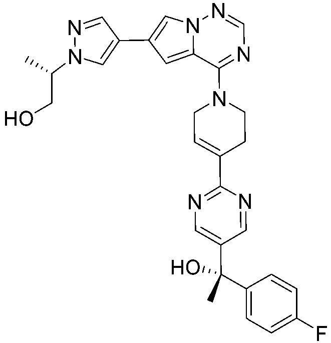 |
| 26 | 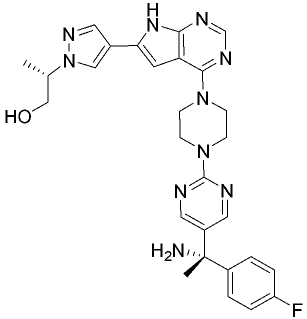 | 27 | 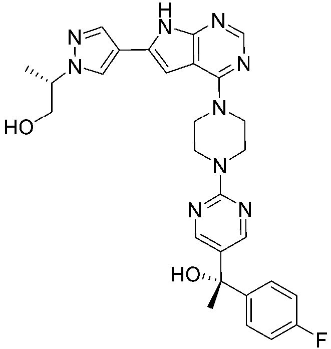 |
| 38 | 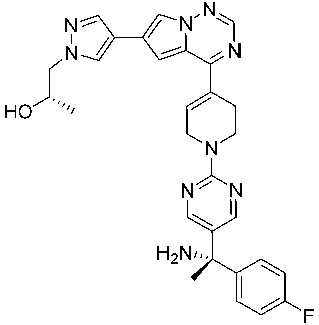 | 39 | 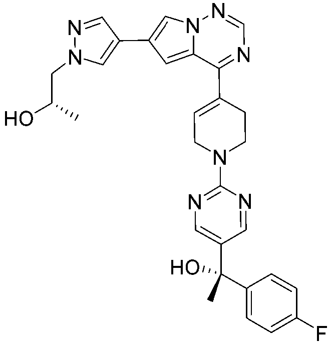 |
| 40 | 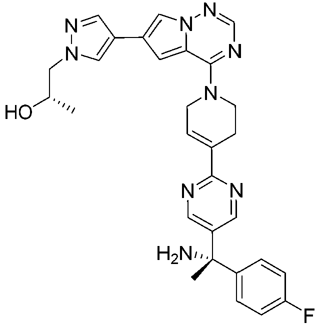 | 41 | 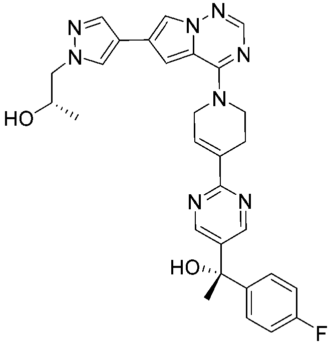 |
| 42 | 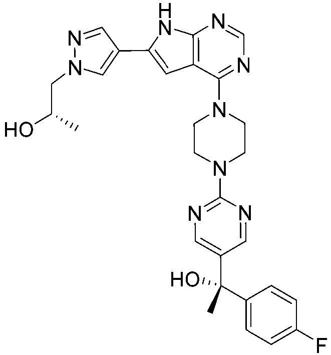 | 44 | 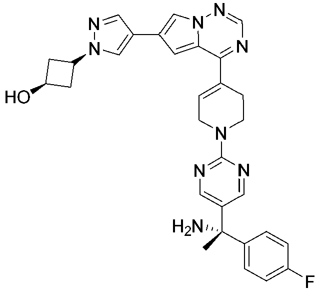 |
| 45 | 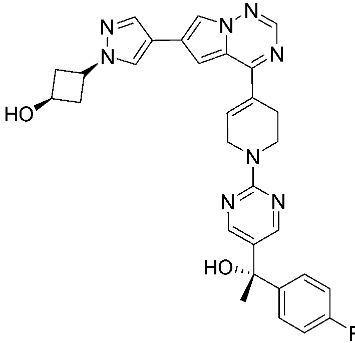 | 46 | 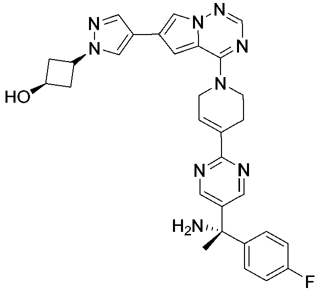 |
| 47 | 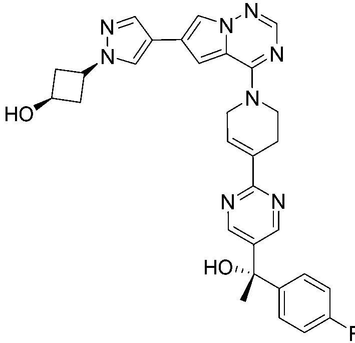 | 48 | 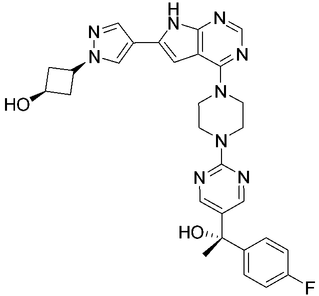 |
| 49 | 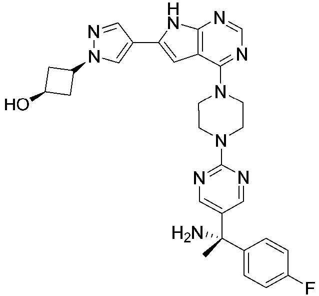 | 50 | 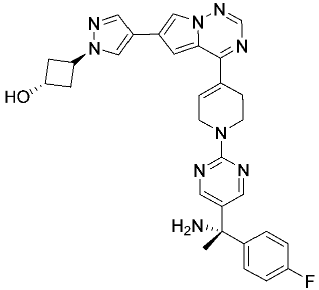 |
| 51 | 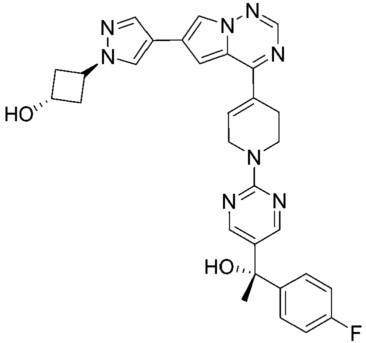 | 52 | 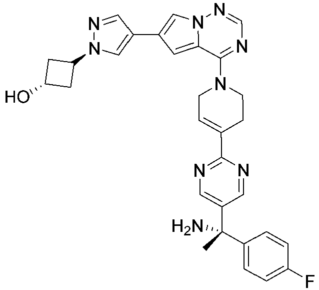 |
| 53 | 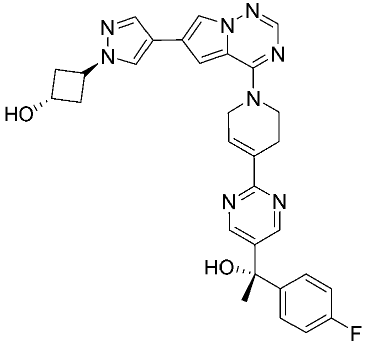 | 54 | 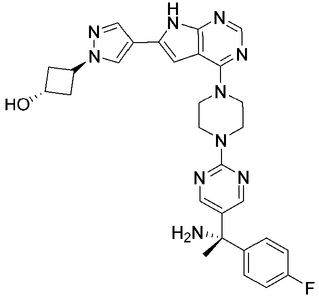 |
| 55 | 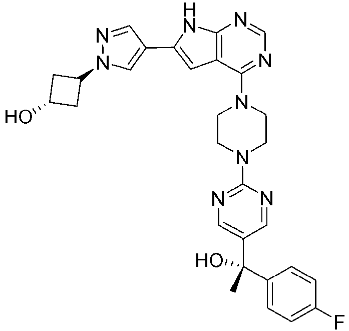 | 56 | 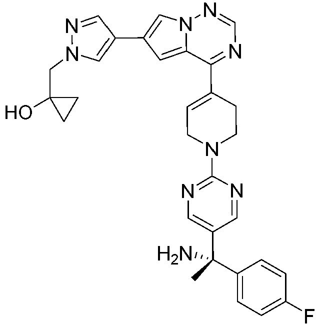 |
| 57 | 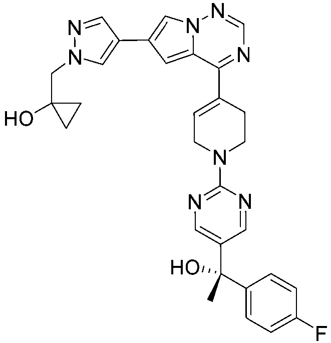 | 58 | 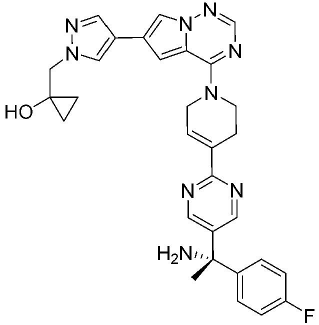 |
| 60 | 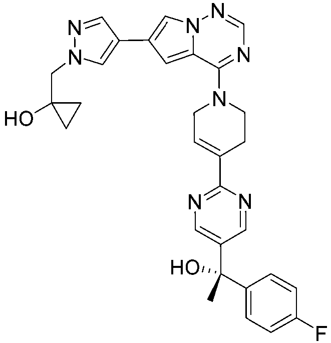 | 61 | 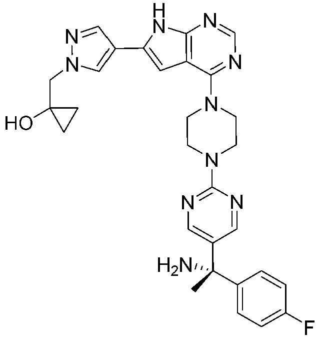 |
| 62 | 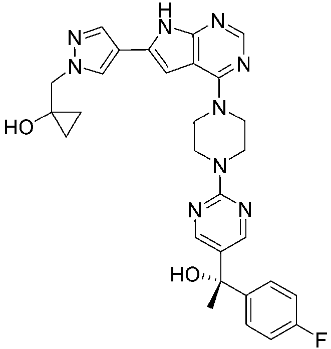 | 63 | 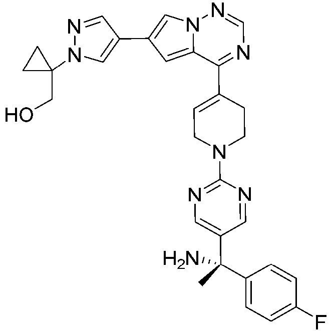 |
| 64 | 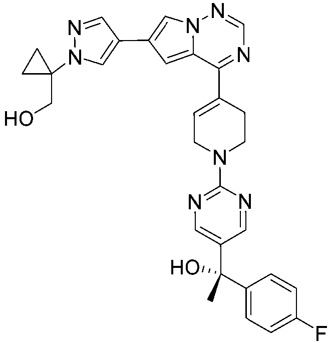 | 65 | 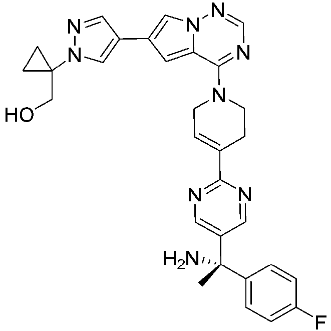 |
| 66 | 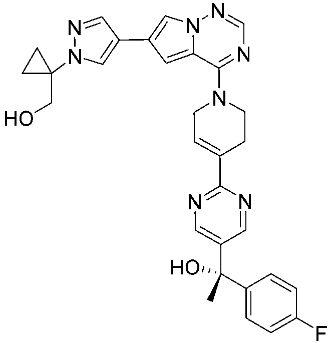 | 67 | 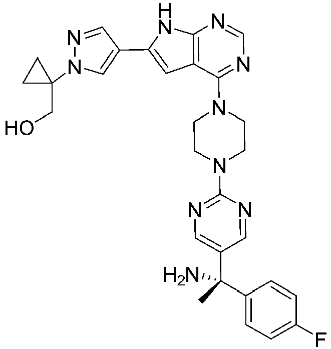 |
| 68 | 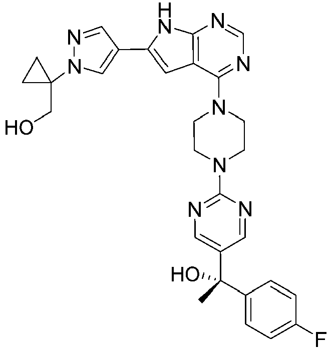 | 69 | 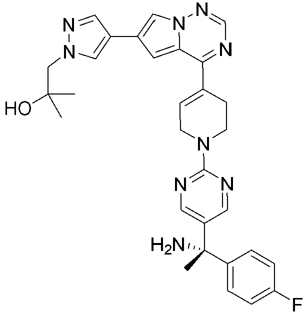 |
| 70 | 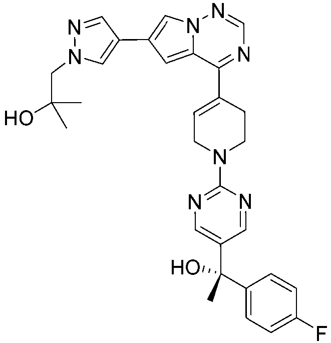 | 71 | 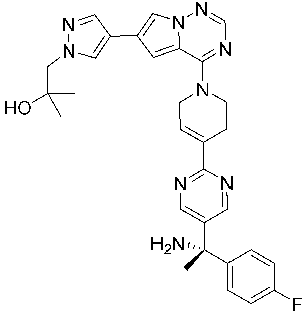 |
| 72 | 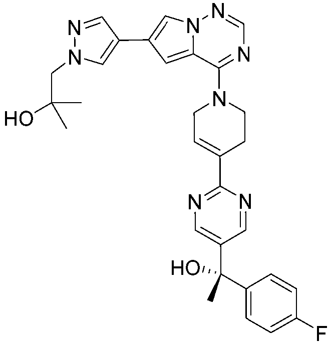 | 73 | 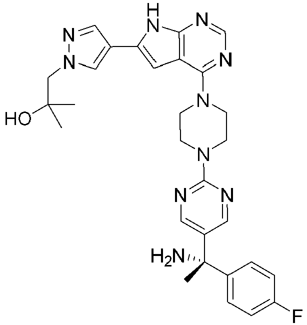 |
| 74 | 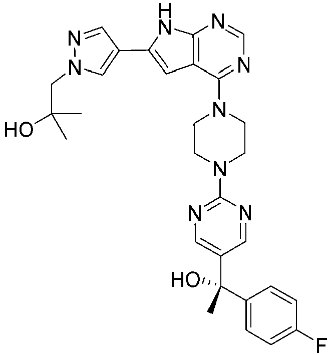 | 75 | 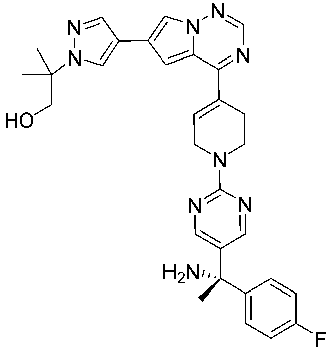 |
| 76 | 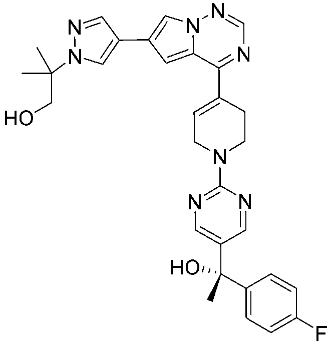 | 77 | 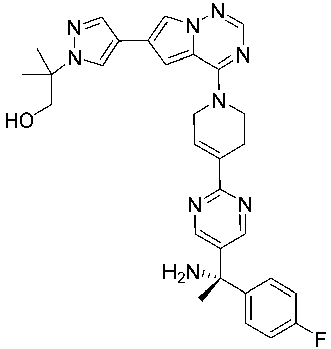 |
| 78 | 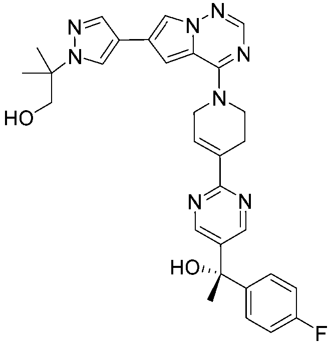 | 79 | 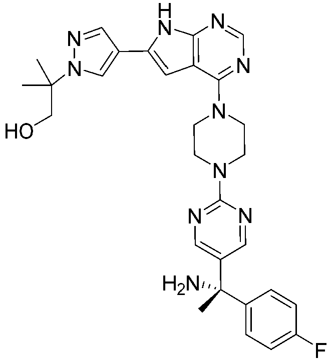 |
| 80 | 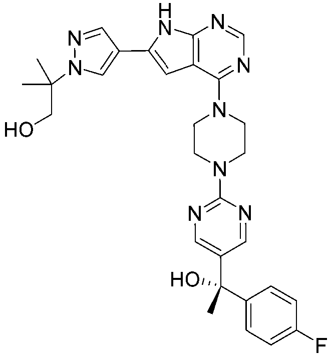 | 81 | 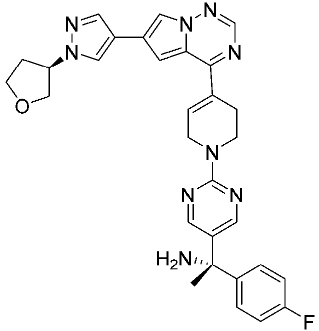 |
| 82 | 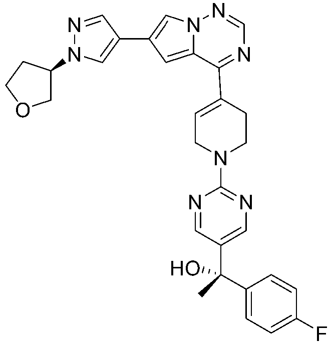 | 83 | 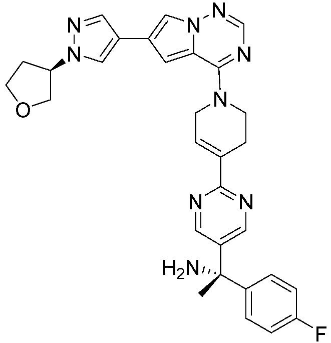 |
| 84 | 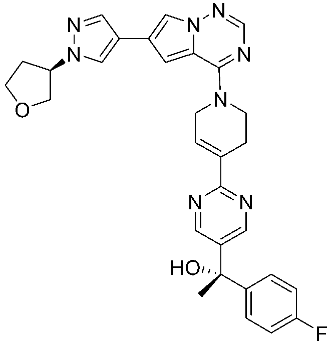 | 85 | 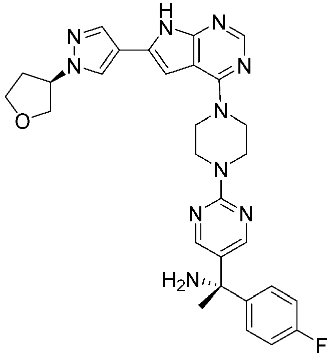 |
| 86 | 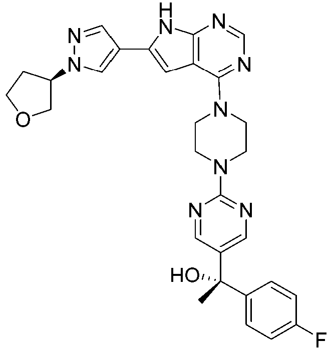 | 87 | 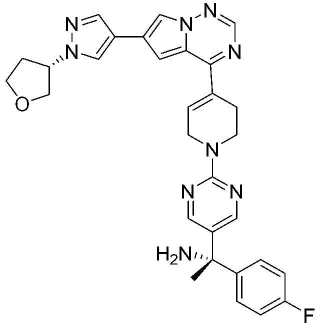 |
| 88 | 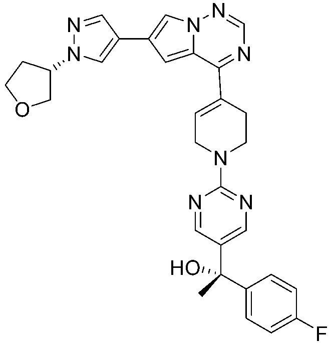 | 89 | 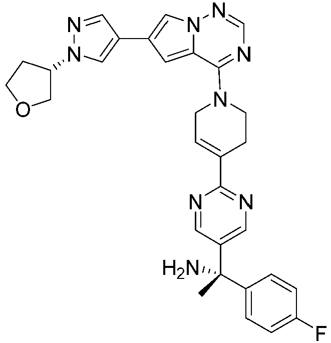 |
| 90 | 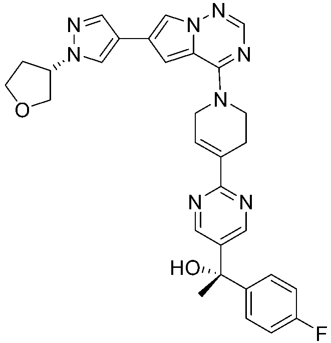 | 91 | 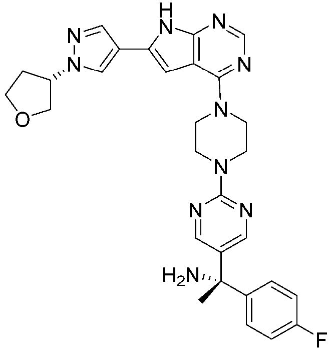 |
| 92 | 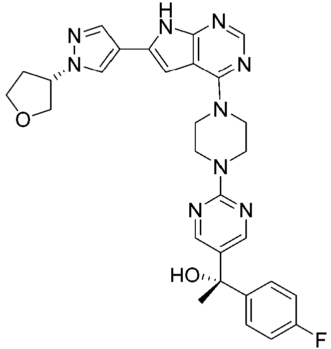 | 93 | 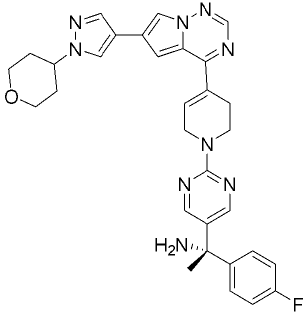 |
| 94 | 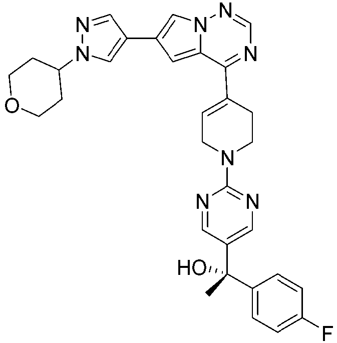 | 95 | 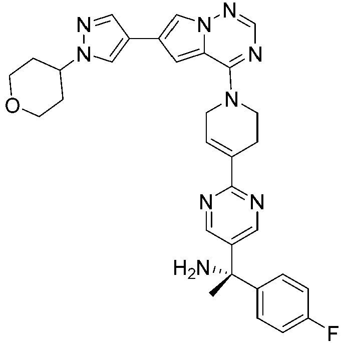 |
| 96 | 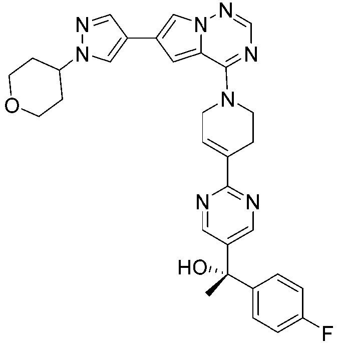 | 97 | 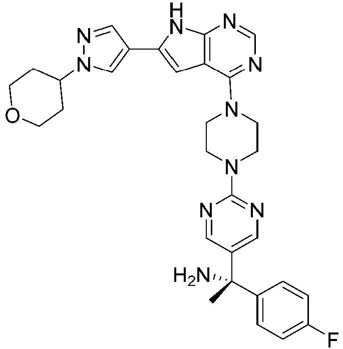 |
| 98 | 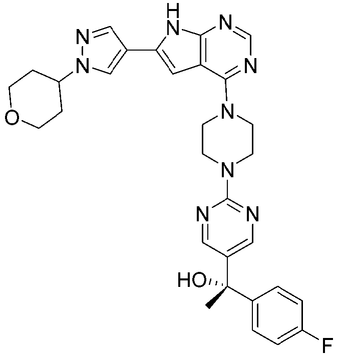 | 99 | 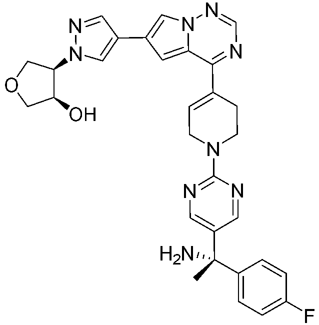 |
| 100 | 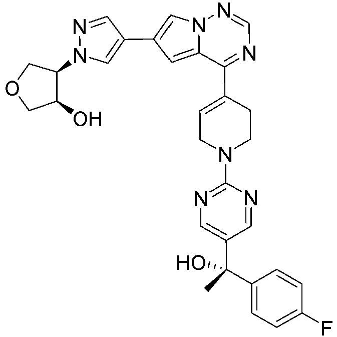 | 101 | 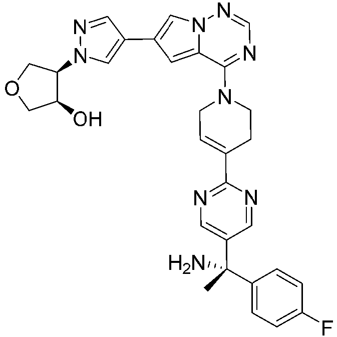 |
| 102 | 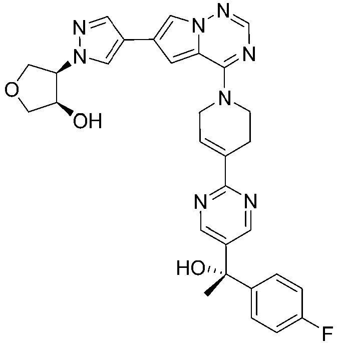 | 103 | 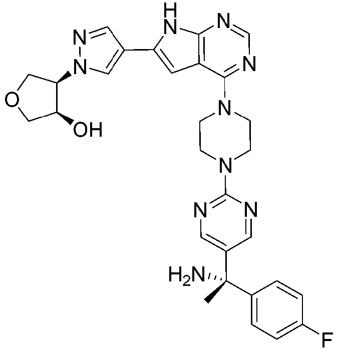 |
| 104 | 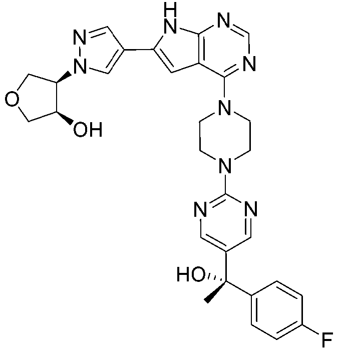 | 105 | 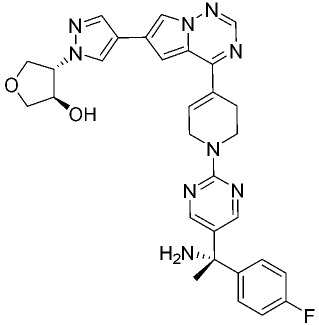 |
| 106 | 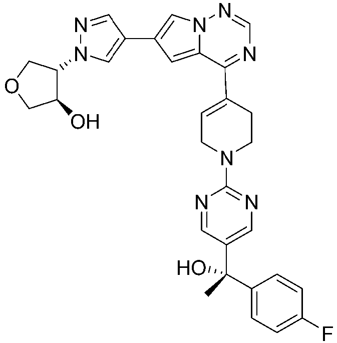 | 107 | 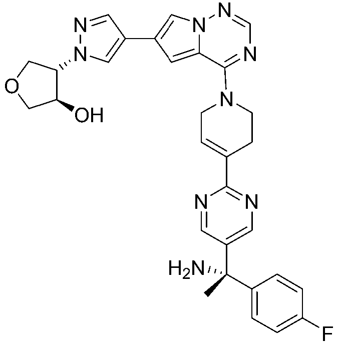 |
| 108 | 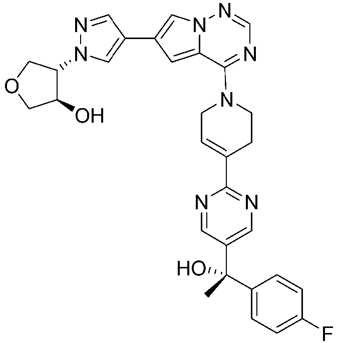 | 109 | 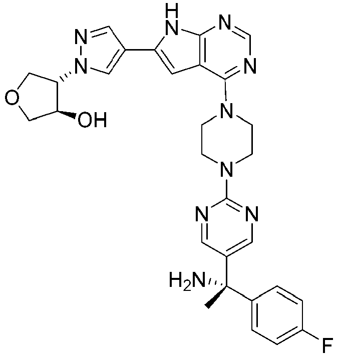 |
| 110 | 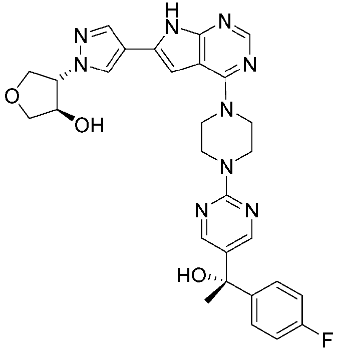 | 111 | 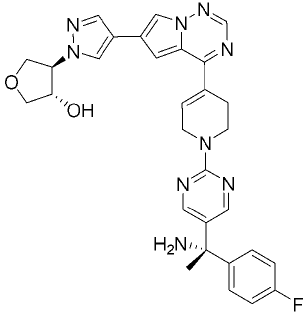 |
| 112 | 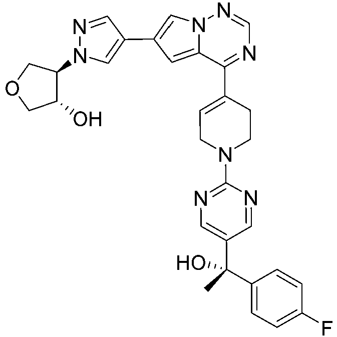 | 113 | 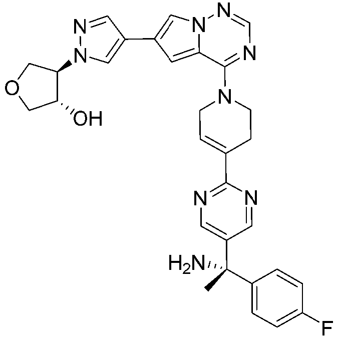 |
| 114 | 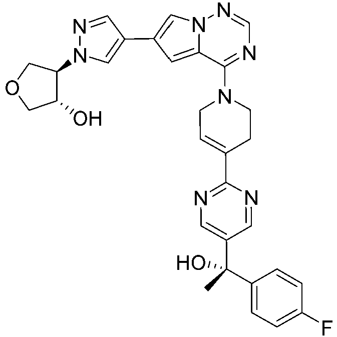 | 115 | 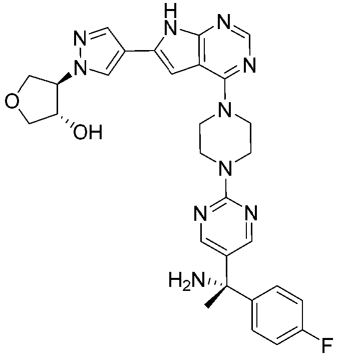 |
| 116 | 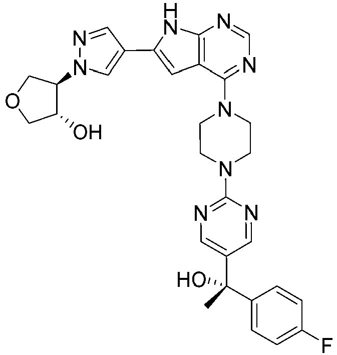 | 117 | 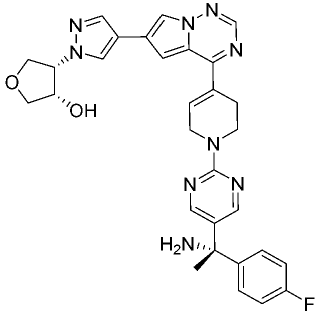 |
| 118 | 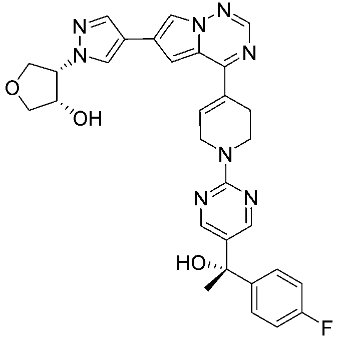 | 119 | 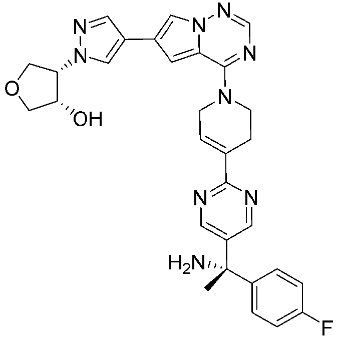 |
| 120 | 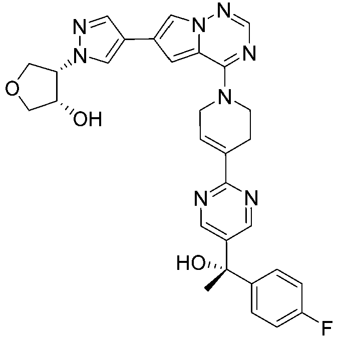 | 121 | 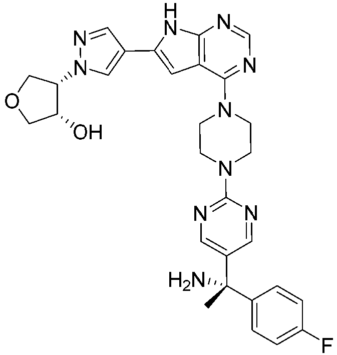 |
| 122 | 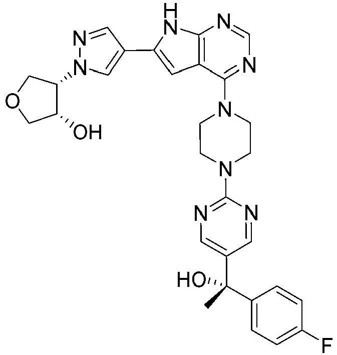 | 123 | 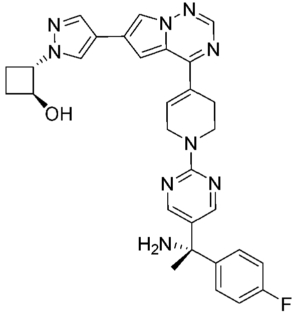 |
| 124 | 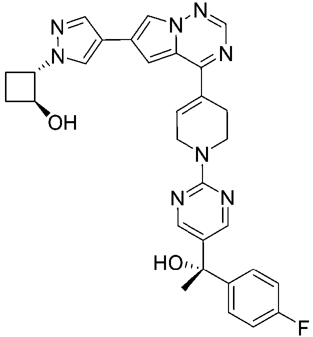 | 125 | 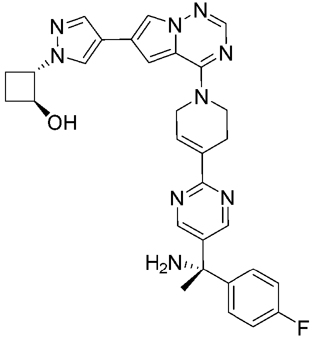 |
| 126 | 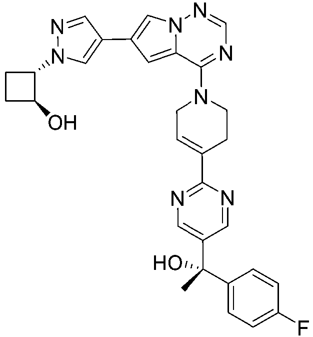 | 127 | 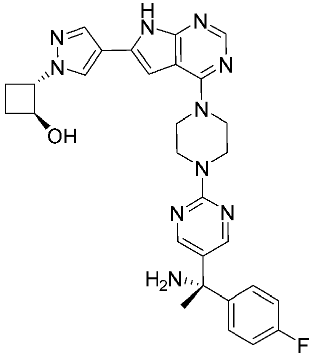 |
| 128 | 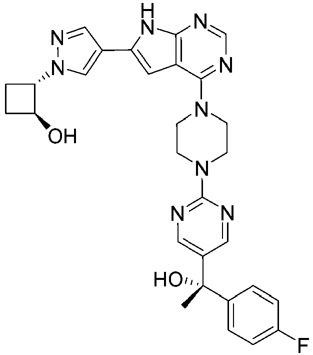 | 129 | 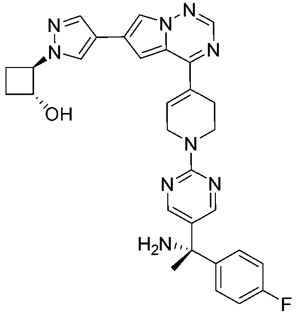 |
| 130 | 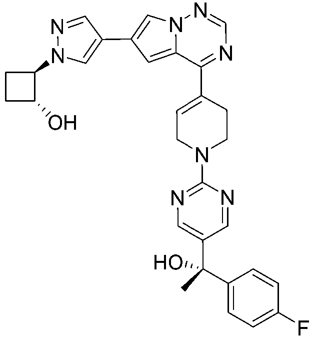 | 131 | 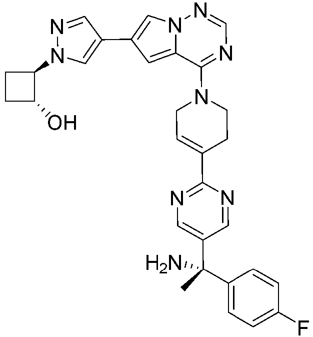 |
| 132 | 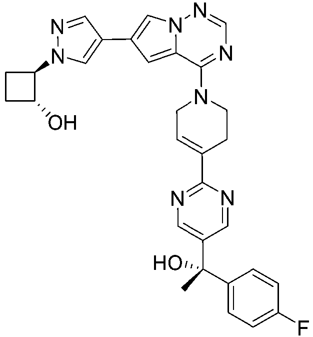 | 133 | 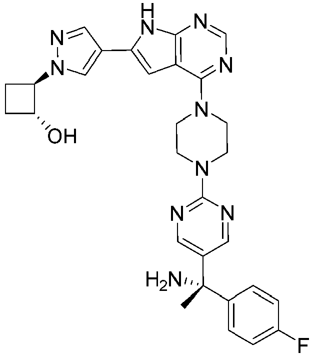 |
| 134 | 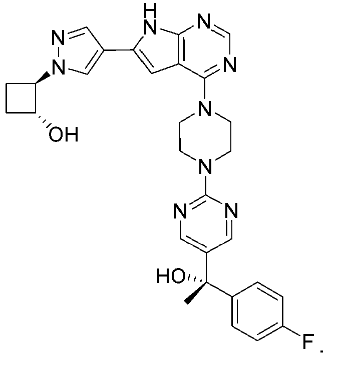 | | |

13. Composition pharmaceutique comprenant :
un composé selon l'une quelconque des revendications 1 à 12, un sel pharmaceutiquement acceptable de celui-ci, et/ou un solvate de l'un quelconque de ce qui précède ; et
un excipient pharmaceutiquement acceptable.

14. Composé selon l'une quelconque des revendications 1 à 12, sel pharmaceutiquement acceptable de celui-ci, et/ou solvate de l'un quelconque de ce qui précède, destiné à une utilisation comme médicament dans le traitement d'une maladie ou d'une affection chez un patient qui en a besoin, la maladie ou l'affection étant choisie parmi la mastocytose systémique, les tumeurs stromales gastro-intestinales, la leucémie myéloïde aiguë, le mélanome, le séminome, les tumeurs intracrâniennes à cellules germinales, le lymphome médiastinal à cellules **B, le** sarcome d'Ewing, le lymphome diffus à grandes cellules **B,** le dysgerminome, le syndrome myélodysplasique, le lymphome NK/T de type nasal, la leucémie myélomonocytaire chronique et le cancer du cerveau ; la maladie ou l'affection étant préférablement une mastocytose systémique ; la mastocytose systémique étant préférablement choisie parmi la mastocytose systémique indolente et la mastocytose systémique latente.
